# EUROPEAN PATENT APPLICATION

(11) **EP 4 653 442 A1**
(43) Date of publication of application: **26.11.2025**
(21) Application number: 24744372.4
(22) Date of filing: 18.01.2024
(51) Int. Cl.: C07D 487/04, C07D 487/00, A61K 31/519, A61P 35/00

(54) **POLYCYCLIC COMPOUND FOR TREATING NEUROLOGICAL DISEASES AND TUMORS**

(30) Priority: 19.01.2023 CN 202310056731
(71) Applicant: SHANGHAI INSTITUTE OF ORGANIC CHEMISTRY, CHINESE ACADEMY OF SCIENCES, Shanghai 200032 (CN)
(72) Inventor: WANG, Zhaoyin, Shanghai 200032 (CN); HE, Zhuohao, Shanghai 200032 (CN); ZHU, Junjie, Shanghai 200032 (CN)
(74) Representative: Mewburn Ellis LLP
(86) International application number: PCT/CN2024/073112
(87) International publication number: WO 2024/153202

(57) **Abstract**

The present invention discloses polycyclic compounds as microtubule stabilizers and preparation methods thereof, the structure of which is represented by general formula (I), wherein Ar, W, Z, X, Y, and heterocycle A are as defined in the Description. The invention also discloses methods for preparing said compounds. The compounds of general formula (I) according to the present invention can be used to prepare a medicament for preventing and/or treating tumors and neurodegenerative diseases.

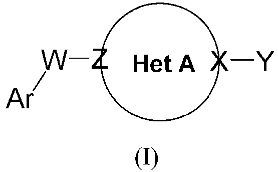

## Description

### TECHNICAL FIELD

The present invention belongs to the field of medicinal chemistry, and specifically relates to compounds containing spiro and fused ring structures for treating nervous system diseases and tumors (including gliomas), and preparation methods thereof.

### BACKGROUND

Microtubules, as fundamental structural components of the cytoskeleton, mediate essential physiological processes such as cell proliferation, maintenance of cell morphology, and intracellular material transport. In the central nervous system, neurons - the primary functional cells - represent a specialized cell type that is post-mitotic and exhibits polarized morphology. The axons responsible for neural signal transmission rely on microtubules and other cytoskeletal proteins to form rapid transport tracks, enabling efficient intracellular transport between the cell body and nerve terminals. Under pathological conditions, changes in microtubule composition and function contribute to the pathogenesis of numerous diseases. For instance, Alzheimer's disease(AD) presents two characteristic pathological features: β-amyloid (Aβ) plaques and Tau protein aggregates. Hyperphosphorylation of Tau protein impairs its microtubule-binding capacity, leading to the formation of neurofibrillary tangles. This disrupts microtubule-mediated axonal transport, ultimately causing distal axonal degeneration. Microtubule stabilizers can promote Tau protein binding to microtubules, improve vesicular transport function through protein quality control systems, and reduce Tau-induced neurofibrillary tangle formation. These properties make them potentially effective treatments for Alzheimer's disease and other neurodegenerative disorders, such as Huntington's disease, Alzheimer's disease, Parkinson's disease, frontotemporal dementia, multiple sclerosis, and traumatic nerve injury.

Conversely, the central nervous system is also affected by highly malignant, treatment-resistant tumors such as gliomas. These diseases typically arise in proliferative glial cells within the brain, and like most tumor cells, disordered cellular proliferation drives the transformation of pathological glial cells into malignant cancer cells. Therefore, microtubule stabilizers capable of crossing the blood-brain barrier, specifically targeting malignant glial cell proliferation while exhibiting minimal harm to neuronal cells, hold strong therapeutic potential for such central nervous system disorders.

The present invention discloses a novel class of compounds containing spiro and fused ring structures, which unexpectedly exhibit high microtubule-stabilizing activity.

### SUMMARY

The object of the present invention is to provide novel compounds containing spiro and fused ring structures with high microtubule-stabilizing activity.

Another object of the present invention is to provide preparation methods for said compounds.

In a first aspect of the present invention, a compound of general formula (I) or a stereoisomer, tautomer, pharmaceutically acceptable salt or prodrug thereof is provided: 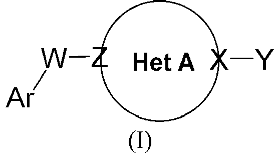 wherein,
Ar is: 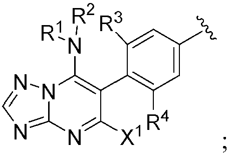
X¹ is Cl, CN, vinyl, -CH=CHC₁₋₆ alkyl, -CH=CHC₃₋₆ cycloalkyl,-C=CH, -C≡C₁₋₆ alkyl, -C≡CC₃₋₆ cycloalkyl, C₁₋₆ alkyl, C₁₋₆ haloalkyl, C₃₋₆ cycloalkyl, C₃₋₆ halocycloalkyl, OC₁₋₆ alkyl, OC₁₋₆ haloalkyl, SC₁₋₆ alkyl;
R¹ is C₁-C₁₀ alkyl, C₁-C₁₀ haloalkyl, C₃-C₁₀ cycloalkyl, C₃-C₁₀ heterocyclyl, C₃-C₁₀ halocycloalkyl,
or R¹ is: 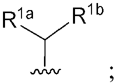
R^{1a} andR^{1b} are independently hydrogen, C₁-C₁₀ alkyl, C₁-C₁₀ haloalkyl, C₃-C₁₀ cycloalkyl, C₃-C₁₀ halocycloalkyl, C₇-C₁₁ spiroalkane, C₅-C₁₀ heterospirocycle, aryl, heteroaryl;
R² is hydrogen;
R³ and R⁴ are independently hydrogen, F, Cl, Br;
W is a chemical bond, -O(CR^{a}R^{b})ₙ-, -(CR^{a}R^{b})ₙO-, -S(CR^{a}R^{b})ₙ-, -(CR^{a}R^{b})ₙS-, -(CR^{a}R^{b})ₙ-NR^{e}-, - N(R^{e})-(CR^{a}R^{b})ₙ-, -C(O)N(R^{e})-(CR^{a}R^{b})ₙ-, -N(R^{e})C(O)-(CR^{a}R^{b})ₙ-, -(CR^{a}R^{b})ₙ-, -C(O)(CR^{a}R^{b})ₙ-, - (CR^{a}R^{b})ₙC(O)-, arylene, (*Z*)-CH=CH-, (*E*)-CH=CH-,-C≡C-, C₆₋₁₀ arylene, C₅-C₉ fused heteroarylene, 5- or 6-membered heteroarylene;
R^{a} and R^{b} are each independently hydrogen, substituted or unsubstituted C₁-C₁₀ alkyl, substituted or unsubstituted C₃-C₁₀ cycloalkyl, substituted or unsubstituted C₂-C₁₀ alkenyl, substituted or unsubstituted C₆-C₂₀ aryl, or substituted or unsubstituted C₃-C₁₄ heteroaryl; R^{a} and R^{b}, together with the carbon atom to which they are attached, may form a 3-8 membered ring or a 4-8 membered heterocycle, wherein the heteroatom may be sulfur, oxygen, NH, or NR^{e};
R^{c} and R^{d} are each independently hydrogen, C₁-C₁₀ alkyl, C₃-C₁₀ cycloalkyl, C₂-C₁₀ alkenyl, C₆-C₂₀ aryl, C₃-C₁₄ heteroaryl; R^{c} and R^{d} may be substituted by one or more groups selected from the group consisting of: halogen, hydroxyl, amino, nitro, cyano, aldehyde, carboxyl, alkoxy, -CF₃, - SF₅. R^{c} and R^{d}, together with the nitrogen atom to which they are attached, may form a 3-8 membered ring or a 4-8 membered heterocycle, wherein the heteroatom may be sulfur, oxygen, NH, or NR^{e};
R^{e} is hydrogen, C₁-C₆ alkyl, -(CR^{a}R^{b})ₙ-C₃-C₆ cycloalkyl, -(CR^{a}R^{b})ₙ-aryl, -(CR^{a}R^{b})ₙ-heteroaryl; R^{e} may be substituted with one or more groups selected from the group consisting of: halogen, hydroxy, amino, nitro, cyano, aldehyde, carboxyl, alkoxy, -CF₃, -SF₅.
Y isH, halogen, OR^{e}, -(CR^{a}R^{b})ₘ-CO₂H, -(CR^{a}R^{b})ₘ-CO(CR^{a}R^{b})ₙ-NR^{a}R^{b}, C₁-C₆ alkyl,-(CR^{a}R^{b})ₙ-C₃-C₆ cycloalkyl, -(CR^{a}R^{b})ₙ-aryl, -(CR^{a}R^{b})ₙ-heteroaryl, -(CR^{a}R^{b})ₙ-NR^{c}R^{d}, -O(CR^{a}R^{b})ₙ-NR^{c}R^{d},-S(CR^{a}R^{b})ₙ-NR^{c}R^{d}, -NR^{e}(CR^{a}R^{b})ₙ-NR^{c}R^{d}, -(CR^{a}R^{b})ₙ-P(O)Me₂, -(CR^{a}R^{b})ₙ-SO₂R^{a}, -(CR^{a}R^{b})ₙ-SO₂NR^{c}R^{d}, -(CR^{a}R^{b})ₙ-NR^{e}CONR^{c}R^{d}, -(CR^{a}R^{b})ₙ-CONR^{c}R^{d}.
m and n are independently integers from 0 or 1 to 6; 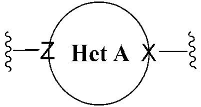 is monocyclichydrocarbyl, spiro hydrocarbyl, fused hydrocarbyl, bridged hydrocarbyl, monocyclic heterocyclyl, spiro heterocyclyl, fused heterocyclyl, or bridged heterocyclyl structures;
X and Z are independently C(R⁵), N;
R⁵ is hydrogen, OH, CN, halogen, NR^{c}R^{d}, C₁-C₁₀ alkyl, C₁-C₁₀ haloalkyl, -(CR^{a}R^{b})ₙ-C₃-C₁₀ cycloalkyl, -(CR^{a}R^{b})ₙ-C₃-C₁₀ halocycloalkyl, -(CR^{a}R^{b})ₙ-CO₂H, -(CR^{a}R^{b})ₙ-CONR^{c}R^{d};

In another preferred embodiment, said Ar is:

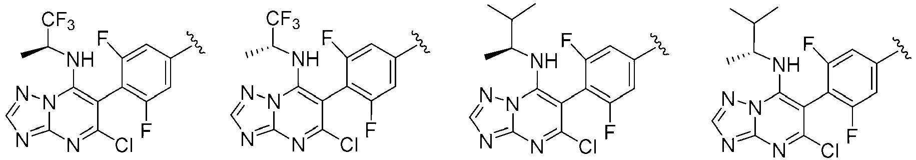

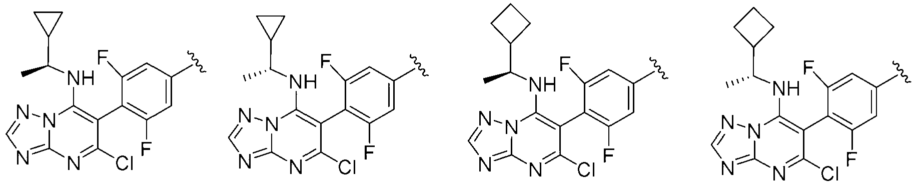

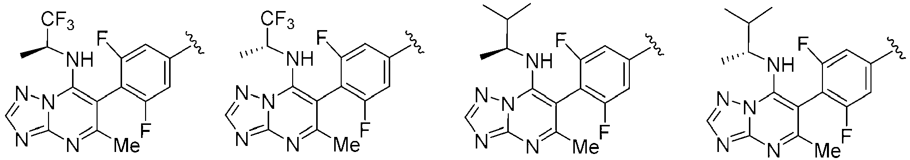

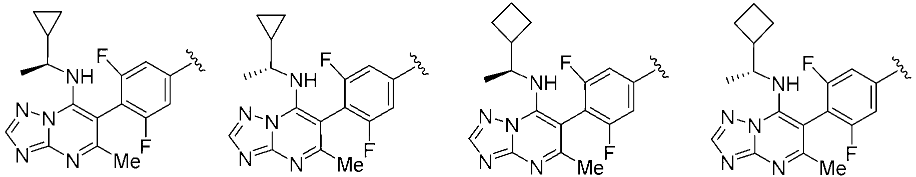

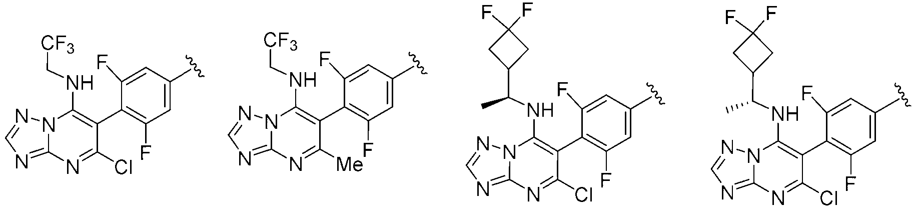

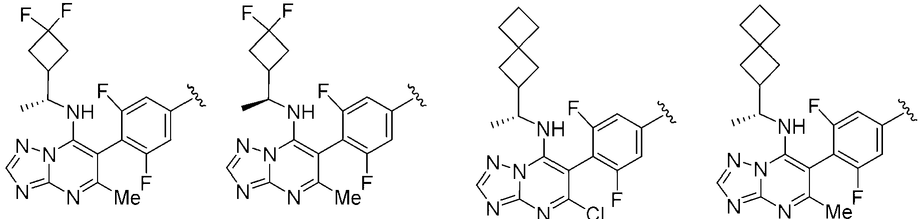

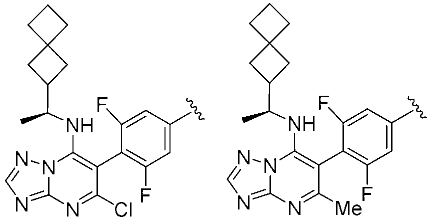

In another preferred embodiment, in general formula (I), 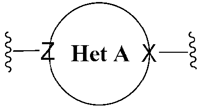 is:

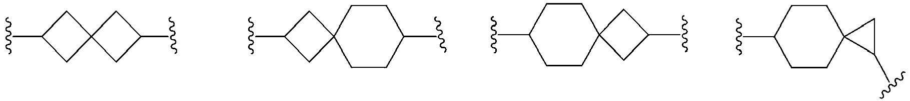

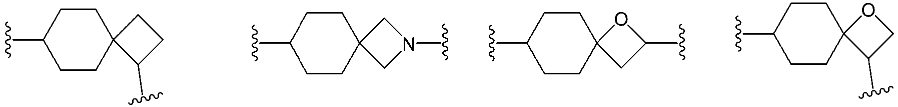

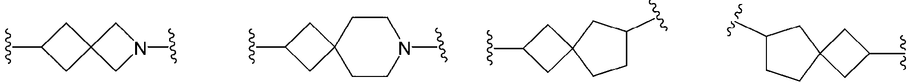

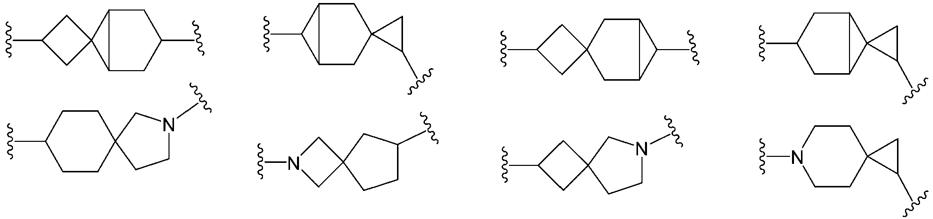

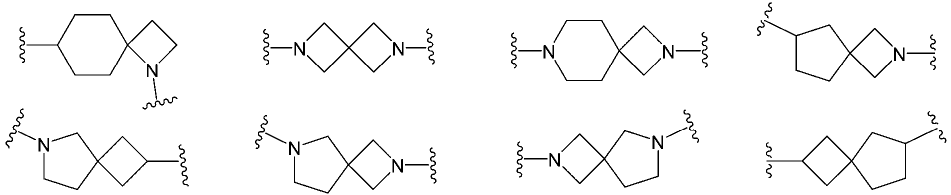

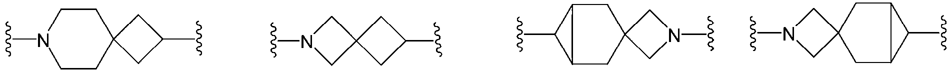

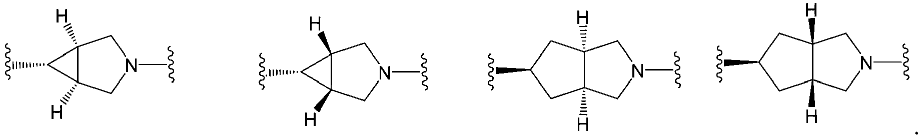

In another preferred embodiment, W in general formula (I) is a chemical bond or O.

In another preferred embodiment, W in general formula (I) is -C≡C-.

In another preferred embodiment, X in general formula (I) is N or CH.

In another preferred embodiment, Y in general formula (I) is H, C₁-C₆ alkyl, C₁-C₆ fluoroalkyl, -C₃-C₆ cycloalkyl, -(CR^{a}R^{b})ₙ-NR^{c}R^{d}, -SO₂R^{a}, -SO₂NR^{c}R^{d}, -CONR^{c}R^{d}, aryl or heteroaryl.
In another preferred embodiment, the compound of general formula (I) is represented by general formula (II): 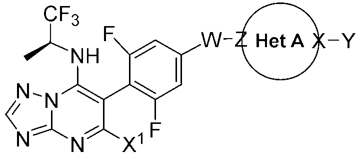 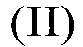 wherein X¹ is Cl, methyl, CF₃, ethyl, or cyclopropyl, and the definitions of W, Z, X, Y, and heterocycle A are as described in general formula (I).

In another preferred embodiment, the compound of general formula (I) is represented by general formula (III): 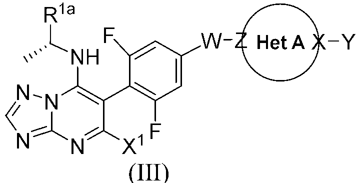
wherein X¹ is Cl, methyl, ethyl, or cyclopropyl, and the definitions of heterocycle A, W, Z, X, and Y are as described in general formula (I);
R^{1a} is CF₃,

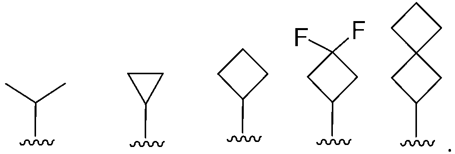

In another preferred embodiment, the compound of general formula (I) is represented by general formula (IV): 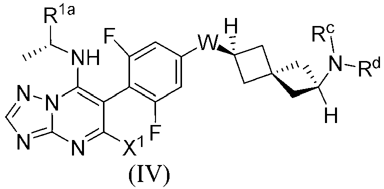 Wherein X¹ is Cl, methyl, CF₃, ethyl, or cyclopropyl, and the definitions of R^{1a}, R^{a}, R^{b}, R^{c}, R^{d} and R^{e} are as described in general formula (I); W is a chemical bond, (Z)-CH=CH-, (E)-CH=CH-, - C≡C-, -O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (V): 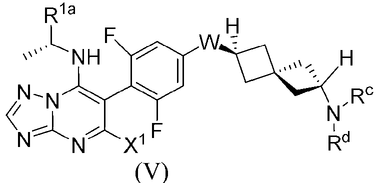 In the formula, X¹ is Cl, methyl, CF₃, ethyl, or cyclopropyl; R^{1a}, R^{a}, R^{b}, R^{c}, R^{d}, and R^{e} are as defined in general formula (I); W is a chemical bond, (Z)-CH=CH-, (E)-CH=CH-, -C≡C-, - O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (VI): 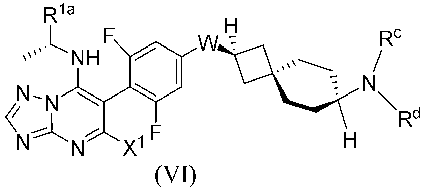 In the formula, X¹ is Cl, methyl, CF₃, ethyl, or cyclopropyl; R^{1a}, R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, - (CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (VII): 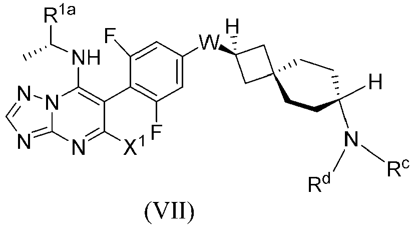 In the formula, X¹ is Cl, methyl, CF₃, ethyl, or cyclopropyl; R^{1a}, R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, - (CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (VIII): 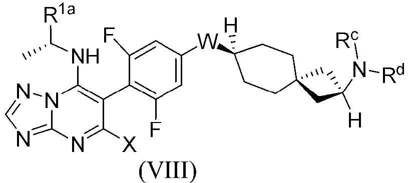 In the formula, X¹ is Cl, methyl, CF₃, ethyl, or cyclopropyl; R^{1a}, R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, - (CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (IX): 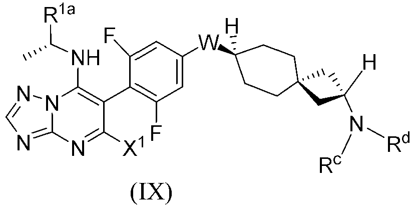 In the formula, X¹ is Cl, methyl, CF₃, ethyl, or cyclopropyl; R^{1a}, R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, - (CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (X): 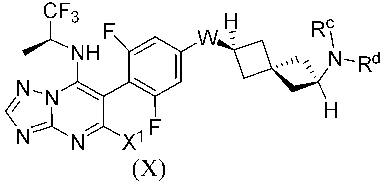 wherein X¹ is Cl, methyl, CF₃, ethyl, or cyclopropyl; R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (XI): 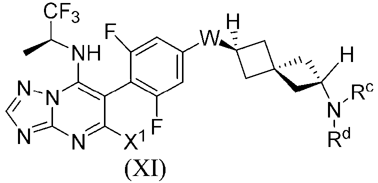 Wherein X¹ is Cl, methyl, CF₃, ethyl, or cyclopropyl; R^{a}, R^{b}, R^{c}, R^{d} and R^{e} are as defined in general formula (I); W is a chemical bond, (*Z*)-CH=CH-, (*E*)-CH=CH-, -C≡C-, -O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3. In another preferred embodiment, the compound of general formula (I) is represented by general formula (XII): 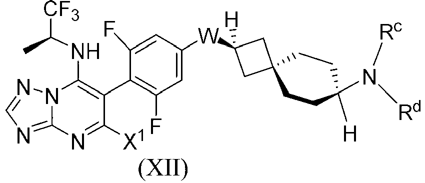 Wherein X¹ is Cl, methyl, CF₃, ethyl, or cyclopropyl; R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (XIII): 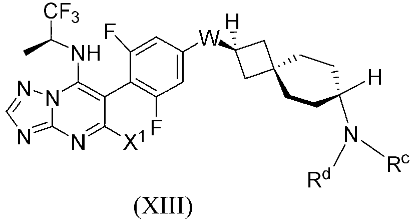 Wherein R^{a}, R^{b}, R^{c}, R^{d} and R^{e} are as defined in general formula (I); W is a chemical bond, (Z)-CH=CH-, (E)-CH=CH-, -C≡C-, -O(CR^{a}R^{b})ₙ-, -(CR^{a}R^{b})ₙO-, -(CR^{a}R^{b})ₙS-, -S(CR^{a}R^{b})ₙ-, - (CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (VIV): 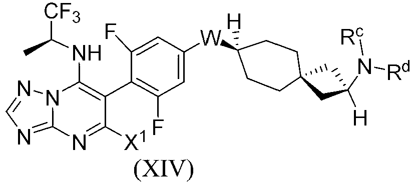 Wherein X¹ is Cl, methyl, CF₃, ethyl, or cyclopropyl; R^{a}, R^{b}, R^{c}, R^{d} and R^{e} are as defined in general formula (I);W is a chemical bond, (*Z*)-CH=CH-, (*E*)-CH=CH-, -C=C-, -O(CR^{a}R^{b})ₙ-, -(CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (XV): 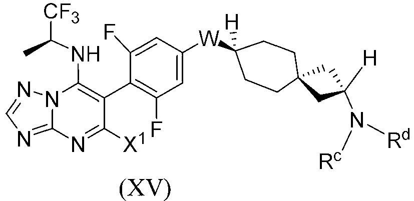 In the formula, X¹ is Cl, methyl, CF₃, ethyl, or cyclopropyl; R^{a}, R^{b}, R^{c}, R^{d} and R^{e} are as defined in general formula (I); W is a chemical bond, (*Z*)-CH=CH-, (*E*)-CH=CH-, -C=C-, -O(CR^{a}R^{b})ₙ-, - (CR^{a}R^{b})ₙO-, -(CR^{a}R^{b})ₙS-, -S(CR^{a}R^{b})ₙ-, -(CR^{a}R^{b})ₙNR^{a}-, or -NR^{e}(CR^{a}R^{b})ₙ-; n is 0 or an integer from 1 to 3.

In another preferred embodiment, the compound of general formula (I) is represented by general formula (XVI): 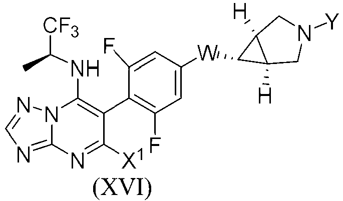 In the formula, X¹ is Cl, methyl, CF₃, ethyl, or cyclopropyl; W is a chemical bond, -O(CR^{a}R^{b})ₙ-, - (CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙNR^{e}-, (*Z*)-CH=CH-, (*E*)-CH=CH-, -C=C-; R^{a}, R^{b}, Y and R^{e} are as defined in general formula (I); n is 0 or an integer from 1 to 6.

In another preferred embodiment, the compound of general formula (I) is represented by general formula (XVII): 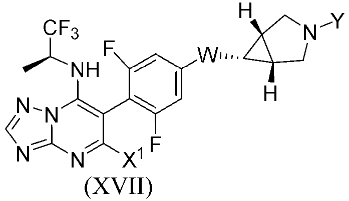 In the formula, X¹ is Cl, methyl, CF₃, ethyl, or cyclopropyl; W is a chemical bond, -O(CR^{a}R^{b})ₙ-, - (CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙNR^{e}-, (*Z*)-CH=CH-, (*E*)-CH=CH-, -C=C-; R^{a}, R^{b}, Y and R^{e} are as defined in general formula (I); n is 0 or an integer from 1 to 6.

In another preferred embodiment, the compound of general formula (I) is represented by general formula (XVIII): 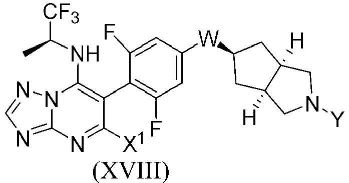 In the formula, X¹ is Cl, methyl, CF₃, ethyl, or cyclopropyl; Y is as defined in general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, - (CR^{a}R^{b})ₙNR^{e}-, (Z)-CH=CH-, (E)-CH=CH-, -C=C-; R^{a}, R^{b}, Y and R^{e} are as defined in general formula (I); n is 0 or an integer from 1 to 6; R^{a}, R^{b}, Y and R^{e} are as defined in general formula (I); n is 0 or an integer from 1 to 6.

In another preferred embodiment, the compound of general formula (I) is represented by general formula (XIX): 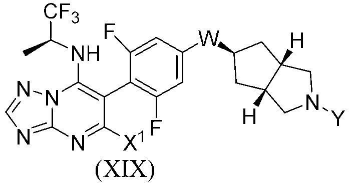 In the formula, X¹ is Cl, methyl, CF₃, ethyl, or cyclopropyl; Y is as defined in general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, - (CR^{a}R^{b})ₙNR^{e}-, (Z)-CH=CH-, (*E*)-CH=CH-, -C=C-; R^{a}, R^{b}, Y and R^{e} are as defined in general formula (I); n is 0 or an integer from 1 to 6; n is 0 or an integer from 1 to 6.

In another preferred embodiment, the compound of general formula (I) is represented by general formula (XX): 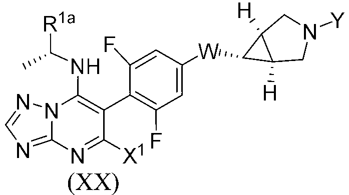
Wherein, X¹ is Cl, methyl, CF₃, ethyl, or cyclopropyl, and Y is as defined in the general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙ NR^{e}-, (*Z*)-CH=CH-, (*E*)-CH=CH-, -C=C-; R^{a}, R^{b}, Y and R^{e} are as defined in the general formula (I); n is 0 or an integer from 1 to 6;
R^{1a} is CF₃,

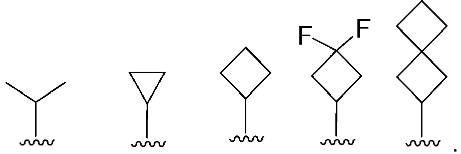

In another preferred embodiment, the compound of the general formula (I) is represented by the general formula (XXI): 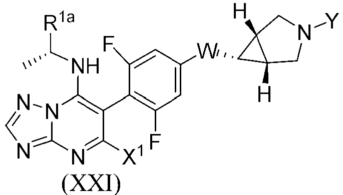
Wherein, X¹ is Cl, methyl, CF₃, ethyl, or cyclopropyl, and Y is as defined in the general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙ NR^{e}-, (Z)-CH=CH-, (E)-CH=CH-, -C≡C-; n is 0 or an integer from 1 to 6;
R^{1a} is CF₃,

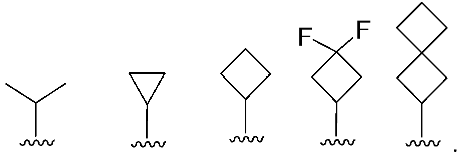

In another preferred embodiment, the compound of the general formula (I) is represented by the general formula (XXII): 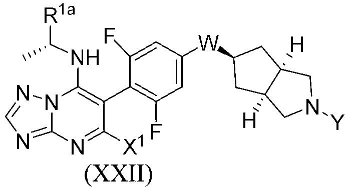
Wherein, X¹ is Cl, methyl, CF₃, ethyl, or cyclopropyl, and Y is as defined in the general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙ NR^{e}-, (*Z*)-CH=CH-, (*E*)-CH=CH-, -C≡C-; n is 0 or an integer from 1 to 6;
R^{1a} is CF,

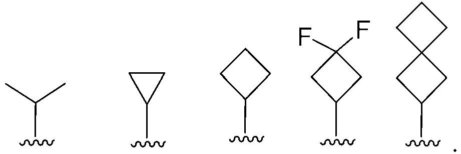

In another preferred embodiment, the compound of the general formula (I) is represented by the general formula (XXIII): 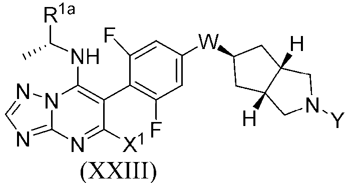
Wherein, X¹ is Cl, methyl, CF₃, ethyl, or cyclopropyl, and Y is as defined in the general formula (I); W is a chemical bond, -O(CR^{a}R^{b})ₙ-, -(CR^{a}R^{b})ₙO-, -S(CR^{a}R^{b})ₙ-, -(CR^{a}R^{b})ₙS-, -NR^{e}-(CR^{a}R^{b})ₙ-, -(CR^{a}R^{b})ₙ-NR^{e}-, (*Z*)-CH=CH-, (*E*)-CH=CH-, -C≡C-; n is 0 or an integer from 1 to 3;
R^{1a} is CF₃,

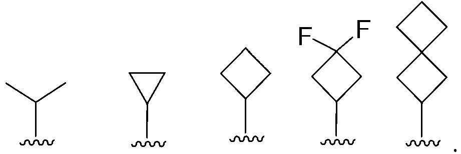

In another preferred embodiment, the compounds of the above general formulas (I) to (XXIII) are:

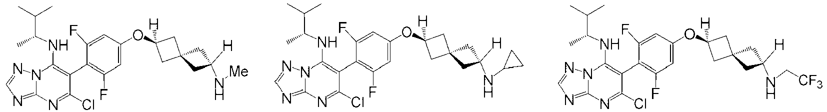

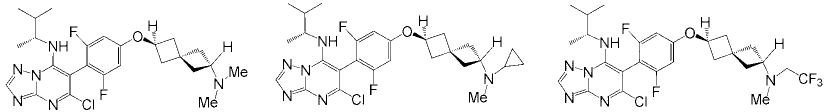

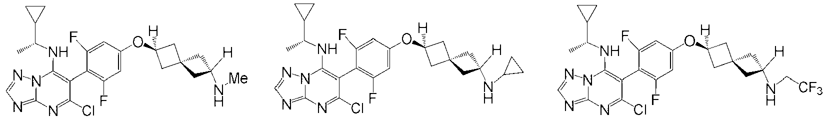

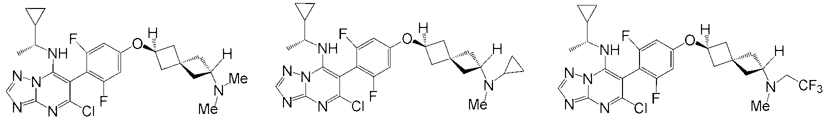

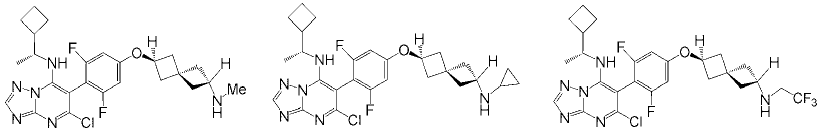

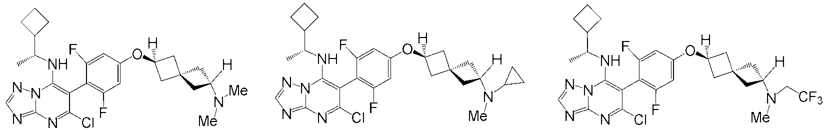

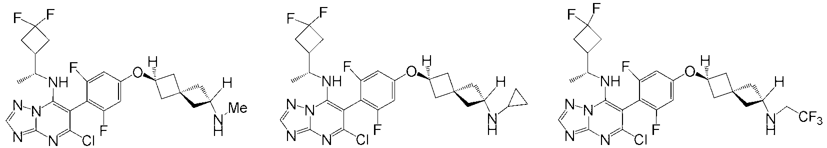

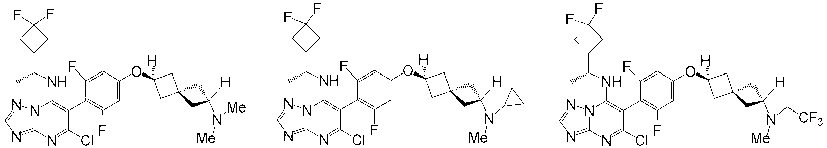

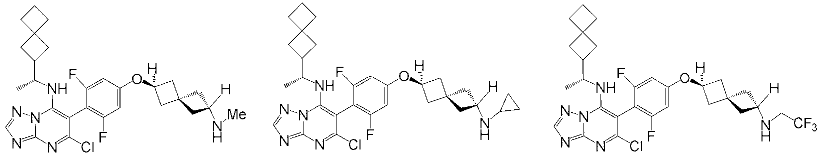

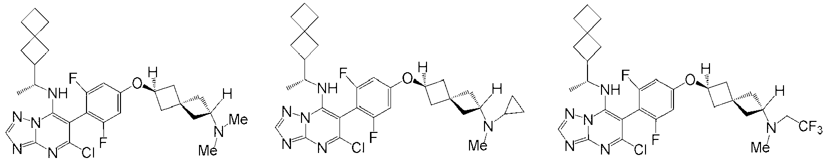

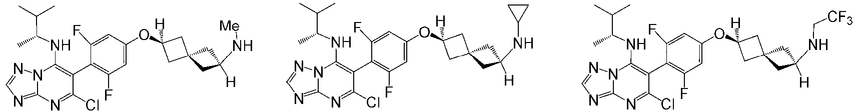

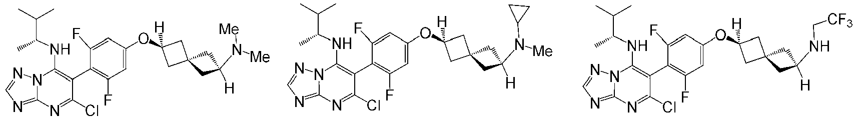

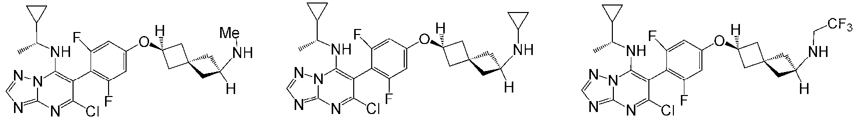

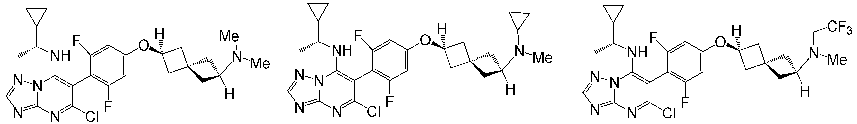

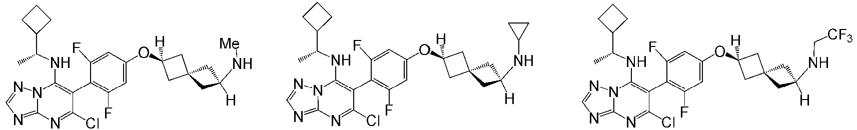

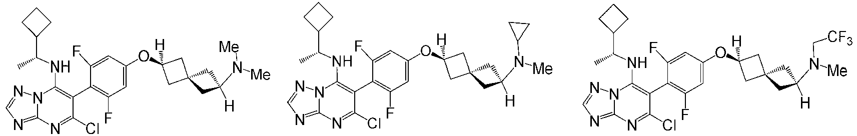

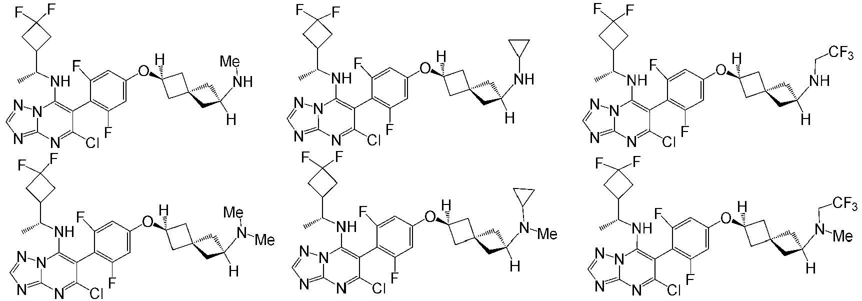

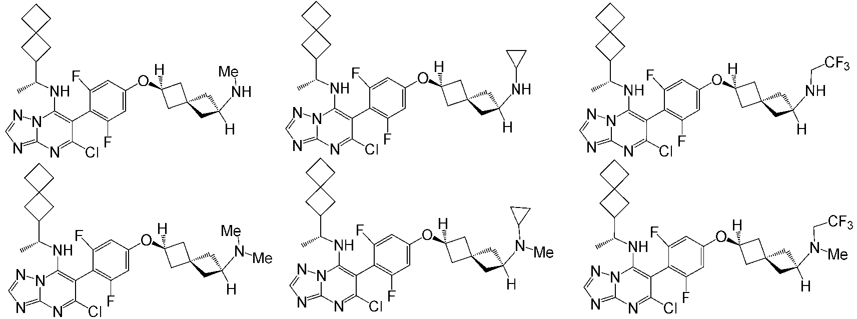

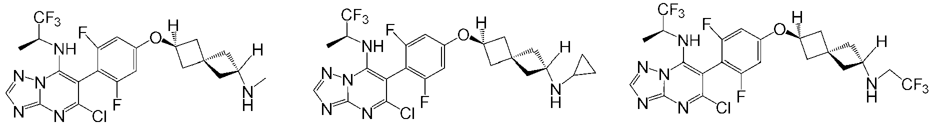

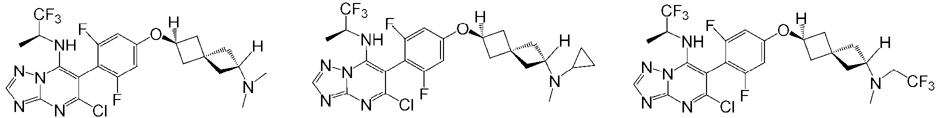

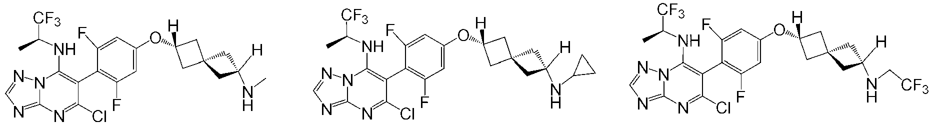

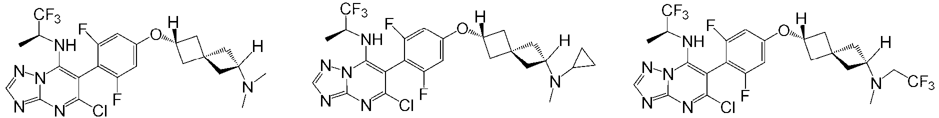

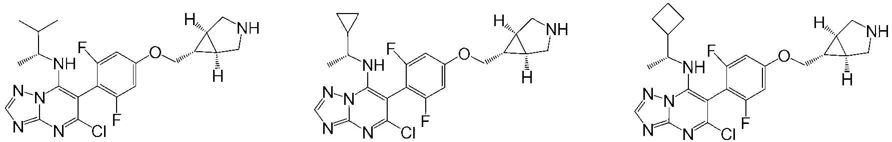

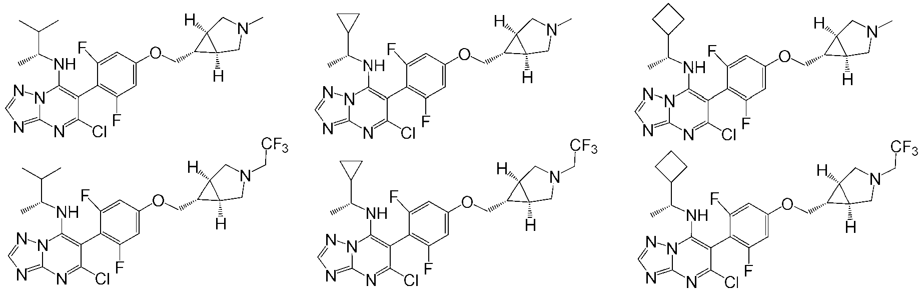

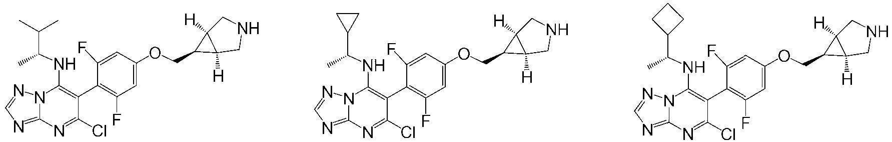

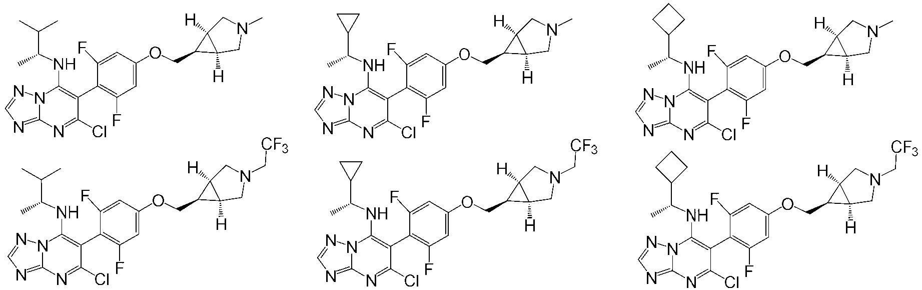

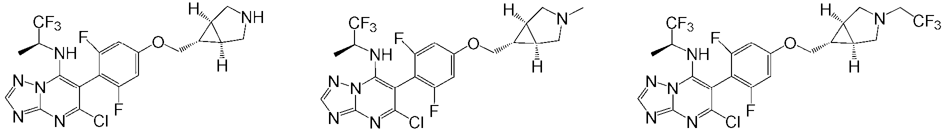

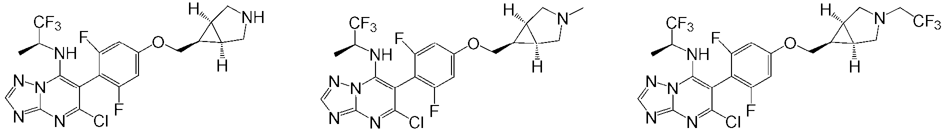

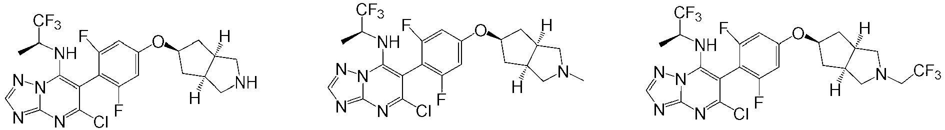

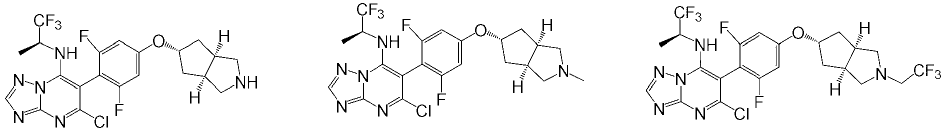

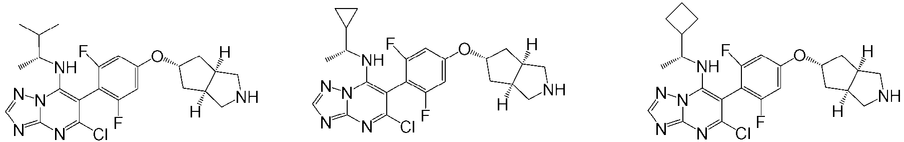

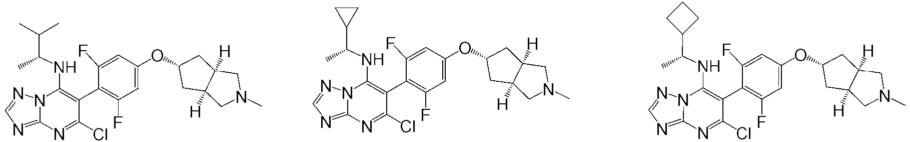

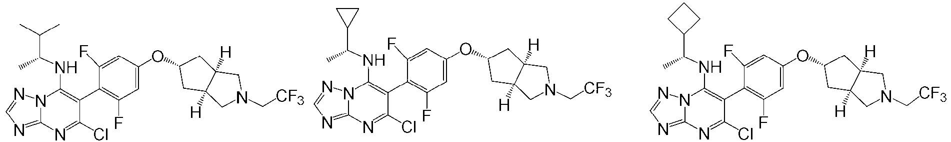

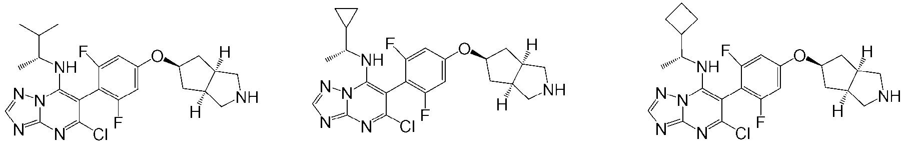

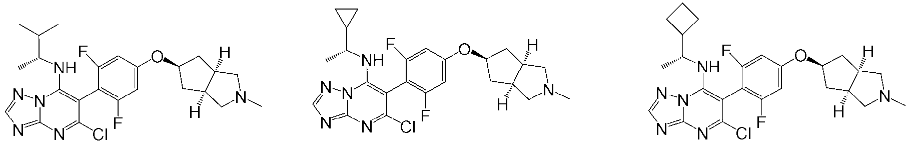

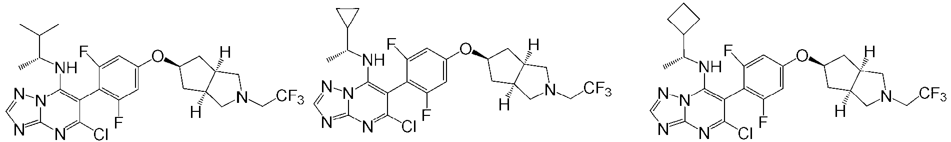

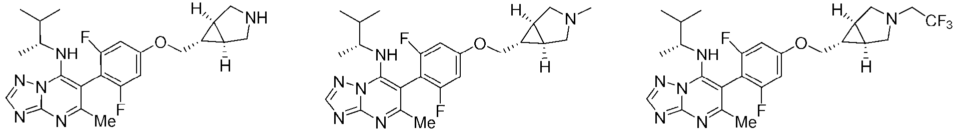

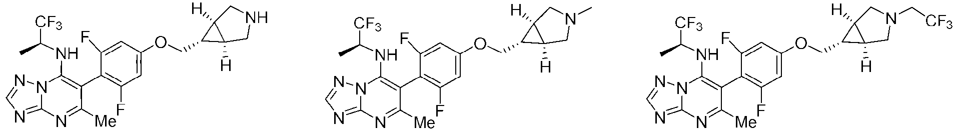

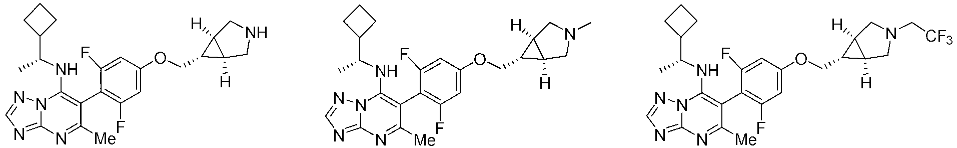

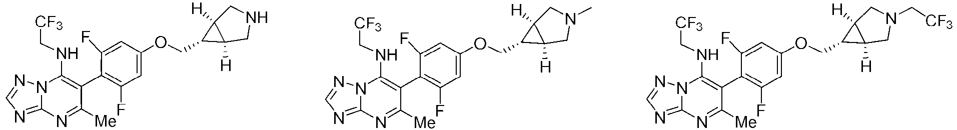

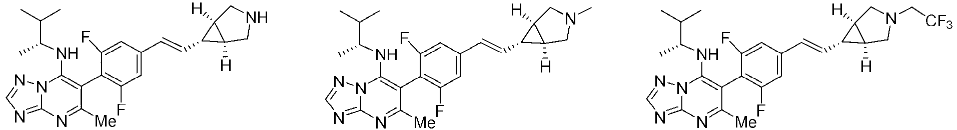

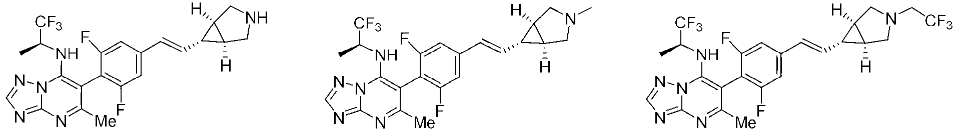

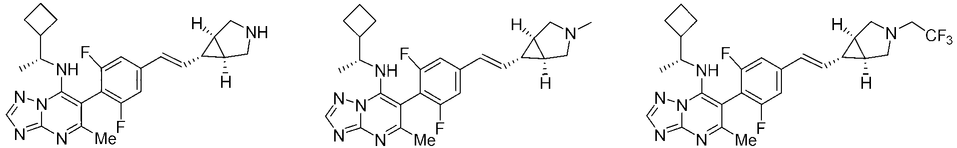

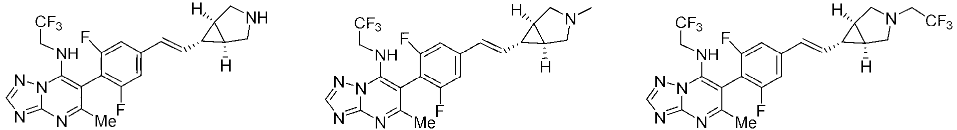

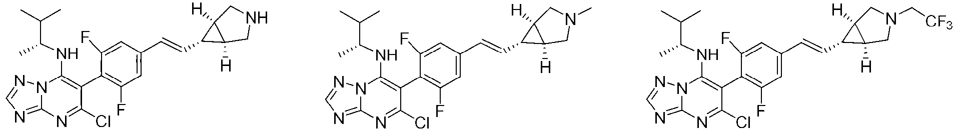

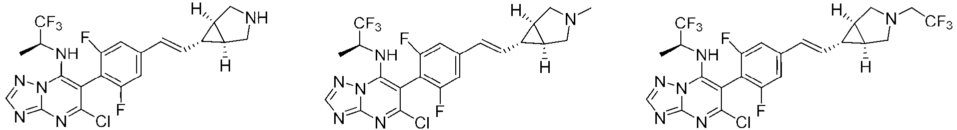

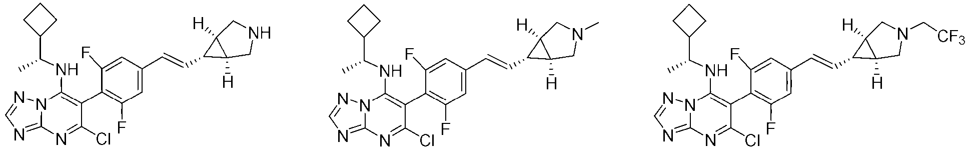

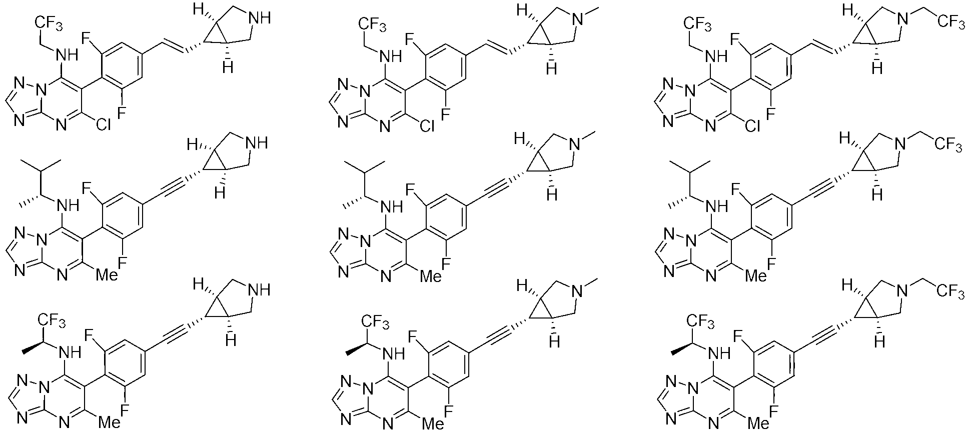

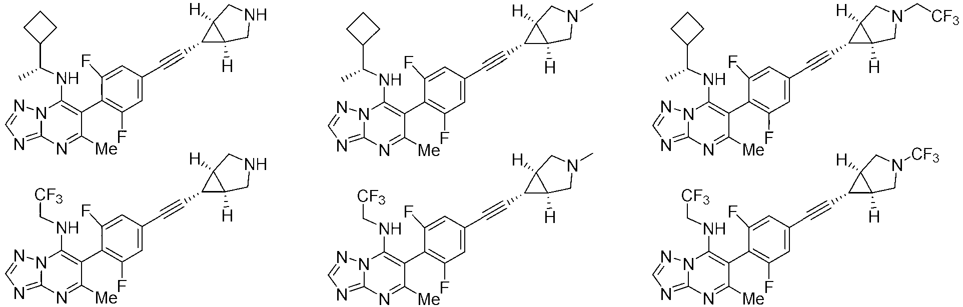

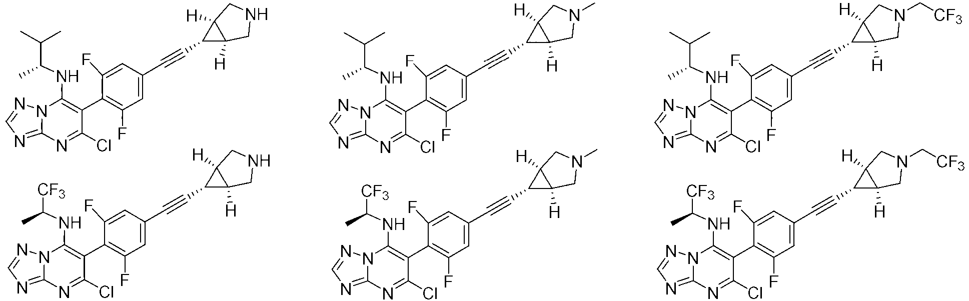

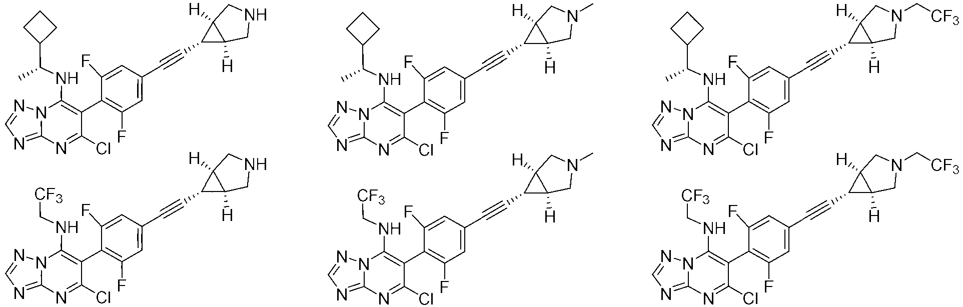

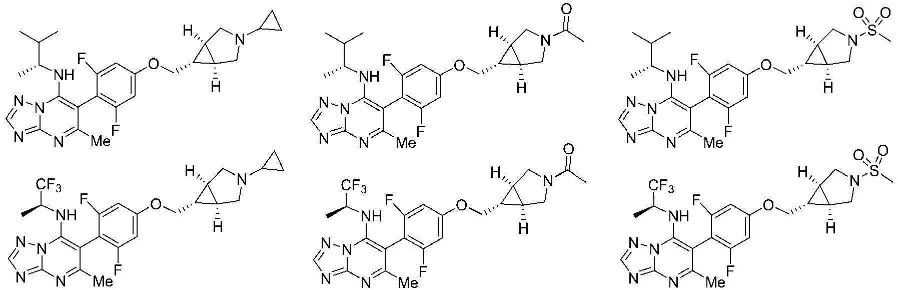

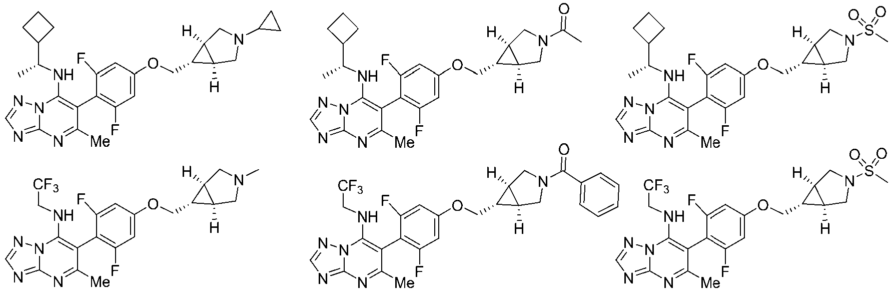

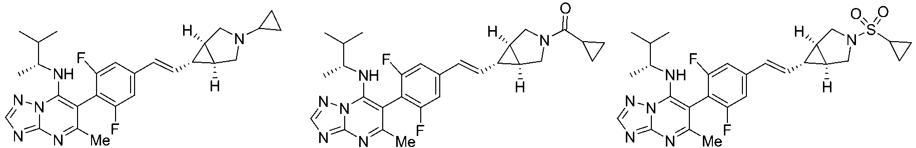

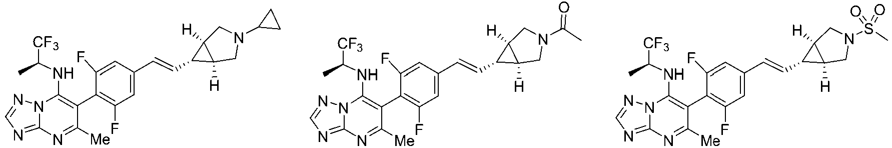

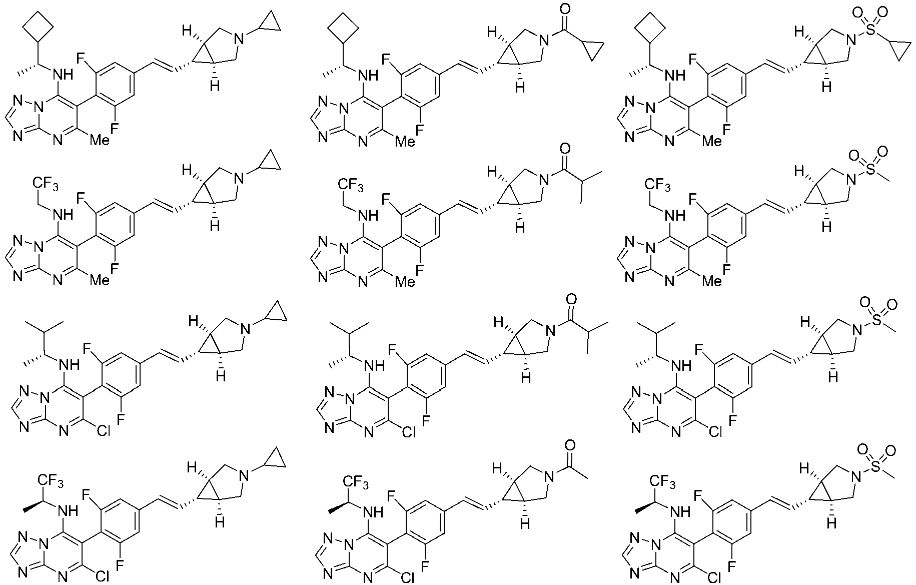

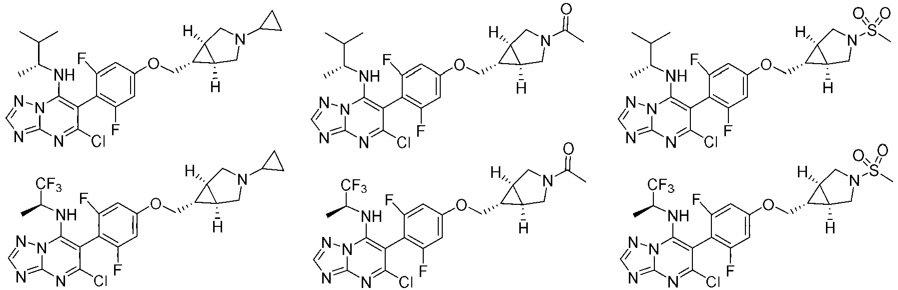

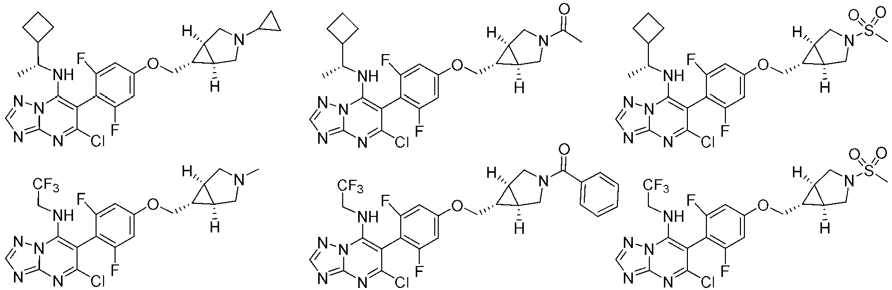

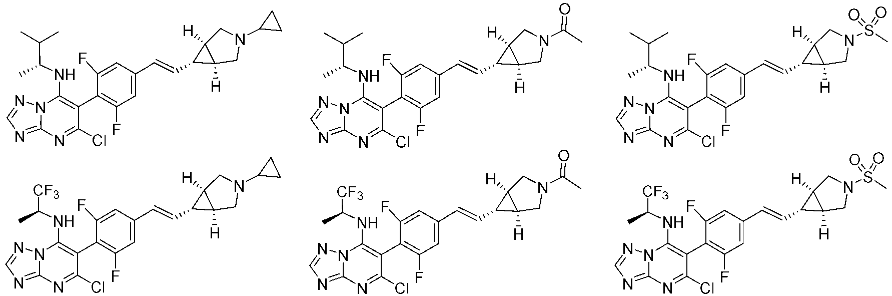

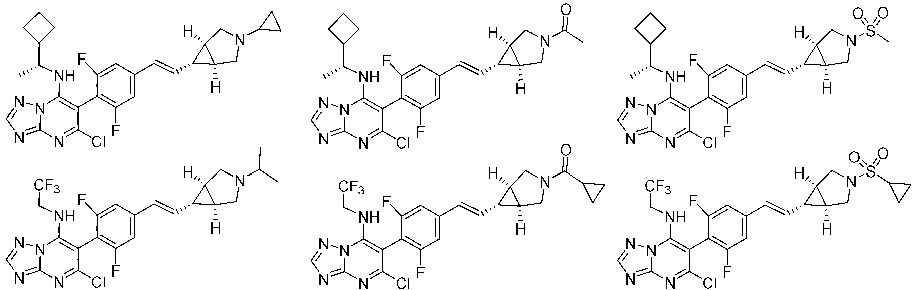

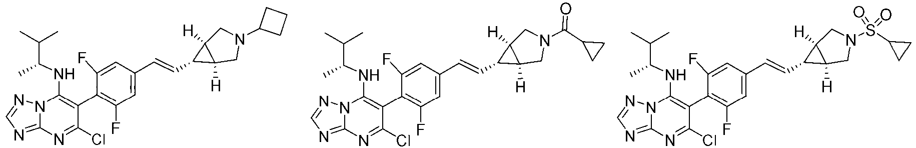

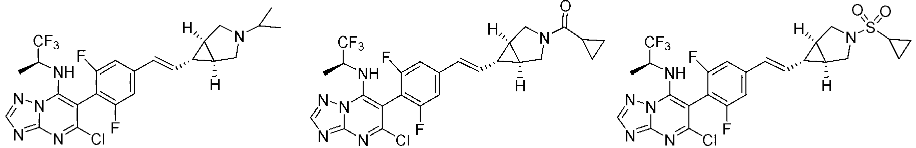

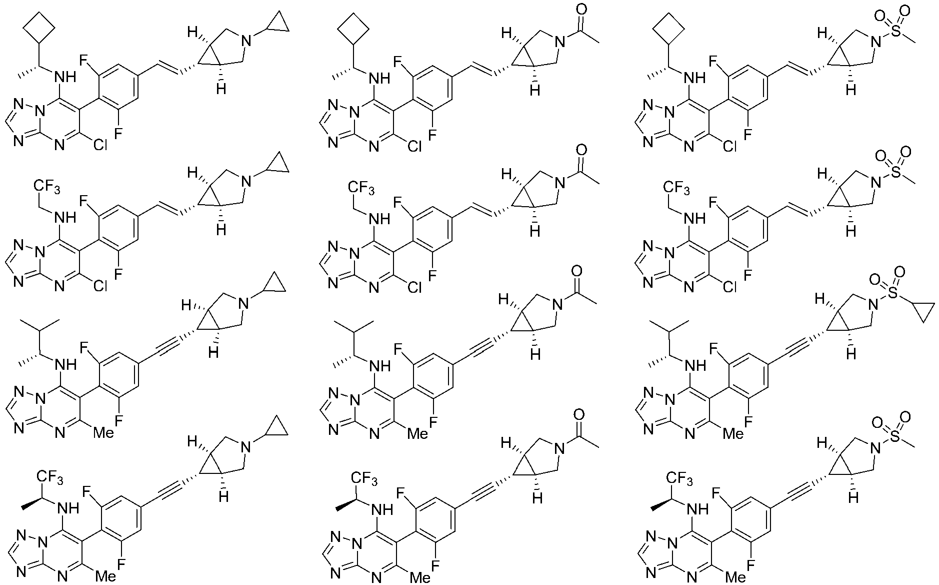

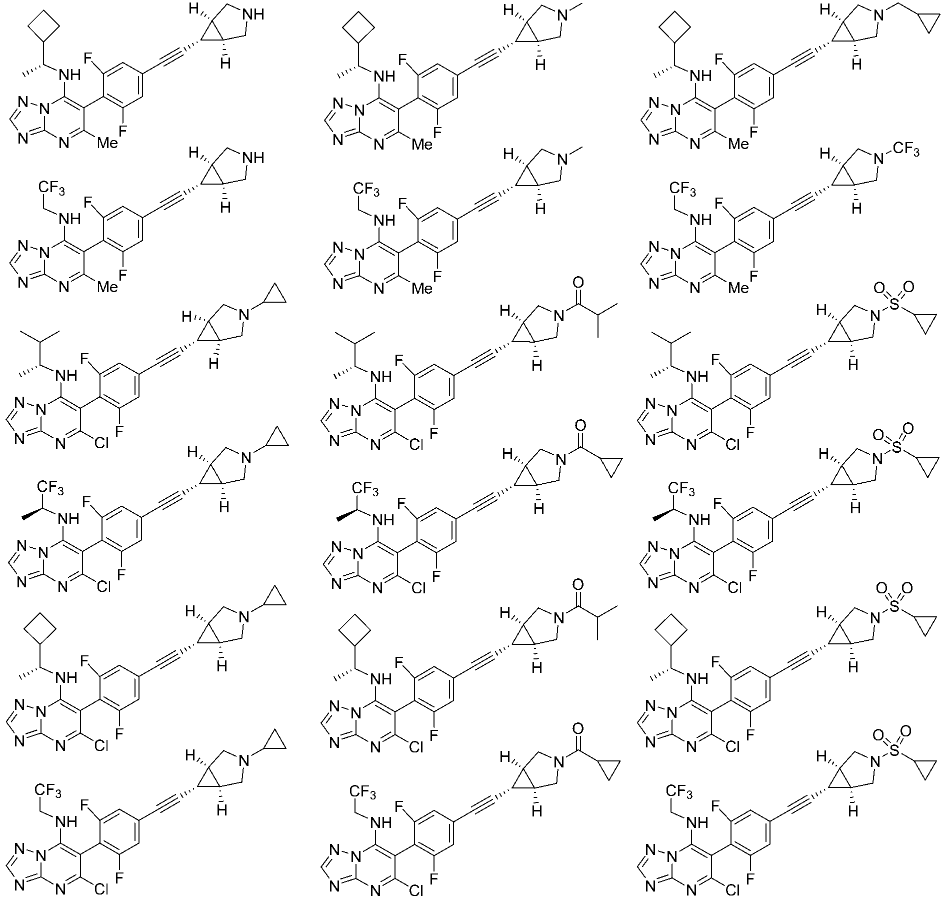

In another preferred embodiment, the stereoisomer is a cis-trans isomer.

In another preferred embodiment, the compound is a racemate.

In another preferred embodiment, the stereoisomer is an enantiomer.

In another preferred embodiment, any one or more hydrogens in the compound may be substituted with deuterium.

In another preferred embodiment, the pharmaceutically acceptable salt is selected from the group consisting of: hydrochloride, hydrobromide, sulfate, phosphate, methanesulfonate, trifluoromethanesulfonate, benzenesulfonate, p-toluenesulfonate (tosylate), 1-naphthalenesulfonate, 2-naphthalenesulfonate, acetate, trifluoroacetate, malate, tartrate, citrate, lactate, oxalate, succinate, fumarate, maleate, benzoate, salicylate, phenylacetate, and mandelate.

The compound of general formula (I) of the present invention can be obtained by the following preparation method, comprising the steps of:

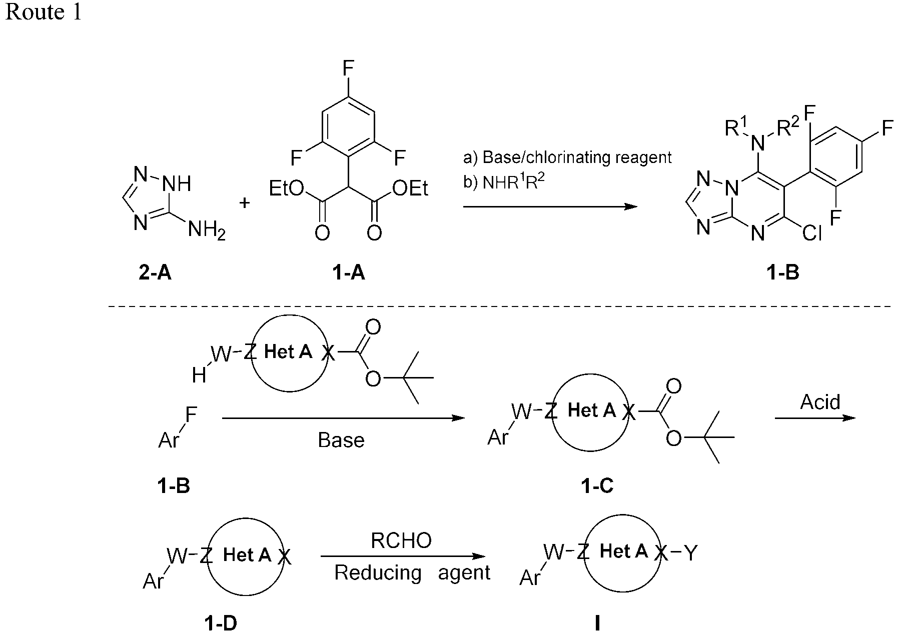

In the formula, R is C₁-C₆ alkyl or C₁-C₆ haloalkyl; Ar, X, W, Z, and Y are as defined above.

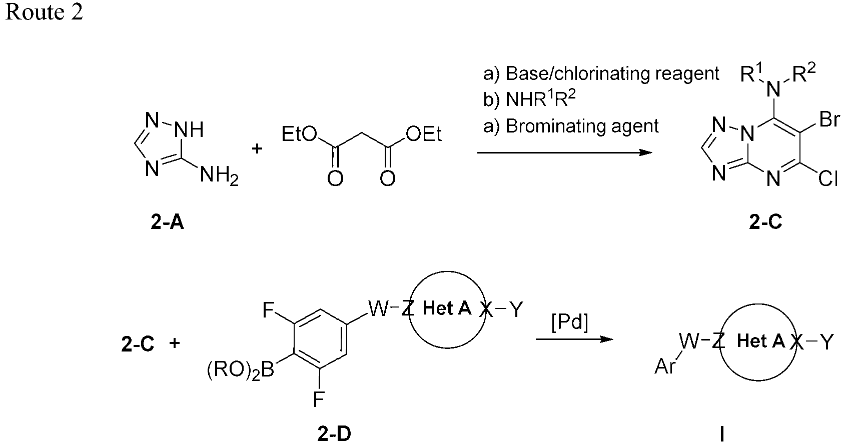

In the formula, R is OH or (RO)₂ is (OCMe₂CMe₂O); Ar, R¹, R², X, W, Z, and Y are as defined above.

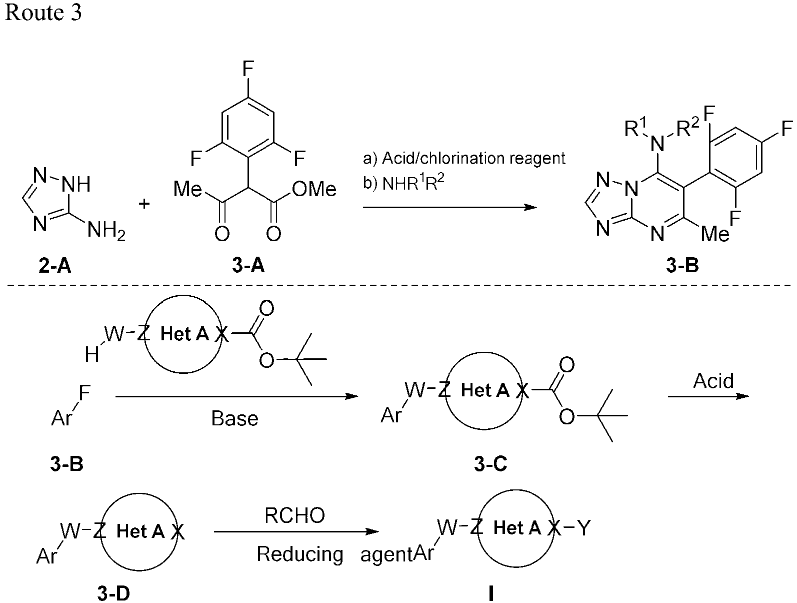

In the formula, Ar, R¹, R², X, W, Z, Y are as defined above.

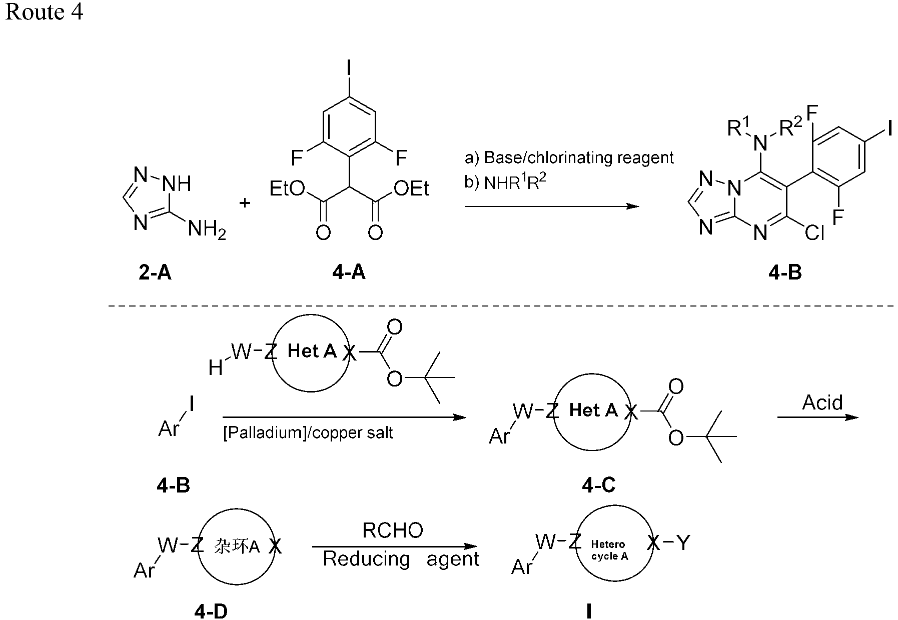

In the formula, Ar, R¹, R², X, W, Z, Y are as defined above.

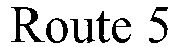

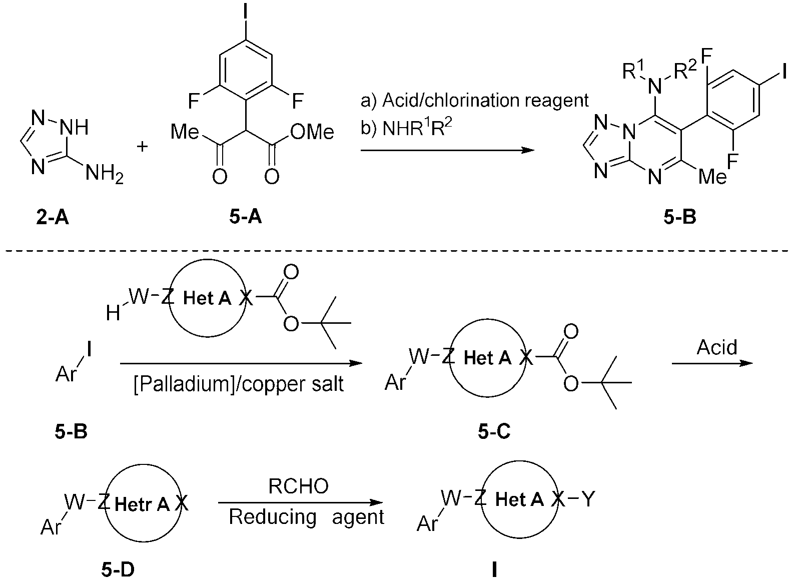

In the formula, Ar, R¹, R², X, W, Z, Y are as defined above.

In the above methods, the base may be selected from the group consisting of: alkali metal hydroxides, alkaline earth metal hydroxides, alkali metal hydrides, alkaline earth metal hydrides, alkali metal (hydrogen)carbonates, alkaline earth metal carbonates, bis(trimethylsilyl)amido alkali metal salts, pyridine, triethylamine, diisopropylethylamine, and the like.

The acid may be selected from the group consisting of: hydrochloric acid, sulfuric acid, trifluoroacetic acid, formic acid, and the like.

The reducing agent may be selected from the group consisting of: lithium aluminum hydride, sodium borohydride, sodium cyanoborohydride, lithium borohydride, borane, sodium triacetoxyborohydride, and the like.

The palladium catalyst may be selected from the group consisting of: tetrakis(triphenylphosphine) palladium, [1,1'-bis(diphenylphosphino)ferrocene]palladium(II) dichloride dichloromethane complex, and the like.

The copper salt may be selected from the group consisting of: copper(I) chloride, copper(I) bromide, copper(I) iodide, copper(I) cyanide, and the like.

The chlorinating agent may be selected from the group consisting of: N-chlorosuccinimide, phosphorus oxychloride, thionyl chloride, and the like.

The brominating agent may be selected from the group consisting of: liquid bromine, N-bromosuccinimide, phosphorus tribromide, and the like.

Another aspect of the present invention provides the use of the compound of formula (I) as described in the first aspect, or a stereoisomer, tautomer, pharmaceutically acceptable salt or prodrug thereof, to:
(i) prepare a microtubule stabilizer;
(ii) microtubule-mediated diseases;
(iii) prepare a medicament for preventing and/or treating cancer and neurodegenerative diseases.

In another preferred embodiment, the cancer includes but is not limited to: glioma, colon cancer, breast cancer, gastric cancer, lung cancer, colorectal cancer, pancreatic cancer, ovarian cancer, prostate cancer, kidney cancer, liver cancer, brain cancer, melanoma, multiple myeloma, chronic myeloid leukemia, hematologic tumors, lymphomas, including metastatic lesions in tissues or organs distant from the primary tumor site.

In another preferred embodiment, the neurodegenerative disease includes but is not limited to: Huntington's disease, Alzheimer's disease, Parkinson's disease, multiple sclerosis, and traumatic brain injury.

Another aspect of the present invention provides a pharmaceutical composition comprising: other antitumor drugs, such as PD-1 antibodies, PD-L1 antibodies, CTLA-4 antibodies, other antitumor chemotherapeutic drugs, and other targeted drugs.

It should be understood that, within the scope of the present disclosure, the aforementioned technical features of the present disclosure and the technical features specifically described hereinafter (e.g., in the examples) can be combined with each other to constitute new or preferred technical solutions. Due to space limitations, they will not be elaborately enumerated here.

### DETAILED DESCRIPTION OF THE INVENTION

After extensive and in-depth research, the inventors have discovered a novel class of polycyclic compounds that can serve as efficient microtubule stabilizers for the prevention and/or treatment of microtubule-mediated diseases, thereby completing the present invention.

### Definitions

The term "C₁-C₁₀ alkyl" refers to a monovalent saturated aliphatic hydrocarbon group having 1 to 10 carbon atoms, including straight-chain and branched-chain hydrocarbon groups, such as methyl (i.e., CH₃-), ethyl (i.e., CH₃CH₂-), n-propyl (i.e., CH₃CH₂CH₂-), isopropyl (i.e., (CH₃)₂CH-), n-butyl (i.e., CH₃CH₂CH₂CH₂-), isobutyl (i.e., (CH₃)₂CHCH₂-), sec-butyl (i.e., (CH₃)(CH₃CH₂)CH-), tert-butyl (i.e., (CH₃)₃C-), n-pentyl (i.e., CH₃CH₂CH₂CH₂CH₂-), and neopentyl (i.e., (CH₃)₃CCH₂-). In the present invention, this term includes substituted or unsubstituted alkyl groups.

As used herein, the term "substituted or unsubstituted" means that the group may be unsubstituted, or the H in the group may be replaced by one or more (preferably 1-6, more preferably 1-3) substituents.

As used herein, "substituted" or "substituted with" means that the group has one or more (preferably 1-6, more preferably 1-3) substituents selected from the group consisting of: halogen, hydroxy, -NH₂, nitro, -CN, C₁-C₆ alkyl, C₁-C₆ haloalkyl, C₁-C₆ alkoxy, C₃-C₆ cycloalkyl, C₂-C₆ alkenyl, C₂-C₆ alkynyl, phenyl, benzyl, C₁-C₆ alkylS(O)₂-, (C₀-C₆ alkyl)₂NS(O)₂-, C₁-C₆ alkylC(O)-, C₃-C₆ cycloalkylC(O)-, C₀-C₆ alkylOC(O)-, (C₀-C₆ alkyl)₂NC(O)-, C₀-C₆ alkylC(O)NH-, (C₀-C₆ alkyl)₂NC(O)NH-.

As used herein, the term "alkoxy" refers to -O-alkyl, wherein the alkyl group may be saturated or unsaturated, branched, straight-chain, or cyclic. Preferably, the alkoxy group has 1-10 carbon atoms, i.e., C₁-C₁₀ alkoxy, more preferably 1-6 carbon atoms. Representative examples include (but not limited to): methoxy, ethoxy, and propoxy.

As used herein, the term "C₆-C₂₀ aryl" refers to a monovalent aromatic carbocyclic group having 6 to 20 (preferably 6-14) carbon atoms, which may be monocyclic (e.g., phenyl) or polycyclic (e.g., naphthyl or anthryl). If the attachment point is on an aromatic carbon atom, the polycyclic ring may be non-aromatic (e.g., 2-benzoxazolone, 2*H*-1,4-benzoxazin-3(4*H*)-one-7-yl, etc.). Preferably, an aryl group includes phenyl and naphthyl. This term encompasses both substituted and unsubstituted forms, wherein the substituents are as defined above.

As used herein, the term "C₂-C₁₀ alkenyl" refers to an alkenyl group having 2 to 10 (e.g., 2 to 6 or 2 to 4) carbon atoms and at least 1 (e.g., 1 to 2) unsaturated alkenyl bond (>C=C<). Examples of such groups include vinyl, allyl, and but-3-enyl.

As used herein, the term "C₃-C₁₀ cycloalkyl" refers to a cyclic alkyl group having 3 to 10 carbon atoms and a monocyclic or polycyclic structure (including fused systems, bridged cycloalkane systems, and spiro cycloalkane systems). In the fused ring systems, one or more rings may be cycloalkyl, heterocyclic, aryl, or heteroaryl, provided that the attachment point is through the cycloalkyl ring. Examples of suitable cycloalkyl include, for instance, adamantyl, cyclopropyl, cyclobutyl, cyclopentyl, and cyclooctyl. "Spirocycloalkane" or "spirocycloalkyl" refers to a polycyclic group of 5- to 20-membered rings sharing one carbon atom (referred to as the spiro atom) between monocycles, which may contain one or more double bonds, preferably 6- to 14-membered, more preferably 7- to 10-membered (e.g., 7, 8, 9, or 10).

As used herein, the term "halo" or "halogen" refers to fluorine, chlorine, bromine, and iodine.

As used herein, the term "heteroaryl" or "heteroaryl group" refers to an aromatic group having 1 to 10 carbon atoms and 1 to 4 heteroatoms selected from oxygen, nitrogen, and sulfur in the ring. For terms indicating the number of carbon atoms, for example, "C₃-C₂₀ heteroaryl", it denotes a heteroaryl group with 3-20 carbon atoms and 1 to 4 heteroatoms selected from oxygen, nitrogen, and sulfur in the aromatic ring. Other terms follow similarly. Such heteroaryl groups may be monocyclic (e.g., pyridyl or furyl) or fused rings (e.g., indolizinyl or benzothienyl), wherein the fused ring may be non-aromatic and/or contain one heteroatom, provided that the attachment point is through an atom of the aromatic heteroaryl. In one embodiment, the ring atoms such as nitrogen and/or sulfur of the heteroaryl group are optionally oxidized to N-oxide (N-O), sulfinyl, or sulfonyl. Preferably, the heteroaryl group includes pyridinyl, pyrrolyl, indolyl, oxazolyl, thiazolyl, pyrazolyl, imidazolyl, triazolyl, tetrazolyl, thienyl, and furanyl. This term encompasses both substituted and unsubstituted heteroaryl groups.

As used herein, the term "substituted heteroaryl" refers to a heteroaryl group substituted with 1 to 5, preferably 1 to 3, more preferably 1 to 2 substituents selected from the same substituents as those defined for the substituted aryl.

As used herein, the terms "heterocycle", "heterocyclic", "heterocycloalkyl", or "heterocyclyl" refer to saturated, partially saturated, or unsaturated (but non-aromatic) groups having a single or fused ring structure (including spirocyclic, fused hydrocarbon, bridged hydrocarbon, monocyclic heterocycle, spiroheterocycle, fused heterocycle, or bridged heterocycle), containing 1 to 10 carbon atoms and 1 to 4 (such as 3) heteroatoms selected from nitrogen, sulfur or oxygen in the ring. In fused-ring systems one or more rings may be cycloalkyl, aryl or heteroaryl, provided that the point of attachment is through a non-aromatic ring. Heterospirocyclic compounds are formed by replacing carbon atoms in carbospirocyclic compounds with heteroatoms (e.g., O, S, N, or NR^{e}). In one embodiment, the nitrogen and/or sulfur atoms of the heterocyclic group are optionally oxidized to provide N-oxide, sulfinyl, or sulfonyl moieties.

As used herein, the terms "substituted heterocyclic", "substituted heterocycloalkyl" or "substituted heterocyclyl" refer to a heterocyclic group substituted with 1 to 5 (e.g., 1 to 3) substituents, which are the same as those defined for the substituted cycloalkyl.

As used herein, the term "stereoisomer" refers to compounds that differ in chirality at one or more stereocenters. Stereoisomers include enantiomers and diastereomers.

As used herein, the term "tautomer" refers to alternative forms of a compound differing in proton position, such as enol-keto and imine-enamine tautomers, or tautomeric forms of a heteroaryl group containing a ring atom connected to both the -NH- and =N- moieties of the ring, such as pyrazole, imidazole, benzimidazole, triazole, and tetrazole.

Unless otherwise specified, the monocyclic hydrocarbyl, spiro hydrocarbyl, fused hydrocarbyl, bridged hydrocarbyl, monocyclic heterocyclyl, spiro heterocyclyl, fused heterocyclyl or bridged heterocyclyl has 5 to 20 ring skeletal atoms, and when the ring skeletal atoms contain heteroatoms, the heteroatoms may be 1 to 4 (such as 3) heteroatoms selected from nitrogen, sulfur or oxygen.

The term "prodrug" refers to any derivative of the exemplified compounds that, when administered to a subject, is capable of directly or indirectly providing the exemplified compound or active metabolites or residues thereof. Particularly preferred derivatives and prodrugs are those that, when administered to a subject, enhance the bioavailability of the exemplified compound (e.g., making orally administered compounds more readily absorbed into the bloodstream) or improve delivery of the parent compounds to biological compartments (e.g., brain or lymphatic system) relative to the parent species. Prodrugs include ester forms of the compounds of the present invention.

### Compounds of the present invention

As used herein, the term "compounds of the present invention" refers to compounds of general formula (I), or racemates, stereoisomers, tautomers, prodrugs, or pharmaceutically acceptable salts thereof.

The present invention relates to: racemic mixtures of these compounds, mixtures enriched with any enantiomer, and any isolated enantiomer. For the scope of the present invention, it should be understood that the racemic mixture refers to a 50%:50% mixture of the two R and S enantiomers. The isolated enantiomer should be understood as a pure enantiomer (i.e., 100%) or a mixture highly enriched with a certain enantiomer (with purity ≥98%, ≥95%, ≥93%, ≥90%, ≥88%, ≥85%, or ≥80%).

Where there are stereoisomers of the compounds according to the present disclosure, the present disclosure encompasses all stereoisomers of the compounds.

Where there are tautomers of the compounds according to the present disclosure, the present disclosure encompasses all tautomers of the compounds.

The present disclosure also includes deuterated compounds generated by replacing one or more hydrogen atoms in said compounds with their stable isotope deuterium.

### Pharmaceutical composition

The present invention further provides a pharmaceutical composition comprising an active ingredient in a safe and effective amount, along with a pharmaceutically acceptable carrier.

The "active ingredient" described in the present invention refers to the compound of general formula (I) as described in the present invention, or a stereoisomer, tautomer, pharmaceutically acceptable salt or prodrug thereof.

"A safe and effective amount" refers to a quantity of the active ingredient sufficient to significantly ameliorate the condition without causing severe side effects. Typically, the pharmaceutical composition contains 1-2000 mg of active ingredient per dose, preferably 10-200 mg of active ingredient per dose. Preferably, the "dose" refers to one tablet.

The term "pharmaceutically acceptable carrier" refers to one or more compatible solid or liquid fillers or gel substances suitable for human use, which must be of sufficient purity and sufficiently low toxicity. The term "compatibility" herein means that the components in the composition can blend with the active ingredients of the present disclosure and with each other without significantly reducing the efficacy of the active ingredients.

The compounds of the preferred embodiments of the present invention can be administered as a single active agent or in combination with one or more other agents for treating cancer. The compounds of the preferred embodiments of the present invention are also effective when used in combination with known therapeutic agents and anticancer agents. Currently known combinations of compounds with other anticancer or chemotherapeutic agents fall within the scope of the preferred embodiments. Examples of such agents can be found in "Cancer Principles and Practice of Oncology", edited by V. T. Devita and S. Hellman, 6th edition (February 15, 2001), Lippincott Williams & Wilkins Publishers. Based on the specific properties of the drug and the cancer involved, a person skilled in the art can identify pharmaceutical combinations. Such anticancer agents include, but are not limited to, the following: estrogen receptor modulators, androgen receptor modulators, retinoid receptor modulators, cytotoxic/cytostatic agents, antiproliferative agents, prenyl-protein transferase inhibitors, histone deacetylase (HDAC) inhibitors, HMG-CoA reductase inhibitors, other angiogenesis inhibitors, inhibitors of cell proliferation and survival signaling, apoptosis inducers, agents that interfere with cell cycle checkpoints, CTLA4 antibodies, PD-1 antibodies, PD-L1 antibodies, and the like. The compounds of the preferred embodiments are also effective when administered concurrently with radiation therapy.

Generally, the compounds of the preferred embodiments will be administered in a therapeutically effective amount via any acceptable mode used for agents with similar effects. The actual dosage of the compounds of the preferred embodiments (i.e., the active ingredients) is determined by multiple factors, such as the severity of the disease to be treated, the age and relative health condition of the patients, the potency of the compound used, the route and form of administration, as well as other factors. The drug may be administered multiple times a day, preferably once or twice a day. All these factors are within the consideration of the attending physicians.

For the purposes of the preferred embodiments, the therapeutically effective dose is generally a total daily dose administered to the patient either as a single dose or in divided doses, for example, from about 0.001 to about 1000 mg/kg body weight per day, and preferably from about 1.0 to about 30 mg/kg body weight per day. Dosage unit composition may contain their dose factors to form the daily dose. The choice of dosage form depends on various factors, such as the mode of administration and the bioavailability of the drug substance. Generally, the compounds of the preferred embodiments may be administered as a pharmaceutical composition via any of the following routes: oral, systemic (e.g., transdermal, intranasal, or via suppository), or parenteral (e.g., intramuscular, intravenous, or subcutaneous) administration. The preferred mode of administration is oral administration, which allows convenient daily dosage adjusted according to the degree of bitterness. The composition may take the form of tablets, pills, capsules, semi-solids, powders, sustained release preparations, solutions, suspensions, elixirs, aerosols, or any other suitable compositions. Another preferred mode of administering the compounds of the preferred embodiments is via inhalation. This is an effective method for delivering the therapeutic agents directly to the respiratory tract (see, for example, U.S. Patent No. 5,607,915).

Suitable pharmaceutically acceptable carriers or excipients include, for example, treatment agents and drug delivery modifiers and enhancers, such as calcium phosphate, magnesium stearate, talc, monosaccharides, disaccharides, starch, gelatin, cellulose, sodium methylcellulose, carboxymethyl cellulose, glucose, hydroxypropyl-B-cyclodextrin, polyvinylpyrrolidone, low-melting-point wax, and ion exchange resins, etc., and any combination of two or more thereof. Liquid and semi-solid excipients may be selected from glycerol, propylene glycol, water, ethanol, and various oils, including petroleum, animal, vegetable, or synthetic oils, such as peanut oil, soybean oil, mineral oil, and sesame oil, etc. Preferred liquid carriers, particularly those for injectable solutions, include water, saline, aqueous solution of glucose, and ethylene glycol. Other suitable pharmaceutically acceptable excipients are described in Remington's Pharmaceutical Sciences, Mack Pub. Co., New Jersey (1991), which is incorporated herein by reference.

As used herein, the term "pharmaceutically acceptable salt" refers to non-toxic acid or alkaline earth metal salts of compounds of general formula I. These salts may be prepared in situ during the final isolation and purification of compounds of general formula I, or by separately reacting a suitable organic or inorganic acid or base with a basic or acidic functional group. Representative salts include, but are not limited to, acetate, adipate, alginate, citrate, aspartate, benzoate, benzenesulfonate, bisulfate, butyrate, camphorate, camphorsulfonate, digluconate, cyclopentanepropionate, dodecyl sulfate, ethanesulfonate, glucoheptonate, glycerophosphate, hemisulfate, heptanoate, hexanoate, fumarate, hydrochloride, hydrobromide, hydroiodide, 2-hydroxyethanesulfonate, lactate, maleate, methanesulfonate, nicotinate, 2-naphthalenesulfonate, oxalate, pamoate, pectate, thiocyanate, 3-phenylpropionate, picrate, pivalate, propionate, succinate, sulfate, tartrate, thiocyanate, tosylate, and undecanoate. Furthermore, a nitrogen-containing basic group may be quaternized with the following reagents: alkyl halides such as methyl, ethyl, propyl, and butyl chlorides, bromides, and iodides; dialkyl sulfates such as dimethyl, diethyl, dibutyl, and dipentyl sulfates; long-chain halides such as decyl, lauryl, myristyl, and stearyl chlorides, bromides, and iodides; and aralkyl halides such as benzyl and phenethyl bromides, thereby producing watersoluble, or oil-soluble, or dispersible products. Examples of acids that may be used to form pharmaceutically acceptable acid addition salts include inorganic acids such as hydrochloric, sulfuric, and phosphoric acids, and organic acids such as oxalic, maleic, methanesulfonic, succinic, and citric acids. Base addition salts may be prepared in situ during the final isolation and purification of compounds of general formula I, or by separately reacting a carboxylic acid moiety with a suitable base, such as hydroxides, carbonates, or bicarbonates of a pharmaceutically acceptable metal cation, or with ammonia or an organic primary, secondary, or tertiary amine. Pharmaceutically acceptable salts include, but are not limited to, those based on alkali and alkaline earth metal cations, such as sodium, lithium, potassium, calcium, magnesium, and aluminum, etc., as well as non-toxic ammonium, quaternary ammonium, and amine cations, including, but not limited to, ammonium, tetramethylammonium, tetraethylammonium, methylamine, dimethylamine, trimethylamine, triethylamine, and ethylamine, etc. Other representative organic amines for forming base addition salts include diethylamine, ethylenediamine, ethanolamine, diethanolamine, and piperazine, etc.

As used herein, the term "pharmaceutically acceptable prodrug" refers to prodrugs of the compounds of the preferred embodiments, which are rapidly converted in vivo into the parent compounds represented by the above general formula, such as by hydrolysis in the blood. A comprehensive discussion is provided in "T. Higuchi and V. Stella, Pro-drugs as Novel Delivery Systems, Vol. 14 of A.C.S. 15 Symposium Series" and "Edward B. Roche, ed., Bioreversible Carriers in Drug Design, American Pharmaceutical Association and Pergamon Press, 1987", both of which are incorporated herein by reference.

The advantages of the present invention are:
(1) Providing a structurally novel compound of general formula (I);
(2) The compounds of the present invention can serve as highly effective microtubule stabilizers;
(3) Exhibiting various pharmacological activities, including the treatment of cancer and neurodegenerative diseases (Huntington's disease, Alzheimer's disease, frontotemporal dementia, Parkinson's disease, multiple sclerosis, and traumatic brain injury), as well as antiinflammatory effects.

The present invention is further illustrated by the following specific embodiments. It should be understood that these embodiments are intended to illustrate the invention without limiting its scope. Unless otherwise defined, all technical and scientific terms as used herein have the same meanings as commonly understood by those skilled in the art. Furthermore, any methods and materials similar or equivalent to those described herein may be used in the methods of the present disclosure. The preferred implementation methods and materials described herein are for illustrative purposes only.

Pre-HPLC conditions: HPLC-MS analysis was performed on a Waters HPLC 2767 using a Waters QDA (Model KBD5205) as the mass detector and a Waters 2489 UV as the detector. The column used was an XTerra^{®} Prep MS C₁₈OBD^{™} Column (5 µm, 19×100 mm). The mobile phase consisted of eluent A (water, 1.0 ‰ FA) and eluent B (CH₃CN), with an elution rate of 20 mL/min. The initial conditions were 95% A for 0.5 minutes, followed by a linear decrease to 40% A over 8.5 min, maintaining 40% A from 8.5 min to 10.5 min, and then returning to 95% A within 1.5 minutes, with a total running time of 12 minutes. The gradient of mobile phase and the running time may be adjusted appropriately based on the properties of the compound.

### Intermediate 1

### 5,7-Dichloro-6-(2,4,6-trifluorophenyl)-[1,2,4]triazolo[1,5-a]pyrimidine 1

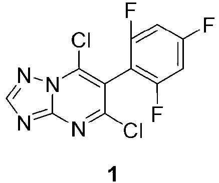

### Step 1: Diethyl 2-(2,4,6-trifluorophenyl)malonate 2

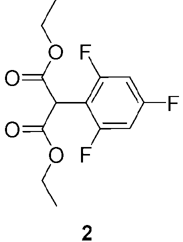

Sodium hydride (11.4 g, 60% wt.) was suspended in 1,4-dioxane, and diethyl malonate (45.7 g) was added dropwise at 0°C. After completion of addition, the reaction mixture was warmed to room temperature and stirred for 10 minutes. Copper(I) bromide (24.6 g) and 1-bromo-2,4,6-trifluorobenzene (30.0 g) were added, and the reaction was carried out at 100°C for 10 hours. The reaction mixture was quenched with saturated ammonium chloride, and the insoluble material was removed by filtration under suction. The filtrate was extracted with ethyl acetate, and the combined organic phases were dried over anhydrous sodium sulfate, filtered, and concentrated to obtain a crude product. Purification was performed by silica gel column chromatography (ethyl acetate: petroleum ether = 0-20%) to afford a colorless oil (38.0 g).

MS ESI: m/z =291.08, [M+H]⁺.

### Step 2: 6-(2,4,6-Trifluorophenyl)-[1,2,4]triazolo[1,5-a]pyrimidine-5,7-diol 3

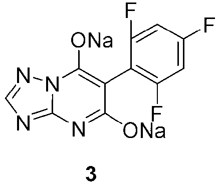

Compound **2** (20.0 g) and 1H-1,2,4-triazol-5-amine (6.08 g) were placed in a 100 mL sealed tube. Tri-n-butylamine (17.8 mL) was added at room temperature, and the reaction was carried out at 180°C for 6 hours. The reaction mixture was then cooled to 110°C, diluted with an appropriate amount of toluene, and 14 mL of 50% sodium hydroxide solution was added. The precipitated white solid was washed three times with toluene, filtered under suction, and dried to afford a white solid.

### Step 3: 5,7-Dichloro-6-(2,4,6-trifluorophenyl)-[1,2,4]triazolo[1,5-a]pyrimidine 1

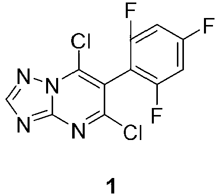

The product from the previous Step 3 was placed in a 500 mL sealed tube, and phosphorus oxychloride (130.0 mL) was slowly added under an ice-water bath. The reaction mixture was heated to 140°C and reacted for 8 hours. The reaction was quenched with saturated sodium bicarbonate, extracted with dichloromethane, and the combined organic phases were washed with saturated brine, dried over anhydrous sodium sulfate, filtered, and concentrated under reduced pressure. Silica gel column chromatography (ethyl acetate: petroleum ether = 0-10%) afforded the target product as a white solid (15 g).
MS ESI: m/z =319.0, [M+H]⁺.

### Intermediate 1A

### 7-Chloro-5-methyl-6-(2,4,6-trifluorophenyl)-[1,2,4]triazolo[1,5-a]pyrimidine 1A

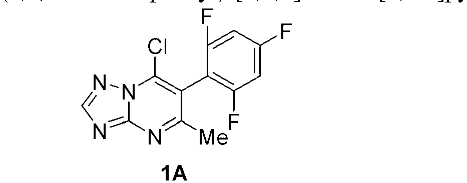

### Step 1: Methyl 3-oxo-2-(2,4,6-trifluorophenyl)butanoate 2A

2-(2,4,6-Trifluorophenyl)acetic acid (10.0 g) was dissolved in methanol (50.0 mL). Thionyl chloride (12.0 mL) was added under ice-water bath conditions, and the reaction was maintained for 3 hours. The reaction mixture was quenched with 50 mL of ice water and extracted with ethyl acetate. The combined organic phases were dried over anhydrous sodium sulfate, filtered under suction, and concentrated to afford the crude product as a colorless oil (10.47 g), which was carried forward without further purification. The intermediate (10.47 g) was dissolved in tetrahydrofuran (51.0 mL). Lithium bis(trimethylsilyl)amide (107.75 mL, 1.0 M in THF) was added at -65°C, and the reaction was maintained for 30 min. Acetyl chloride (4.1 mL) was introduced, and the reaction was allowed to proceed at room temperature for 5 hours. The mixture was diluted with water and extracted with ethyl acetate. The organic phase was dried over anhydrous sodium sulfate, filtered, and concentrated. The resulting crude product was purified by silica gel column chromatography (ethyl acetate: petroleum ether, 0-10% gradient) to afford the target compound 2A as a white solid (12.9 g).
¹H NMR (400 MHz, CDCl₃): δ 13.21 (s, 1H), 6.68 (t, *J =* 7.9 Hz, 2H), 3.70 (d, *J =* 1.2 Hz, 3H), 1.87 (s, 3H).

### Step 2: 7-Chloro-5-methyl-6-(2,4,6-trifluorophenyl)-[1,2,4]triazolo[1,5-a]pyrimidine 1A

The product obtained from the previous step (**2A**, 11.9 g) was placed in a 100 mL sealed tube and dissolved in acetic acid (54.1 mL). 1H-1,2,4-Triazol-5-amine (4.14 g) was added to the solution. The reaction mixture was heated at 120°C for 24 hours. After completion of the reaction, the excess acetic acid was evaporated under reduced pressure. Methyl tert-butyl ether was added to the residue, and the mixture was stirred at 60°C for 2 hours. The resulting suspension was gradually cooled to room temperature and filtered under suction to afford the target product as a yellow solid. The yellow solid obtained from the previous step was dissolved in phosphorus oxychloride (30 mL) in a 100 mL sealed tube. The reaction mixture was maintained at 130°C for 20 hours. Upon completion of the reaction, the mixture was carefully quenched by slow addition of saturated sodium bicarbonate solution. The resulting mixture was extracted with dichloromethane, and the organic phase was dried over anhydrous sodium sulfate. After filtration, the solution was concentrated under reduced pressure. The crude product was purified by silica gel column chromatography (methanol: dichloromethane = 0-5%) to afford 1.5 g of intermediate 1A as a white solid.
MS ESI: m/z =299.0, [M+H]⁺.

### Intermediate 1B

### 5,7-Dichloro-6-(2,6-difluoro-4-iodophenyl)-[1,2,4]triazolo[1,5-a]pyrimidine 1B

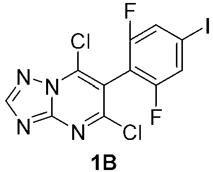

### Step 1: Diethyl 2-(2,6-difluoro-4-nitrophenyl)malonate 2B

1,2,3-Trifluoro-5-nitrobenzene (5.0 g) was dissolved in N,N-dimethylformamide (32.0 mL). Diethyl malonate (4.28 g) and potassium carbonate (7.81 g) were sequentially added to the solution. The reaction mixture was maintained at 65°C for 2 hours. The reaction was quenched with 1N hydrochloric acid and extracted with ethyl acetate. The combined organic phases were washed with saturated brine. The crude product was filtered under suction and concentrated. Purification was performed by silica gel column chromatography (ethyl acetate: petroleum ether = 0-10%) to afford the target product **2B** (7.4 g) as a yellow oil.

### Step 2: Diethyl 2-(4-amino-2,6-difluorophenyl)malonate 3B

The product obtained from Step 1 (5.0 g) was dissolved in methanol (50 mL). Palladium on carbon was added to the solution, and the reaction was conducted under a hydrogen atmosphere at room temperature for 8 hours. The reaction mixture was filtered under suction, and the filtrate was concentrated under reduced pressure. The resulting crude product was purified by silica gel column chromatography (ethyl acetate: petroleum ether = 0-30%) to afford the target compound **3B** (3.57 g) as a white solid.
¹H NMR (400 MHz, CDCl₃): δ 6.20 - 6.13 (m, 2H), 4.80 (s, 1H), 4.22 (q, *J =* 7.1 Hz, 4H), 1.25 (t, *J = 7.1* Hz, 6H).

### Step 3: Diethyl 2-(2,6-difluoro-4-iodophenyl)malonate 4B

The product obtained from Step 2 (2.0 g) was dissolved in 6N aqueous hydrochloric acid solution (12.0 mL). At 0°C, a solution of sodium nitrite (492.0 mg) in water (2.7 mL) was added dropwise, followed by the addition of a solution of potassium iodide (4.92 g) in water (5.0 mL). The reaction mixture was allowed to warm to room temperature and was maintained for 3 hours. The resulting mixture was extracted with ethyl acetate, and the combined organic phases were dried over anhydrous sodium sulfate. After filtration, the solution was concentrated. The crude product was purified by silica gel column chromatography (ethyl acetate: petroleum ether = 0-10%) to afford the target product **4B** (1.4 g) as a colorless oil.
¹H NMR (400 MHz, Chloroform-d): δ 7.33 - 7.29 (m, 2H), 4.89 (s, 1H), 4.25 (q, *J =* 7.1 Hz, 4H), 1.27 (t, *J* = 7.1 Hz, 6H).

### Step 4: Diethyl 2-(2,6-difluoro-4-iodophenyl)malonate 1B

The product from Step 3 (**4B**, 2.0 g) and 1H-1,2,4-triazol-5-amine (443.67 mg) were dissolved in tri-n-butylamine (1.3 mL). The reaction mixture was maintained at 170°C for 3 hours. Subsequently, the mixture was diluted with an appropriate amount of toluene at 110°C, followed by the addition of 50% sodium hydroxide solution at 50°C. The temperature was gradually reduced to room temperature, and a white solid was obtained by filtration under suction. The white solid thus obtained was dissolved in phosphorus oxychloride (10 mL), and the resulting solution was heated at 130°C for 6 hours. The reaction was quenched with saturated sodium bicarbonate solution and extracted with dichloromethane. The organic phase was dried over anhydrous sodium sulfate, filtered under suction, and concentrated. The crude product was purified by silica gel column chromatography (ethyl acetate: petroleum ether = 0-22%) to afford the target intermediate **1B**.
MS ESI: m/z =426.8, [M+H]⁺.

### Intermediate 4

### (R)-5-Chloro-N-(3-methylbutan-2-yl)-6-(2,4,6-trifluorophenyl)-[1,2,4]triazolo[1,5-a]pyrimidin-7-amine 4

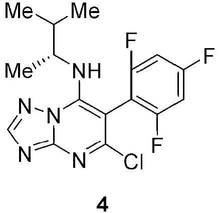

Intermediate **1** (500.0 mg) was dissolved in N-methylpyrrolidone (3.1 mL). (2R)-3-Methylbutan-2-amine hydrochloride (407.96 mg) and sodium bicarbonate powder (329.7 mg) were sequentially added to the solution at room temperature. The reaction mixture was heated to 60°C and maintained at this temperature for 3 hours. After completion of the reaction, saturated sodium chloride solution was added. The aqueous phase was extracted with ethyl acetate three times. The combined organic phases were washed with saturated brine three times and dried over anhydrous sodium sulfate. Following filtration, the solution was concentrated. The crude product was purified by silica gel column chromatography (ethyl acetate: petroleum ether = 0-20%) to afford the target intermediate **4** (560.0 mg) as a white solid.
MS ESI: m/z =370.0, [M+H]⁺.

### Intermediate 4A

### (R)-5-Methyl-N-(3-methylbutan-2-yl)-6-(2,4,6-trifluorophenyl)-[1,2,4]triazolo[1,5-a]pyrimidin-7-amine 4A

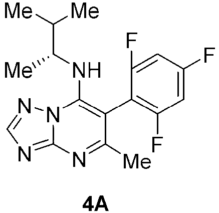

General intermediate **1** (159.6 mg) was dissolved in N-methylpyrrolidone (1.1 mL). (2R)-3-Methylbutan-2-amine hydrochloride (139.5 mg) and sodium bicarbonate powder (112.7 mg) were sequentially added to the solution at room temperature. The reaction mixture was heated to 60°C and maintained at this temperature for 3 hours. After completion of the reaction, saturated sodium chloride solution was added. The aqueous phase was extracted with ethyl acetate three times. The combined organic phases were washed with saturated brine three times and dried over anhydrous sodium sulfate. Following filtration, the solution was concentrated. The crude product was purified by silica gel column chromatography (ethyl acetate: petroleum ether = 0-29%) to afford the target intermediate **4A** (182.0 mg) as a white solid.
MS ESI: m/z =350.1, [M+H]⁺.

### Intermediate 4B

### (R)-5-Chloro-6-(2,6-difluoro-4-iodophenyl)-N-(3-methylbutan-2-yl)-[1,2,4]triazolo[1,5-a]pyrimidin-7-amine 4B

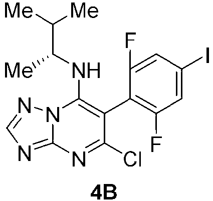

Using (2R)-3-methylbutan-2-amine hydrochloride (258.1 mg) and intermediate **1B** (0.5 g) as starting materials, with sodium bicarbonate (207.92 mg) as the base, the target intermediate **4B** (629.0 mg) was obtained as a white solid via the same synthetic method as intermediate **4**.
MS ESI: m/z =475.9, [M-H]⁻.

### Intermediate 5

### (S)-5-Chloro-6-(2,4,6-trifluorophenyl)-N-(1,1,1-trifluoropropan-2-yl)-[1,2,4]triazolo[1,5-a]pyrimidin-7-amine 5

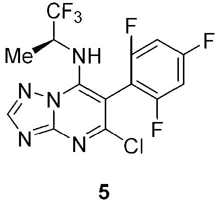

Using (S)-1,1,1-trifluoropropan-2-amine hydrochloride (491.76 mg) and intermediate **1** (0.5 g) as starting materials, with sodium bicarbonate (330.2 mg), the target intermediate **5** (266.7 mg) was obtained as a white solid via the same synthetic method as intermediate 4.
MS ESI: m/z =396.0, [M+H]⁺.

### Intermediate 5B

### (S)-5-Chloro-6-(2,6-difluoro-4-iodophenyl)-N-(1,1,1-trifluoropropan-2-yl)-[1,2,4]triazolo[1,5-a]pyrimidin-7-amine 5B

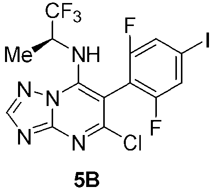

Using (S)-1,1,1-trifluoropropan-2-amine hydrochloride (800.96 mg) and intermediate **4B** (1.09 g) as starting materials, and sodium bicarbonate (451.1 mg), the target intermediate **5B** (964.7 mg) was obtained as a white solid via the same synthetic method as intermediate **4**.
MS ESI: m/z =501.9, [M-H]⁻.

### Intermediate 6

### (R)-5-Chloro-N-(1-cyclobutylethyl)-6-(2,4,6-trifluorophenyl)-[1,2,4]triazolo[1,5-a]pyrimidin-7-amine 6

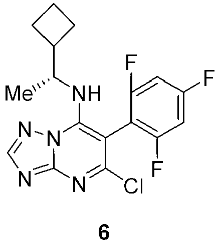

Using (R)-1-cyclobutylethan-1-amine hydrochloride (447.9 mg) and intermediate **1** (0.5 g) as starting materials, and sodium bicarbonate (330.2 mg), the target intermediate **6** (590 mg) was obtained as a white solid via the same synthetic method as intermediate **4**.
MS ESI: m/z =382.0, [M+H]⁺.

### Intermediate 6A

### (R)-5-Methyl-N-(1-cyclobutylethyl)-6-(2,4,6-trifluorophenyl)-[1,2,4]triazolo[1,5-a]pyrimidin-7-amine 6A

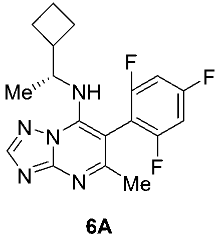

Using (R)-1-cyclobutylethan-1-amine hydrochloride (191.25 mg) and intermediate **1A** (0.2 g) as starting materials, and sodium bicarbonate (140.91 mg), the target intermediate **6A** (229 mg) was obtained as a white solid via the same synthetic method as intermediate **4**.
MS ESI: m/z =362.1, [M+H]⁺.

### Intermediate 6B

### (R)-5-Chloro-N-(1-cyclobutylethyl)-6-(2,6-difluoro-4-iodophenyl)-[1,2,4]triazolo[1,5-a]pyrimidin-7-amine 6B

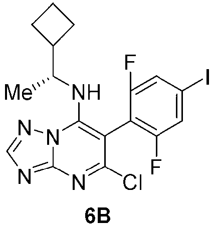

Using (R)-1-cyclobutylethan-1-amine hydrochloride (199.4 mg) and intermediate **4B** (0.3 g) as starting materials, and sodium bicarbonate (123.5 mg), the target intermediate **6B** (253.6 mg) was obtained as a white solid via the same synthetic method as intermediate **4**.
MS ESI: m/z =488.0, [M-H]⁻.

### Intermediate 7

### 5-Chloro-N-(2,2,2-trifluoroethyl)-6-(2,4,6-trifluorophenyl)-[1,2,4]triazolo[1,5-a]pyrimidin-7-amine 7

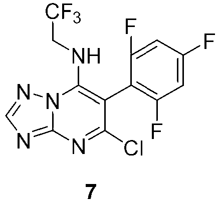

Using 2,2,2-trifluoroethan-1-amine hydrochloride (446.8 mg) and intermediate**1** (0.5 g) as starting materials, and sodium bicarbonate (329.7 mg), the target intermediate**7** (517 mg) was obtained as a white solid via the same synthetic method as intermediate **4**.
MS ESI: m/z =382.0, [M+H]⁺.

### Intermediate 7A

### 5-Methyl-N-(2,2,2-trifluoroethyl)-6-(2,4,6-trifluorophenyl)-[1,2,4]triazolo[1,5-a]pyrimidin-7-amine 7A

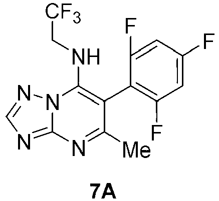

Using 2,2,2-trifluoroethan-1-amine hydrochloride (286.5 mg) and intermediate **1A** (0.3 g) as starting materials, and sodium bicarbonate (211.3 mg), the target intermediate **7A** (517 mg) was obtained as a white solid via the same synthetic method as intermediate **4.**
MS ESI: m/z =382.0, [M+H]⁺.

### Intermediate 7B

### 5-Chloro-6-(2,6-difluoro-4-iodophenyl)-N-(2,2,2-trifluoroethyl)-[1,2,4]triazolo[1,5-a]pyrimidin-7-amine 7B

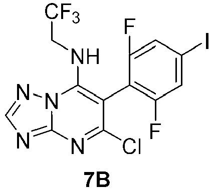

Using 2,2,2-trifluoroethan-1-amine hydrochloride (199.2 mg) and intermediate **1B** (0.3 g) as starting materials, and sodium bicarbonate (123.5 mg), the target intermediate **7B** (297.4 mg) was obtained as a white solid via the same synthetic method as intermediate **4.**
MS ESI: m/z =487.9, [M-H]⁻.

### Intermediate 14

### (±)2-(Hydroxy)-spiro[3.5]nonan-7-yl(methyl)carbamic acid tert-butyl ester 14

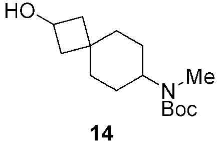

### Step 1: Benzyl (4-methylenepiperidin-1-yl) carbamate 9

Methyltriphenylphosphonium bromide (58.9 g) was dissolved in 200 mL of tetrahydrofuran and cooled to -10°C. n-Butyllithium (103.1 mL, 1.6M in hexane) was added, and the mixture was stirred for 30 minutes. At -78°C, a solution of tert-butyl (4-oxocyclohexyl) carbamate (25.0 g) in tetrahydrofuran (100 mL) was added. After reacting for 10 minutes, the temperature was raised to room temperature, and the mixture was stirred for 3 hours. The reaction was quenched with saturated ammonium chloride, extracted three times with ethyl acetate, and the combined organic phases were washed with saturated brine. The organic phase was dried over anhydrous sodium sulfate, filtered under suction, and concentrated under reduced pressure. Purification was performed by silica gel column chromatography (ethyl acetate: petroleum ether = 0-10%) to afford the target product **9** (22.0 g) as a colorless oil.
¹H NMR (400 MHz, CDCl₃): δ 4.64 (t, *J =* 1.8 Hz, 2H), 2.70 (s, 3H), 2.35 (dp, *J =* 13.4, 2.1 Hz, 2H), 2.14 (td, *J =* 12.4, 6.8 Hz, 2H), 1.76 (ddt, *J =* 12.7, 4.9, 2.3 Hz, 2H), 1.47 (s, 12H).

### Step 2: Benzyl methyl(4-methylenecyclohexyl)carbamate 11

Intermediate **9** (5.0 g) was treated with trifluoroacetic acid (5.2 mL) at room temperature for 2 hours. The reaction mixture was concentrated to afford the crude trifluoroacetate salt **10** as a dark black oil (4.0 g). The crude **10** (4.0 g) was dissolved in acetonitrile (41.0 mL) and water (41.0 mL), followed by sequential addition of benzyl chloroformate (4.6 g) and sodium bicarbonate powder (4.45 g). The mixture was stirred at room temperature for 10 hours. Acetonitrile was removed by distillation, and the residue was extracted three times with ethyl acetate. The combined organic phases were washed three times with saturated brine and dried over anhydrous sodium sulfate. After filtration under suction and concentration under reduced pressure, the crude product was purified by silica gel column chromatography (ethyl acetate: petroleum ether = 0-5%) to afford the target intermediate **11** (2.6 g) as a colorless oil.
MS ESI: m/z =260.1, [M+H]⁺.

### Step 3: Benzyl methyl(2-oxaspiro[3.5]non-7-yl)carbamate 12

Intermediate **11** (2.6 g) was dissolved in diethyl ether, and zinc-copper couple (5.2 g) was added. A solution of trichloroacetyl chloride (3.36 mL) in diethyl ether (104 mL) was slowly added dropwise. After reacting for 16 hours, the mixture was quenched with saturated sodium bicarbonate solution, filtered under suction, and washed three times with ethyl acetate. The filtrate was extracted with ethyl acetate, and the combined organic phases were dried over anhydrous sodium sulfate. After filtration under suction and concentration, the crude product (2.0 g) was obtained. The crude product was dissolved in acetic acid (6.0 mL), and zinc powder (1.4 g) was added. The reaction was carried out at 80°C for 5 hours. Water was added, and the mixture was filtered under suction. The filtrate was extracted with dichloromethane, and the combined organic phases were washed three times with saturated sodium bicarbonate and dried over anhydrous sodium sulfate. After filtration under suction and concentration, the crude product was purified by silica gel column chromatography (ethyl acetate: petroleum ether = 0-22%) to afford the target intermediate **12** as a white solid (3.0 g).
MS ESI: m/z =302.1, [M+H]⁺.

### Step 4: tert-Butyl (2-hydroxyspiro[3.5]non-7-yl)(methyl)carbamate 14

Intermediate **12** (3.0 g) was dissolved in methanol (20.0 mL), and sodium borohydride powder (565.2 mg) was added. After 5 hours, saturated brine was added, and the mixture was extracted with dichloromethane. The organic phase was dried over anhydrous sodium sulfate, filtered under suction, and concentrated under reduced pressure to afford a viscous oil, which was used directly in the next step without purification. The oil was dissolved in 76 mL of methanol, and wet palladium on carbon (760.0 mg, 10% wt.) was added. The reaction was carried out under a hydrogen atmosphere for 5 hours. After filtration under suction and concentration, the crude product was obtained as a white solid and used directly in the next step without purification. The crude product was dissolved in dichloromethane (45 mL), and di-tert-butyl dicarbonate (2.73 mL) and triethylamine (2.1 mL) were added sequentially in an ice-water bath. The reaction was stirred at room temperature for 18 hours. The reaction was quenched with water, extracted with dichloromethane, and dried over anhydrous sodium sulfate. After filtration under suction and concentration, the crude product was purified by silica gel column chromatography (methanol: dichloromethane = 0-10%) to afford the target intermediate **14** (2.5 g).
¹H NMR (400 MHz, CDCl₃): δ 4.21 (t, *J =* 7.2 Hz, 1H), 2.65 (s, 3H), 2.47 (d, *J =* 37.5 Hz, 1H), 2.30 - 2.21 (m, 1H), 2.08 (dd, *J =* 12.0, 6.0 Hz, 1H), 1.63 - 1.36 (m, 19H).

### Intermediate 15

### (±)tert-Butyl (6-hydroxyspiro[3.3]heptan-2-yl)(methyl)carbamate 15

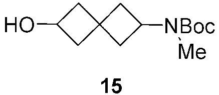

### Step 1: 6-(Methylamino)spiro[3.3]heptan-2-ol

tert-Butyl (6-hydroxyspiro[3.3]heptan-2-yl)carbamate (1.0 g) was dissolved in 20.0 mL of tetrahydrofuran, and lithium aluminum hydride (22.0 mL, 1.0 M in THF) was added dropwise at 0°C. The reaction was stirred at 50°C for 3 hours, quenched with methanol, and concentrated to afford a viscous semi-solid crude product, which was used directly in the next step without further purification.
MS ESI: m/z =142.2, [M+H]⁺.

### Step 2: tert-Butyl (6-hydroxyspiro[3.3]heptan-2-yl)(methyl)carbamate 15

The product from the previous step was dissolved in 20.0 mL of dichloromethane, and di-tert-butyl dicarbonate (1.5 g) and triethylamine (1.2 mL) were added at 0°C. The reaction was stirred at room temperature for 18 hours, quenched with water, and extracted with dichloromethane. The organic phase was dried over anhydrous sodium sulfate, filtered under suction, and concentrated. The crude product was purified by silica gel column chromatography (methanol: dichloromethane = 0-10%) to afford the target fragment **15** (0.75 g).
MS ESI: m/z =186.2, [M+H]⁺.

### Intermediate 17

### tert-Butyl (3aR,5r,6aS)-5-hydroxyhexahydrocyclopenta[c]pyrrole-2(1H)-carboxylate 17

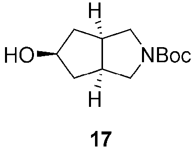

tert-Butyl cis-5-oxohexahydrocyclopenta[c]pyrrole-2(1H)-carboxylate 16 (1.0 g) was dissolved in 10 mL of methanol, and sodium borohydride (251.83 mg) was added at 0°C. The reaction was stirred for 2 hours, quenched with saturated brine, and extracted with dichloromethane. The organic phase was dried over anhydrous sodium sulfate, filtered under suction, and concentrated to afford a viscous residue. Purification was performed by silica gel column chromatography (ethyl acetate: petroleum ether = 0-20%) to afford the target compound 17 (820.0 mg).
¹H NMR (400 MHz, CDCl₃): δ 4.30 (t, *J=* 6.4 Hz, 1H), 3.50 (dd, *J=* 11.2, 7.7 Hz, 2H), 3.34 (dd, *J =* 11.3, 3.4 Hz, 2H), 2.60 (q, *J=* 4.8, 3.4 Hz, 2H), 2.21 - 2.12 (m, 2H), 2.03 (s, 1H), 1.45 (s, 11H).

### Intermediate 19

### tert-Butyl (3aR,5s,6aS)-5-hydroxyhexahydrocyclopenta[c]pyrrole-2(1H)-carboxylate 19

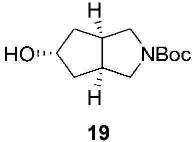

### Step 1: tert-Butyl (3aR,5s,6aS)-5-((4-nitrobenzoyl)oxy)hexahydrocyclopenta[c]pyrrole-2(1H)-carboxylate 18

Compound **17** (4.3 g) was dissolved in 126 mL of diethyl ether, followed by addition of triphenylphosphine (4.96 g) and p-nitrobenzoic acid (2.37 g). Diisopropyl azodicarboxylate (1.91 g) was added at -78 °C, and the reaction was stirred at room temperature for 16 hours. The reaction was quenched with methanol, and the crude product was concentrated and purified by silica gel column chromatography (ethyl acetate: petroleum ether = 0-10%) to afford compound **18** (1.5 g). ¹H NMR (400 MHz, CDCl₃): δ 8.30 - 8.26 (m, 2H), 8.19 - 8.15 (m, 2H), 5.57 (dt, *J =* 5.6, 2.9 Hz, 1H), 3.59 - 3.50 (m, 2H), 3.24 (s, 2H), 2.89 (dd, *J =* 9.2, 5.5 Hz, 2H), 2.21 - 2.14 (m, 2H), 1.93 (dt, *J =* 14.4, 5.7 Hz, 2H), 1.47 (s, 9H).

### Step 2: tert-Butyl (3aR,5s,6aS)-5-hydroxyhexahydrocyclopenta[c]pyrrole-2(1H)-carboxylate 19

Compound **18** (1.5 g) was dissolved in methanol (49.0 mL) and water (16.0 mL) at room temperature, followed by addition of solid potassium carbonate (1.1 g). The reaction was stirred at room temperature for 8 hours. Methanol was evaporated, and the mixture was extracted with ethyl acetate. The organic phase was dried over anhydrous sodium sulfate, filtered under suction, and concentrated. The crude product was purified by silica gel column chromatography (ethyl acetate: petroleum ether = 0-50%) to afford the target intermediate **19** (791.0 mg).
MS ESI: m/z =172.1, [M-57+H]⁺.

### Intermediate 19A

### tert-Butyl (1R,5S,6s)-6-ethynyl-3-azabicyclo[3.1.0]hexane-3-carboxylate 19A

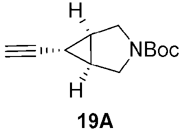

tert-Butyl (1R,5S,6r)-6-formyl-3-azabicyclo[3.1.0]hexane-3-carboxylate (5.66 g) was dissolved in 50 mL of methanol, followed by sequential addition of potassium carbonate (5.54 g) and dimethyl (1-diazo-2-oxopropyl)phosphonate (6.16 g). The reaction was stirred at room temperature for 8 hours, quenched with water, and extracted with ethyl acetate. The organic phase was dried over anhydrous sodium sulfate, filtered under suction, and concentrated. The crude product was purified by column chromatography to afford the target intermediate **19A** (4.6 g) as a white solid.
¹H NMR (400 MHz, CDCl₃): δ 3.65 (d, *J =* 11.3 Hz, 1H), 3.56 (d, *J =* 11.1 Hz, 1H), 3.33 (tt, *J =* 7.9, 3.4 Hz, 2H), 1.87 (d, *J =* 2.2 Hz, 1H), 1.82 (t, *J =* 2.9 Hz, 2H), 1.42 (s, 9H), 1.10 (d, *J =* 2.7 Hz, 1H).

### Intermediate 19B

### tert-Butyl (1R,5S,6s)-6-ethynyl-3-azabicyclo[3.1.0]hexane-3-carboxylate 19B

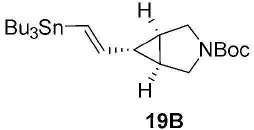

Copper(I) cyanide (211.36 mg) was suspended in tetrahydrofuran (6.32 mL). At -78 °C, n-butyllithium (2.0 mL, 2.5 M in hexane) was added dropwise, and the mixture was stirred at this temperature for 15 minutes. Tributyltin hydride (1.35 mL) was then added, and stirring was continued for 10 minutes. A solution of **19A** (0.5 g) in tetrahydrofuran (2.1 mL) was introduced, and the reaction was maintained at -78 °C for an additional 10 minutes. The reaction was quenched with ammonia-ammonium chloride buffer solution (pH = 12) and filtered under suction. The filtrate was extracted with ethyl acetate, and the organic phase was dried over anhydrous sodium sulfate. After filtration under suction and concentration, the crude product **19B** (350.0 mg) was obtained and used directly without further purification.

### Example 1

### (R)-6-(4-((2-azaspiro[3.3]hept-6-yl)oxy)-2,6-difluorophenyl)-5-chloro-N-(3-methylbut-2-yl)-[1,2,4]triazolo[1,5-a]pyrimidin-7-amine 23

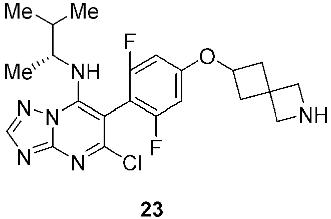

### Step 1: (R)-6-(4-(5-chloro-7-((3-methylbut-2-yl)amino)-[1,2,4]triazolo[1,5-a]pyrimidin-6-yl)-3,5-difluorophenoxy)-2-azaspiro[3.3]heptane-2-carboxylic acid tert-butyl ester 22

Sodium hydride (43.34 mg, 60% wt. in mineral oil) was suspended in dimethyl sulfoxide (0.86 mL) and tetrahydrofuran (0.43 mL). 6-Hydroxy-2-azaspiro[3.3]heptane-2-carboxylic acid tert-butyl ester (231.12 mg) was added at room temperature, and the mixture stirred at 60 °C for 1 hour. A solution of intermediate **4** (100.0 mg) in dimethyl sulfoxide (1.0 mL) and tetrahydrofuran (1.0 mL) was then added dropwise. The reaction was maintained at 60 °C for 3 hours, then quenched with saturated ammonium chloride solution. The mixture was extracted with ethyl acetate, and the organic phase was dried over anhydrous sodium sulfate, filtered under suction, concentrated to afford intermediate **22** (149.0 mg).

### Step 2: (R)-6-(4-((2-azaspiro[3.3]hept-6-yl)oxy)-2,6-difluorophenyl)-5-chloro-N-(3-methylbut-2-yl)-[1,2,4]triazolo[1,5-a]pyrimidin-7-amine 23

Intermediate 22 was dissolved in dichloromethane (2.77 mL). Trifluoroacetic acid (0.89 mL) was added dropwise at 0°C, and the reaction mixture was allowed to warm to room temperature. The reaction was maintained at room temperature for 2 hours before being quenched with saturated sodium bicarbonate solution. The mixture was extracted with dichloromethane, and the combined organic phases were dried over anhydrous sodium sulfate. After filtration under suction, the solution was concentrated to afford the crude product, which was purified by preparative HPLC using acetonitrile and water (containing 1‰ formic acid) to afford the target compound 23 (80.0 mg) as a white powder.
MS ESI: m/z =463.1, [M+H]⁺.
¹H NMR (400 MHz, DMSO-*d*₆): δ 8.59 (s, 1H), 8.40 (s, 1H), 6.89 (dd, *J =* 9.7, 3.4 Hz, 2H), 4.71 (d, *J =* 6.8 Hz, 1H), 3.96 (s, 2H), 3.89 (s, 2H), 2.83 (dd, *J =* 12.8, 6.7 Hz, 2H), 2.26 (p, *J =* 5.5 Hz, 2H), 1.81 - 1.71 (m, 1H), 1.04 (d, *J =* 6.5 Hz, 3H), 0.69 (d, *J =* 6.7 Hz, 6H).

### Example 2

### (R)-6-(4-((2-methyl-2-azaspiro[3.3]hept-6-yl)oxy)-2,6-difluorophenyl)-5-chloro-N-(3-methylbut-2-yl)-[1,2,4]triazolo[1,5-a]pyrimidin-7-amine 24

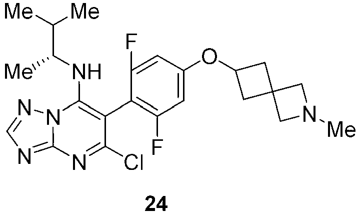

Compound **23** (16.2 mg) was dissolved in 1,2-dichloroethane (2.0 mL). Sodium triacetoxyborohydride (22.25 mg) and formaldehyde aqueous solution (6.0 µL, 37 wt%) were sequentially added at 0°C. The reaction mixture was stirred for 5 minutes, followed by addition of 1.0 M aqueous sodium hydroxide solution (0.5 mL). The mixture was extracted with dichloromethane, and the combined organic phases were dried over anhydrous sodium sulfate. After filtration under suction, the solution was concentrated under reduced pressure to afford the crude product, which was purified by preparative HPLC using acetonitrile and water (containing 1‰ formic acid) to afford the target compound **24** (13.0 mg) as a white powder.
MS ESI: m/z =477.2, [M+H]⁺.
¹H NMR (400 MHz, DMSO-*d*₆): δ 8.59 (s, 1H), 7.93 (s, 1H), 6.87 (dd, *J =* 10.4, 4.3 Hz, 2H), 4.72 (q, *J =* 7.0 Hz, 1H), 3.22 (s, 1H), 3.15 (s, 1H), 2.70 (d, *J =* 10.6 Hz, 2H), 2.18 (d, *J =* 12.9 Hz, 7H), 1.76 (q, *J* = 7.1 Hz, 1H), 1.04 (d, *J =* 6.4 Hz, 4H), 0.69 (d, *J =* 6.5 Hz, 6H).

### Example 3

### (S)-6-(4-((2-Azaspiro[3.3]heptan-6-yl)oxy)-2,6-difluorophenyl)-5-chloro-N-(1,1,1-trifluoropropan-2-yl)-[1,2,4]triazolo[1,5-a]pyrimidin-7-amine 26

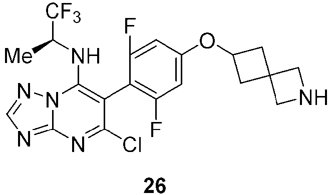

### Step 1: (S)-tert-Butyl 6-(4-(5-chloro-7-((1,1,1-trifluoropropan-2-yl)amino)-[1,2,4]triazolo[1,5-a]pyrimidin-6-yl)-3,5-difluorophenoxy)-2-azaspiro[3.3]heptane-2-carboxylate 25

Using intermediate **5** (100.0 mg) and tert-butyl 6-hydroxy-2-azaspiro[3.3]heptane-2-carboxylate (216.0 mg) as starting materials, with sodium hydride (40.5 mg, 60% wt. in mineral oil) as the base, intermediate **25** (142.7 mg) was obtained under the same conditions as described in Step 1 of Example 1.

### Step 2: (S)-6-(4-((2-Azaspiro[3.3]heptan-6-yl)oxy)-2,6-difluorophenyl)-5-chloro-N-(1,1,1-trifluoropropan-2-yl)-[1,2,4]triazolo[1,5-a]pyrimidin-7-amine 26

Using intermediate **25** (142.7 mg) as the starting material, compound **26** (61.0 mg) was obtained as a white powder under the same conditions as described in Step 2 of Example 1.
MS ESI: m/z =489.0, [M+H]⁺.
¹H NMR (400 MHz, DMSO-*d*₆): δ 8.34 (s, 1H), 8.24 (d, *J* = 4.4 Hz, 1H), 6.73 (d, *J* = 10.5 Hz, 2H), 5.81 (d, *J* = 12.5 Hz, 1H), 4.68 (s, 1H), 4.02 (s, 2H), 3.96 (s, 2H), 2.84 (dd, *J =* 12.5, 6.5 Hz, 2H), 2.30 (dd, *J =* 12.0, 7.0 Hz, 2H), 1.30 (d, *J =* 6.5 Hz, 3H).

### Example 4

### (S)-6-(4-((2-Methyl-2-azaspiro[3.3]heptan-6-yl)oxy)-2,6-difluorophenyl)-5-chloro-N-(1,1,1-trifluoropropan-2-yl)-[1,2,4]triazolo[1,5-a]pyrimidin-7-amine 27

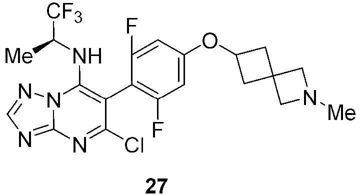

Using compound **26** (34.2 mg) as the starting material, with sodium triacetoxyborohydride (44.5 mg) and formaldehyde aqueous solution (8.08 µL, wt. 37%) as reagents, the target compound **27** (30.0 mg) was obtained as a white powder via the same method as described in Example 2. MS ESI: m/z =503.1, [M+H]⁺.
¹H NMR (400 MHz, DMSO-*d*₆): δ 8.33 (s, 1H), 8.24 (s, 1H), 6.74 - 6.68 (m, 2H), 5.85 - 5.76 (m, 1H), 4.73 - 4.65 (m, 1H), 3.60 (s, 2H), 3.54 (s, 2H), 2.78 - 2.71 (m, 2H), 2.44 (s, 3H), 2.22 (d, *J* = 9.9 Hz, 2H), 1.29 (d, *J =* 6.6 Hz, 3H).

### Example 5

### (R)-6-(4-((7-Azaspiro[3.5]nonan-2-yl)oxy)-2,6-difluorophenyl)-5-chloro-N-(3-methylbutan-2-yl)-[1,2,4]triazolo[1,5-a]pyrimidin-7-amine 30

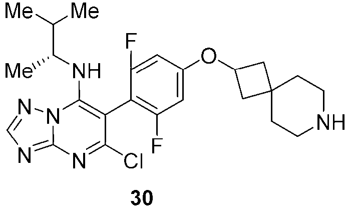

### Step 1: (R)-tert-Butyl 2-(4-(5-chloro-7-((3-methylbutan-2-yl)amino)-[1,2,4]triazolo[1,5-a]pyrimidin-6-yl)-3,5-difluorophenoxy)-7-azaspiro[3.5]nonane-7-carboxylate 29

Using intermediate **4** (100.0 mg) and tert-butyl 2-hydroxy-7-azaspiro[3.5]nonane-7-carboxylate (261.6 mg) as starting materials, and sodium hydride (43.36 mg, 60% wt. in mineral oil) as the base, the reaction was carried out under the same conditions as described in Step 1 of Example 1 to afford intermediate **29** (159.0 mg) as a white powder.

### Step 2: (R)-6-(4-((7-Azaspiro[3.5]nonan-2-yl)oxy)-2,6-difluorophenyl)-5-chloro-N-(3-methylbutan-2-yl)-[1,2,4]triazolo[1,5-a]pyrimidin-7-amine 30

Using intermediate**29** (159.0 mg) as the starting material, the reaction was carried out under the same conditions as described in Step 2 of Example 1 to afford compound **30** (90.0 mg) as a white powder.
MS ESI: m/z =491.1, [M+H]⁺.
¹H NMR (400 MHz, DMSO-*d*₆): δ 8.59 (s, 1H), 8.40 (s, 1H), 6.91 - 6.85 (m, 2H), 4.87 (t, *J =* 6.8 Hz, 1H), 2.97 (t, *J =* 5.7 Hz, 2H), 2.88 (d, *J =* 6.0 Hz, 2H), 2.54 (d, *J =* 7.6 Hz, 3H), 1.86 (dq, *J =* 10.2, 3.4 Hz, 2H), 1.80 - 1.66 (m, 5H), 1.05 (d, *J =* 6.4 Hz, 3H), 0.69 (d, *J =* 6.5 Hz, 6H).

### Example 6

### (R)-6-(4-((7-Methyl-7-azaspiro[3.5]nonan-2-yl)oxy)-2,6-difluorophenyl)-5-chloro-N-(3-methylbutan-2-yl)-[1,2,4]triazolo[1,5-a]pyrimidin-7-amine 31

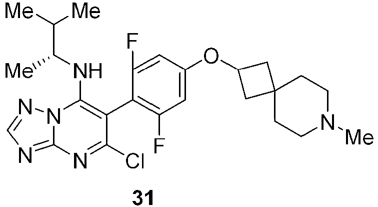

Using compound **30** (66.0 mg) as the starting material, sodium triacetoxyborohydride (35.6 mg), and aqueous formaldehyde solution (22.92 µL, wt. 37%) as reagents, the reaction was carried out via the same method as described in Example 2 to afford the target compound **31** (51.0 mg) as a white powder.
MS ESI: m/z =505.2, [M+H]⁺.
¹H NMR (400 MHz, DMSO-*d*₆): δ 8.59 (s, 1H), 7.93 (s, 1H), 6.90 - 6.85 (m, 2H), 4.86 (t, *J =* 6.8 Hz, 1H), 2.42 (d, *J =* 9.5 Hz, 2H), 2.27 (d, *J =* 31.2 Hz, 4H), 2.15 (s, 3H), 1.78 (dq, *J =* 13.3, 6.8, 6.0 Hz, 4H), 1.62 - 1.56 (m, 4H), 1.04 (d, *J =* 6.5 Hz, 3H), 0.69 (d, *J =* 6.6 Hz, 6H).

### Example 7

### (S)-6-(4-((7-Azaspiro[3.5]nonan-2-yl)oxy)-2,6-difluorophenyl)-5-chloro-N-(1,1,1-trifluoropropan-2-yl)-[1,2,4]triazolo[1,5-a]pyrimidin-7-amine 33

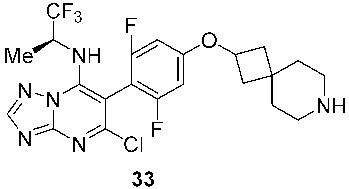

### Step 1: (S)-2-(4-(5-Chloro-7-((1,1,1-trifluoropropan-2-yl)amino)-[1,2,4]triazolo[1,5-a]pyrimidin-6-yl)-3,5-difluorophenoxy)-7-azaspiro[3.5]nonane-7-carboxylic acid tert-butyl ester 32

Using intermediate **5** (100.0 mg) and 2-hydroxy-7-azaspiro[3.5]nonane-7-carboxylic acid tert-butyl ester (244.3 mg) as starting materials, with sodium hydride (40.5 mg, 60% wt. in mineral oil) as the base, intermediate **32** (142.7 mg) was obtained under the same conditions as described in Step 1 of Example 1.

### Step 2: (S)-6-(4-((7-Azaspiro[3.5]nonan-2-yl)oxy)-2,6-difluorophenyl)-5-chloro-N-(1,1,1-trifluoropropan-2-yl)-[1,2,4]triazolo[1,5-a]pyrimidin-7-amine 33

Using intermediate **32** (139.0 mg) as the starting material, compound **33** (35.0 mg) was obtained as a white powder under the same conditions as described in Step 2 of Example 1.
MS ESI: m/z =517.0, [M+H]⁺.
¹H NMR (400 MHz, DMSO-*d*₆): δ 8.26 (d, *J =* 27.8 Hz, 2H), 6.72 - 6.64 (m, 2H), 5.83 - 5.72 (m, 1H), 4.82 (t, *J =* 6.8 Hz, 1H), 3.02 (t, *J =* 5.3 Hz, 2H), 2.94 (t, *J =* 5.6 Hz, 2H), 2.54 (t, *J =* 4.4 Hz, 2H), 1.89 (dd, *J =* 12.1, 6.4 Hz, 2H), 1.74 (dt, *J =* 17.5, 5.5 Hz, 4H), 1.25 (d, *J =* 6.7 Hz, 3H).

### Example 8

### (S)-6-(4-((7-Methyl-7-azaspiro[3.5]nonan-2-yl)oxy)-2,6-difluorophenyl)-5-chloro-N-(1,1,1-trifluoropropan-2-yl)-[1,2,4]triazolo[1,5-a]pyrimidin-7-amine 34

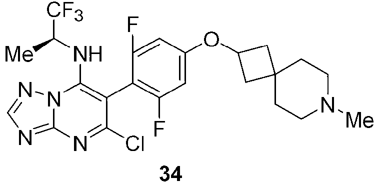

Using compound **33** (19.6 mg) as the starting material, with sodium triacetoxyborohydride (24.2 mg) and aqueous formaldehyde solution (5.5 µL, wt. 37%) as reagents, the target compound **34** (13.0 mg) was obtained as a white powder via the same method as described in Example 2. MS ESI: m/z =531.2, [M+H]⁺.
¹H NMR (400 MHz, DMSO-*d*₆): δ 8.32 (d, *J =* 97.9 Hz, 2H), 6.76 (s, 2H), 5.88 - 5.77 (m, 1H), 4.83 (t, *J =* 7.1 Hz, 1H), 2.45 (s, 4H), 2.32 (s, 3H), 1.87 - 1.78 (m, 3H), 1.65 (d, *J =* 17.1 Hz, 6H), 1.33 (s, 3H).

### Example 9

### (R)-5-Chloro-6-(2,6-difluoro-4-((6-(methylamino)spiro[3.3]heptan-2-yl)oxy)phenyl)-N-(3-methylbutan-2-yl)-[1,2,4]triazolo[1,5-a]pyrimidin-7-amine 36

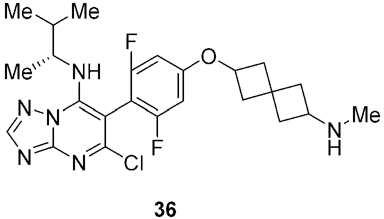

### Step 1: tert-Butyl (R)-(6-(4-(5-chloro-7-((3-methylbutan-2-yl)amino)-[1,2,4]triazolo[1,5-a]pyrimidin-6-yl)-3,5-difluorophenoxy)spiro[3.3]heptan-2-yl)(methyl)carbamate

Using intermediate **4** (110.0 mg) and tert-butyl (6-hydroxyspiro[3.3]heptan-2-yl)(methyl)carbamate **15** (287.5 mg) as starting materials, and sodium hydride (47.7 mg, 60% wt. in mineral oil, 4.0 eq.) as the base, intermediate **35** (170.0 mg) was obtained under the same conditions as described in Step 1 of Example 1.

### Step 2: (R)-5-Chloro-6-(2,6-difluoro-4-((6-(methylamino)spiro[3.3]heptan-2-yl)oxy)phenyl)-N-(3-methylbutan-2-yl)-[1,2,4]triazolo[1,5-a]pyrimidin-7-amine 36

Using intermediate **35** (170.0 mg) as the starting material, compound **36** (93.0 mg) was obtained as a white powder under the same conditions as described in Step 2 of Example 1.
MS ESI: m/z =491.1, [M+H]⁺.
¹H NMR (400 MHz, DMSO-*d*₆): δ 8.59 (s, 1H), 8.38 (s, 1H), 6.89 - 6.85 (m, 2H), 4.76 (t, *J =* 6.9 Hz, 1H), 3.21 (t, *J* = 7.7 Hz, 1H), 2.70 - 2.64 (m, 1H), 2.33 (q, *J =* 6.3 Hz, 1H), 2.24 (s, 5H), 2.11 - 2.03 (m, 3H), 1.95 (td, *J =* 8.3, 7.9, 4.3 Hz, 2H), 1.76 (q, *J =* 6.9 Hz, 1H), 1.04 (d, *J =* 6.5 Hz, 3H), 0.69 (d, *J =* 6.6 Hz, 6H).

### Example 10

### (R)-5-Chloro-6-(4-((6-(dimethylamino)spiro[3.3]heptan-2-yl)oxy)-2,6-difluorophenyl)-N-(3-methylbutan-2-yl)-[1,2,4]triazolo[1,5-a]pyrimidin-7-amine 37

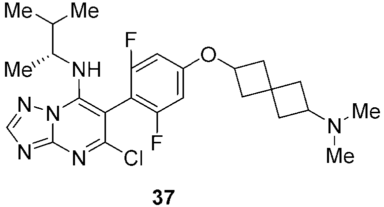

Using compound **36** (69.9 mg) as the starting material, sodium triacetoxyborohydride (90.9 mg), and aqueous formaldehyde solution (26.5 µL, wt. 37%) as reagents, the target compound **37** (59.0 mg) was obtained as a white powder via the same method as described in Example 2.
MS ESI: m/z =505.2, [M+H]⁺.
¹H NMR (400 MHz, DMSO-*d*₆): δ 8.59 (s, 1H), 7.92 (s, 1H), 6.90 - 6.83 (m, 2H), 4.76 (s, 1H), 2.70 - 2.61 (m, 1H), 2.54 (d, *J* = 11.9 Hz, 1H), 2.19 (dd, *J* = 11.0, 5.7 Hz, 1H), 2.01 (s, 10H), 1.79 (dt, *J =* 30.5, 8.1 Hz, 4H), 1.04 (d, *J =* 6.4 Hz, 3H), 0.69 (d, *J =* 6.4 Hz, 6H).

### Example 11

### (S)-5-Chloro-6-(2,6-difluoro-4-((6-(methylamino)spiro[3.3]heptan-2-yl)oxy)phenyl)-N-(1,1,1-trifluoropropan-2-yl)-[1,2,4]triazolo[1,5-a]pyrimidin-7-amine 39

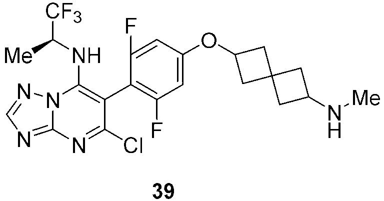

### Step 1: (S)-tert-butyl (6-(4-(5-chloro-7-((1,1,1-trifluoropropan-2-yl)amino)-[1,2,4]triazolo[1,5-a]pyrimidin-6-yl)-3,5-difluorophenoxy)spiro[3.3]heptan-2-yl)(methyl)carbamate 38

Using intermediate **5** (100.0 mg) and tert-butyl (6-hydroxyspiro[3.3]heptan-2-yl)(methyl)carbamate **15** (244.06 mg) as starting materials, and sodium hydride (40.48 mg, 60% wt. in mineral oil, 4.0 eq.) as base, intermediate **38** (134.1 mg) was obtained under the same conditions as described in Step 1 of Example 1.

### Step 2: (S)-5-chloro-6-(2,6-difluoro-4-((6-(methylamino)spiro[3.3]heptan-2-yl)oxy)phenyl)-N-(1,1,1-trifluoropropan-2-yl)-[1,2,4]triazolo[1,5-a]pyrimidin-7-amine 39

Using intermediate **38** (134.1 mg) as starting material, compound **39** (51.0 mg) was obtained under the same conditions as described in Step 2 of Example 1.
MS ESI: m/z =517.0, [M+H]⁺.
¹H NMR (400 MHz, DMSO-*d*₆): δ 8.21 (d, *J=* 7.0 Hz, 2H), 6.63 (dd, *J =* 10.0, 3.4 Hz, 2H), 5.77 - 5.68 (m, 1H), 4.64 (t, *J =* 6.9 Hz, 1H), 3.41 (t, *J =* 7.9 Hz, 1H), 2.63 (dt, *J =* 11.1, 5.5 Hz, 1H), 2.52 - 2.46 (m, 1H), 2.32 (s, 4H), 2.19 (ddd, *J =* 12.1, 7.3, 4.6 Hz, 1H), 2.07 (dq, *J =* 11.8, 6.1, 5.5 Hz, 4H), 1.21 (d, *J=* 6.7 Hz, 3H).

### Example 12

### (S)-5-chloro-6-(4-((6-(dimethylamino)spiro[3.3]heptan-2-yl)oxy)-2,6-difluorophenyl)-N-(1,1,1-trifluoropropan-2-yl)-[1,2,4]triazolo[1,5-a]pyrimidin-7-amine 40

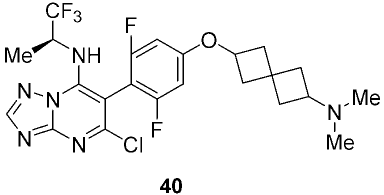

Using compound **39** (23.5 mg) as starting material, sodium triacetoxyborohydride (28.93 mg), and aqueous formaldehyde solution (5.5 µL, wt. 37%) as reagents, the target compound **40** (22.0 mg) was obtained as a white powder via the same method as described in Example 2.
MS ESI: m/z =531.2, [M+H]⁺.
¹H NMR (400 MHz, DMSO-*d*₆): δ 8.51 (s, 1H), 6.78 (d, *J* = 10.6 Hz, 2H), 5.84 (q, *J* = 7.5, 7.0 Hz, 1H), 4.73 (t, *J* = 6.9 Hz, 1H), 2.70 (dt, *J =* 29.5, 6.9 Hz, 2H), 2.24 (p, *J =* 5.7 Hz, 1H), 2.16 - 2.03 (m, 11H), 1.91 (t, *J =* 9.7 Hz, 2H), 1.36 (d, *J =* 6.8 Hz, 3H).

### Example 13

### (R)-5-chloro-N-(1-cyclobutylethyl)-6-(2,6-difluoro-4-((6-(methylamino)spiro[3.3]heptan-2-yl)oxy)phenyl)-[1,2,4]triazolo[1,5-a]pyrimidin-7-amine 42

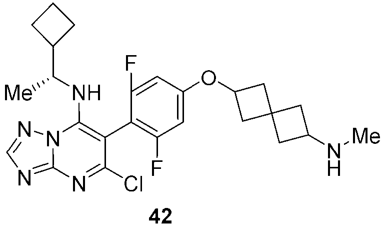

### Step 1: (R)-tert-Butyl (6-(4-(5-chloro-7-((1-cyclobutylethyl)amino)-[1,2,4]triazolo[1,5-a]pyrimidin-6-yl)-3,5-difluorophenoxy)spiro[3.3]heptan-2-yl)(methyl)carbamate 41

Using intermediate **6** (60.0 mg) and tert-butyl (6-hydroxyspiro[3.3]heptan-2-yl)(methyl)carbamate**15** (151.8 mg) as starting materials, with sodium hydride (25.12 mg, 60% wt. in mineral oil) as the base, intermediate **41** (85.6 mg) was obtained as a white powder under the same conditions as described in Step 1 of Example 1.

### Step 2: (R)-5-Chloro-N-(1-cyclobutylethyl)-6-(2,6-difluoro-4-((6-(methylamino)spiro[3.3]heptan-2-yl)oxy)phenyl)-[1,2,4]triazolo[1,5-a]pyrimidin-7-amine 42

Using intermediate **41** (85.6 mg) as the starting material, compound **42** (21.0 mg) was obtained as a white powder under the same conditions as described in Step 2 of Example 1.
MS ESI: m/z =503.2, [M+H]⁺.
¹H NMR (400 MHz, DMSO-*d*₆): δ 8.58 (s, 1H), 8.37 (s, 1H), 6.90 (dd, *J =* 14.9, 11.7 Hz, 2H), 4.77 (t, *J =* 6.8 Hz, 1H), 3.20 (t, *J* = 7.7 Hz, 1H), 2.68 (p, *J* = 6.2, 5.5 Hz, 1H), 2.55 (d, *J =* 6.9 Hz, 1H), 2.46 (s, 1H), 2.34 (p, *J =* 6.2, 5.7 Hz, 1H), 2.24 (s, 4H), 2.08 (qd, *J =* 8.7, 5.7, 3.9 Hz, 2H), 1.98 - 1.78 (m, 4H), 1.76 - 1.67 (m, 1H), 1.60 (dd, *J =* 9.6, 4.9 Hz, 1H), 1.43 (t, *J* = 9.2 Hz, 1H), 1.34 - 1.20 (m, 2H), 0.95 (d, *J =* 6.3 Hz, 3H).

### Example 14

### (R)-5-Chloro-N-(1-cyclobutylethyl)-6-(4-((6-(dimethylamino)spiro[3.3]heptan-2-yl)oxy)-2,6-difluorophenyl)-[1,2,4]triazolo[1,5-a]pyrimidin-7-amine 43

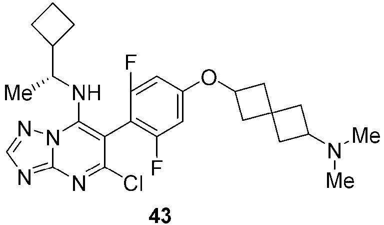

Using compound **42** (9.6 mg) as the starting material, with sodium triacetoxyborohydride (12.65 mg) and aqueous formaldehyde solution (3.0 µL, wt. 37%) as reagents, the target compound **40** (8.1 mg) was obtained as a white powder via the same method as described in Example 2.
MS ESI: m/z =517.2, [M+H]⁺.
¹H NMR (400 MHz, DMSO-*d*₆): δ 8.58 (s, 1H), 7.89 (s, 1H), 6.89 (t, *J =* 13.2 Hz, 2H), 4.77 (t, *J* = 6.9 Hz, 1H), 2.71 - 2.61 (m, 1H), 2.24 - 1.38 (m, 19H), 1.25 (d, *J =* 14.2 Hz, 2H), 0.95 (d, *J =* 6.3 Hz, 3H).

### Example 15

### 5-Chloro-6-(2,6-difluoro-4-((6-(methylamino)spiro[3.3]heptan-2-yl)oxy)phenyl)-N-(2,2,2-trifluoroethyl)-[1,2,4]triazolo[1,5-a]pyrimidin-7-amine 45

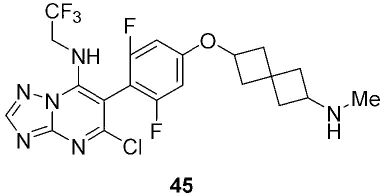

### Step 1: tert-Butyl (6-(4-(5-chloro-7-((2,2,2-trifluoroethyl)amino)-[1,2,4]triazolo[1,5-a]pyrimidin-6-yl)-3,5-difluorophenoxy)spiro[3.3]heptan-2-yl)(methyl)carbamate 44

Using intermediate **7** (100.0 mg) and tert-butyl (6-hydroxyspiro[3.3]heptan-2-yl)(methyl)carbamate **15** (253.2 mg) as starting materials, with sodium hydride (42.0 mg, 60% wt. in mineral oil) as the base, intermediate **44** (108.0 mg) was obtained under the same conditions as described in Step 1 of Example 1.

### Step 2: 5-Chloro-6-(2,6-difluoro-4-((6-(methylamino)spiro[3.3]heptan-2-yl)oxy)phenyl)-N-(2,2,2-trifluoroethyl)-[1,2,4]triazolo[1,5-a]pyrimidin-7-amine 45

Using intermediate **44** (86.9 mg) as the starting material, compound **42** (30.0 mg) was obtained as a white powder under the same conditions as described in Step 2 of Example 1.
MS ESI: m/z =503.1, [M+H]⁺.
¹H NMR (400 MHz, DMSO-*d*₆): δ 8.37 (s, 1H), 8.29 (s, 1H), 6.75 (d, *J* = 9.4 Hz, 2H), 4.74 - 4.67 (m, 3H), 3.43 (t, *J* = 7.8 Hz, 1H), 2.69 (dt, *J =* 11.4, 5.3 Hz, 1H), 2.59 - 2.54 (m, 1H), 2.36 (s, 4H), 2.29 - 2.20 (m, 1H), 2.11 (dt, *J =* 12.1, 6.3 Hz, 4H).

### Example 16

### 5-Chloro-6-(4-(((6-(dimethylamino)spiro[3.3]heptan-2-yl)oxy)-2,6-difluorophenyl)-N-(2,2,2-trifluoroethyl)-[1,2,4]triazolo[1,5-a]pyrimidin-7-amine 46

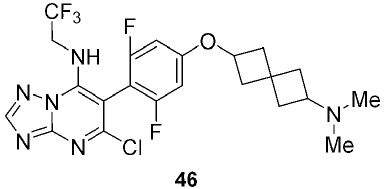

Using compound **45** (18.6 mg) as the starting material, with sodium triacetoxyborohydride (23.55 mg) and aqueous formaldehyde solution (4.3 µL, wt.37 %) as reagents, the target compound **46** (17.0 mg) was obtained as a white powder via the same method as described in Example 2.
MS ESI: m/z =517.1, [M+H]⁺.
¹H NMR (400 MHz, DMSO-*d*₆): δ 8.55 (s, 1H), 6.82 (d, *J =* 10.3 Hz, 2H), 4.73 (d, *J* = 8.7 Hz, 3H), 2.66 (t, *J* = 7.6 Hz, 2H), 2.27 - 2.19 (m, 1H), 2.08 (s, 10H), 1.89 (t, *J* = 9.7 Hz, 2H).

### Example 17

### 5-Chloro-6-(2,6-difluoro-4-((6-(methylamino)spiro[3.3]heptan-2-yl)oxy)phenyl)-N-(2,2,2-trifluoroethyl)-[1,2,4]triazolo[1,5-a]pyrimidin-7-amine 48

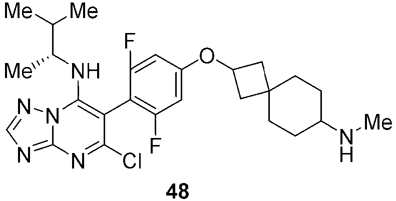

### Step 1: tert-Butyl (R)-(2-(4-(7-chloro-5-((3-methylbutan-2-yl)amino)imidazo[1,2-a]pyrimidin-6-yl)-3,5-difluorophenoxy)spiro[3.5]nonan-7-yl)(methyl)carbamate 47

Using intermediate **4** (100.0 mg) and tert-butyl (2-hydroxyspiro[3.5]nonan-7-yl)(methyl)carbamate **14** (291.8 mg) as starting materials, and sodium hydride (43.36 mg, 60% wt. in mineral oil) as the base, intermediate **47** (96.7 mg) was obtained under the same conditions as described in Step 1 of Example 1.

### Step 2: (R)-5-Chloro-6-(2,6-difluoro-4-((7-(methylamino)spiro[3.5]nonan-2-yl)oxy)phenyl)-N-(3-methylbutan-2-yl)-[1,2,4]triazolo[1,5-a]pyrimidin-7-amine 48

Using intermediate **47** (96.7 mg) as the starting material, compound **48** (35.0 mg) was obtained as a white powder under the same conditions as described in Step 2 of Example 1.
MS ESI: m/z =519.2, [M+H]⁺.
¹H NMR (400 MHz, DMSO-*d*₆): δ 8.59 (s, 1H), 8.40 (s, 1H), 6.88 (dd, *J =* 10.0, 4.5 Hz, 2H), 4.89 - 4.80 (m, 1H), 2.64 (d, *J =* 22.3 Hz, 2H), 2.36 (d, *J =* 31.2 Hz, 3H), 1.90 - 1.62 (m, 8H), 1.44 - 1.20 (m, 5H), 1.05 (d, *J =* 6.4 Hz, 3H), 0.69 (d, *J =* 6.6 Hz, 6H).

### Example 18

### (R)-5-Chloro-6-(2,6-difluoro-4-((7-(dimethylamino)spiro[3.5]nonan-2-yl)oxy)phenyl)-N-(3-methylbutan-2-yl)-[1,2,4]triazolo[1,5-a]pyrimidin-7-amine 49

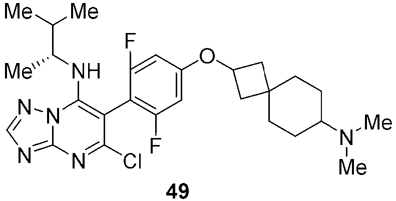

Using compound **48** (11.9 mg) as the starting material, sodium triacetoxyborohydride (14.62 mg), and aqueous formaldehyde solution (3.3 µL, wt. 37%) as reagents, the target compound **49** (9.1 mg) was obtained as a white powder via the same method as described in Example 2.
MS ESI: m/z =533.3, [M+H]⁺.
¹H NMR (400 MHz, DMSO-*d*₆): δ 8.59 (s, 1H), 7.93 (s, 1H), 6.87 (d, *J =* 10.3 Hz, 2H), 4.84 (t, *J* = 6.9 Hz, 1H), 2.31 *(d, J =* 10.6 Hz, 1H), 2.18 (s, 7H), 1.83 - 1.59 (m, 8H), 1.28 (dt, *J* = 42.8, 15.7 Hz, 5H), 1.05 (d, *J =* 6.5 Hz, 3H), 0.69 (d, *J =* 6.6 Hz, 6H).

### Example 19

### (S)-5-Chloro-6-(2,6-difluoro-4-((7-(methylamino)spiro[3.5]nonan-2-yl)oxy)phenyl)-N-(1,1,1-trifluoropropan-2-yl)-[1,2,4]triazolo[1,5-a]pyrimidin-7-amine 51

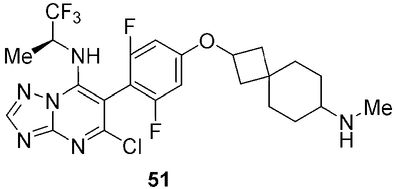

### Step 1: (S)-tert-Butyl (2-(4-(5-chloro-7-((1,1,1-trifluoropropan-2-yl)amino)-[1,2,4]triazolo[1,5-a]pyrimidin-6-yl)-3,5-difluorophenoxy)spiro[3.5]nonan-7-yl)(methyl)carbamate 50

Using intermediate **5** (100.0 mg) and (2-hydroxyspiro[3.5]nonan-7-yl)(methyl)carbamic acid tert-butyl ester **14** (272.4 mg) as starting materials, with sodium hydride (40.48 mg, 60% wt. in mineral oil) as the base, the reaction was carried out under the same conditions as described in Step 1 of Example 1 to afford intermediate **50** (60.2 mg) as a white powder.

### Step 2: (S)-5-Chloro-6-(2,6-difluoro-4-((7-(methylamino)spiro[3.5]nonan-2-yl)oxy)phenyl)-N-(1,1,1-trifluoropropan-2-yl)-[1,2,4]triazolo[1,5-a]pyrimidin-7-amine 51

Using intermediate **50** (60.2 mg) as the starting material, the reaction was carried out under the same conditions as described in Step 2 of Example 1 to afford compound **51** (40.0 mg) as a white powder.
MS ESI: m/z =545.2, [M+H]⁺.
¹H NMR (400 MHz, DMSO-*d*₆): δ 8.29 (s, 1H), 8.15 (s, 1H), 6.65 (d, *J =* 10.5 Hz, 2H), 5.76 (p, *J* = 7.8 Hz, 1H), 4.78 (p, *J =* 6.5 Hz, 1H), 2.83 (s, 1H), 2.31 (d, *J* = 9.8 Hz, 1H), 1.93 - 1.67 (m, 8H), 1.30 (dd, *J =* 54.8, 7.9 Hz, 9H).

### Example 20

### (S)-5-Chloro-6-(2,6-difluoro-4-((7-(dimethylamino)spiro[3.5]nonan-2-yl)oxy)phenyl)-N-(1,1,1-trifluoropropan-2-yl)-[1,2,4]triazolo[1,5-a]pyrimidin-7-amine 52

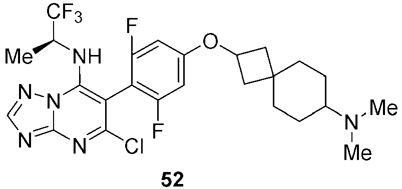

Using compound **51** (31.4 mg) as the starting material, with sodium triacetoxyborohydride (36.68 mg) and aqueous formaldehyde solution (6.67 µL, wt. 37%) as reagents, the reaction was carried out via the same method as described in Example 2 to afford the target compound 52 (22.0 mg) as a white powder.
MS ESI: m/z =559.2, [M+H]⁺.
¹H NMR (400 MHz, DMSO-*d*₆): δ 8.29 (d, *J=* 41.2 Hz, 2H), 6.72 (d, *J=* 10.6 Hz, 2H), 5.80 (q, *J* = 7.8 Hz, 1H), 4.80 (p, *J* = 6.8 Hz, 1H), 2.41 (s, 6H), 2.32 (d, *J* = 7.2 Hz, 1H), 1.87 - 1.82 (m, 2H), 1.75 - 1.69 (m, 4H), 1.39 - 1.29 (m, 6H).

### Example 21

### 6-(4-(((1R,5S,6r)-3-Azabicyclo[3.1.0]hexan-6-yl)methoxy)-2,6-difluorophenyl)-5-chloro-N-((R)-3-methylbutan-2-yl)-[1,2,4]triazolo[1,5-a]pyrimidin-7-amine 55

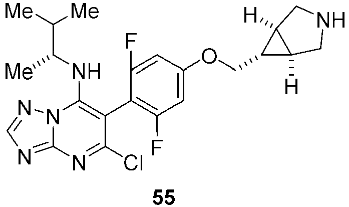

### Step 1: (1R,5S,6r)-6-((4-(5-Chloro-7-(((R)-3-methylbutan-2-yl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 54

Using intermediate **4** (100.0 mg) and (1R,5S,6r)-6-(hydroxymethyl)-3-azabicyclo[3.1.0]hexane-3-carboxylic acid tert-butyl ester **53** (231.18 mg) as starting materials, and sodium hydride (43.35 mg, 60% wt. in mineral oil) as the base, intermediate **54** (138.7 mg) was obtained under the same conditions as described in Step 1 of Example 1.

### Step 2: 6-(4-(((1R,5S,6r)-3-Azabicyclo[3.1.0]hex-6-yl)methoxy)-2,6-difluorophenyl)-5-chloro-N-((R)-3-methylbutan-2-yl)-[1,2,4]triazolo[1,5-a]pyrimidin-7-amine 55

Using intermediate **54** (138.7 mg) as the starting material, compound **55** (56.0 mg) was obtained as a white powder under the same conditions as described in Step 2 of Example 1.
MS ESI: m/z =463.1, [M+H]⁺.
¹H NMR (400 MHz, DMSO-*d*₆): δ 8.59 (s, 1H), 8.28 (s, 1H), 6.97 (dd, *J =* 10.0, 3.4 Hz, 2H), 4.00 (d, *J =* 7.2 Hz, 2H), 3.13 (d, *J =* 11.4 Hz, 2H), 3.02 (d, *J =* 11.2 Hz, 2H), 2.54 (s, 1H), 1.82 - 1.71 (m, 1H), 1.66 (s, 2H), 1.34 (dt, *J =* 7.5, 3.6 Hz, 1H), 1.06 (d, *J =* 6.5 Hz, 3H), 0.71 (d, *J =* 6.6 Hz, 6H).

### Example 22

### 5-Chloro-6-(2,6-difluoro-4-(((1R,5S,6r)-3-methyl-3-azabicyclo[3.1.0]hex-6-yl)methoxy)phenyl)-N-((R)-3-methylbutan-2-yl)-[1,2,4]triazolo[1,5-a]pyrimidin-7-amine 56

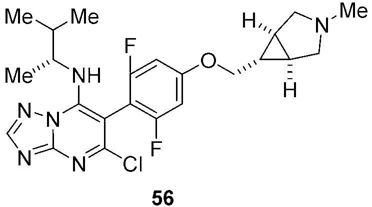

Using compound **55** (33.3 mg) as the starting material, sodium triacetoxyborohydride (45.78 mg), and aqueous formaldehyde solution (10.2 µL, wt. 37%) as reagents, the target compound **56** (20.0 mg) was obtained as a white powder via the same method as described in Example 2.
MS ESI: m/z =477.2, [M+H]⁺.
¹H NMR (400 MHz, DMSO-*d*₆): δ 8.59 (s, 1H), 7.88 (s, 1H), 6.96 (td, *J =* 7.6, 2.9 Hz, 2H), 3.93 (d, *J* = 7.3 Hz, 2H), 2.95 (d, *J =* 8.9 Hz, 2H), 2.27 (d, *J =* 23.5 Hz, 5H), 1.77 (q, *J =* 6.9 Hz, 1H), 1.56 (dq, *J =* 7.3, 3.4 Hz, 1H), 1.48 (d, *J* = 3.2 Hz, 2H), 1.05 (d, *J =* 6.5 Hz, 3H), 0.70 (d, *J* = 6.6 Hz, 6H).

### Example 23

### 6-(4-(((1R,5S,6r)-3-Azabicyclo[3.1.0]hex-6-yl)methoxy)-2,6-difluorophenyl)-5-methyl-N-((R)-3-methylbutan-2-yl)-[1,2,4]triazolo[1,5-a]pyrimidin-7-amine 55A

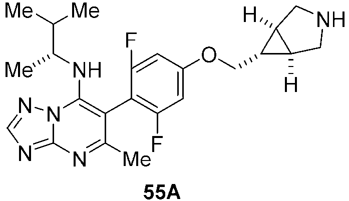

### Step 1: (1R,5S,6r)-6-((4-(5-Methyl-7-(((R)-3-methylbutan-2-yl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 54A

Using intermediate **4A** (90.0 mg) and (1R,5S,6r)-6-(hydroxymethyl)-3-azabicyclo[3.1.0]hexane-3-carboxylic acid tert-butyl ester **53** (219.67 mg) as starting materials, with sodium hydride (41.2 mg, 60% wt. in mineral oil) as the base, intermediate **54A** (118.7 mg) was obtained under the same conditions as described in Step 1 of Example 1.

### Step 2: 6-( 4-(((1R,5S,6r)-3-Azabicyclo[3.1.0]hex-6-yl)methoxy)-2,6-difluorophenyl)-5-methyl-N-((R)-3-methylbutan-2-yl)-[1,2,4]triazolo[1,5-a]pyrimidin-7-amine 55A

Using intermediate **54A** (118.7 mg) as the starting material, compound **55A** (40.0 mg) was obtained as a white powder under the same conditions as described in Step 2 of Example 1.
MS ESI: m/z =443.2, [M+H]⁺.
¹H NMR (400 MHz, DMSO-*d*₆): δ 8.48 (s, 1H), 7.21 (d, *J =* 10.2 Hz, 1H), 7.00 - 6.95 (m, 2H), 3.99 (d, *J =* 7.1 Hz, 2H), 3.12 (d, *J =* 11.3 Hz, 2H), 3.01 (d, *J =* 11.2 Hz, 2H), 2.13 (s, 3H), 1.65 (t, *J =* 2.7 Hz, 4H), 1.32 (tt, *J =* 6.9, 3.2 Hz, 1H), 1.02 (d, *J =* 6.5 Hz, 3H), 0.70 (d, *J =* 6.5 Hz, 6H).

### Example 24

### 6-(4-(((1R,5S,6r)-3-Azabicyclo[3.1.0]hex-6-yl)methoxy)-2,6-difluorophenyl)-5-chloro-N-((S)-1,1,1-trifluoropropan-2-yl)-[1,2,4]triazolo[1,5-a]pyrimidin-7-amine 58

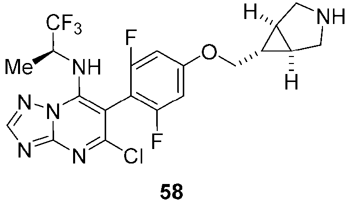

### Step 1: (1R,5S,6r)-6-((4-(5-Chloro-7-(((S)-1,1,1-trifluoropropan-2-yl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 57

Using intermediate **5** (100.0 mg) and (1R,5S,6r)-6-(hydroxymethyl)-3-azabicyclo[3.1.0]hexane-3-carboxylic acid tert-butyl ester **53** (215.93 mg) as starting materials, with sodium hydride (40.5 mg, 60% wt. in mineral oil) as the base, intermediate **57** (140.0 mg) was obtained under the same conditions as described in Step 1 of Example 1.

### Step 2: 6-(4-(((1R,5S,6r)-3-Azabicyclo[3.1.0]hex-6-yl)methoxy)-2,6-difluorophenyl)-5-chloro-N-((S)-1,1,1-trifluoropropan-2-yl)-[1,2,4]triazolo[1,5-a]pyrimidin-7-amine 58

Using intermediate **57** (140.0 mg) as the starting material, compound **58** (39.0 mg) was obtained as a white powder under the same conditions as described in Step 2 of Example 1.
MS ESI: m/z =489.0, [M+H]⁺.
¹H NMR (400 MHz, DMSO-*d*₆): δ 8.43 (s, 1H), 8.21 (s, 1H), 6.85 (dd, *J =* 9.9, 4.1 Hz, 2H), 5.91 - 5.80 (m, 1H), 3.97 (d, *J =* 7.0 Hz, 2H), 3.26 (d, *J =* 11.5 Hz, 2H), 3.18 (d, *J =* 11.3 Hz, 2H), 1.76 (t, *J =* 2.9 Hz, 2H), 1.36 (dd, *J =* 19.4, 5.2 Hz, 4H).

### Example 25

### 5-Chloro-6-(2,6-difluoro-4-(((1R,5S,6r)-3-methyl-3-azabicyclo[3.1.0]hexan-6-yl)methoxy)phenyl)-N-((S)-1,1,1-trifluoropropan-2-yl)-[1,2,4]triazolo[1,5-a]pyrimidin-7-amine 59

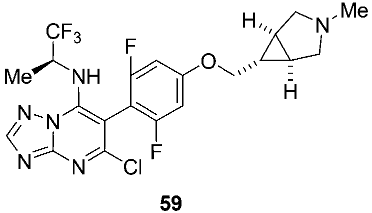

Using compound **58** (28.7 mg) as the starting material, sodium triacetoxyborohydride (37.38 mg), and aqueous formaldehyde solution (8.3 µL, wt.37 %) as reagents, the target compound **59** (15.0 mg) was obtained as a white powder via the same method as described in Example 2.
MS ESI: m/z =503.1, [M+H]⁺.
¹H NMR (400 MHz, DMSO-*d*₆): δ 8.60 (s, 1H), 6.90 (d, *J =* 10.2 Hz, 2H), 5.96 - 5.84 (m, 1H), 3.91 (d, *J* = 7.2 Hz, 2H), 3.00 (d, *J =* 9.0 Hz, 2H), 2.36 (d, *J* = 9.1 Hz, 2H), 2.28 (s, 3H), 1.60 - 1.48 (m, 3H), 1.39 (d, *J* = 6.8 Hz, 3H).

### Example 26

### 6-(4-(((1R,5S,6r)-3-Azabicyclo[3.1.0]hexan-6-yl)methoxy)-2,6-difluorophenyl)-5-chloro-N-((R)-1-cyclobutylethyl)-[1,2,4]triazolo[1,5-a]pyrimidin-7-amine 61

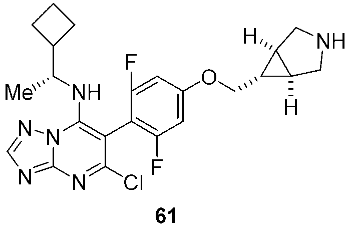

### Step 1: (1R,5S,6r)-6-((4-(5-Chloro-7-(((R)-1-cyclobutylethyl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 60

Using intermediate **6** (100.0 mg) and (1R,5S,6r)-6-(hydroxymethyl)-3-azabicyclo[3.1.0]hexane-3-carboxylic acid tert-butyl ester 53 (223.85 mg) as starting materials, with sodium hydride (42.0 mg, 60% wt. in mineral oil) as the base, intermediate **60** (137.3 mg) was obtained under the same conditions as described in Step 1 of Example 1.

### Step 2: 6-(4-(((1R,5S,6r)-3-Azabicyclo[3.1.0]hexan-6-yl)methoxy)-2,6-difluorophenyl)-5-chloro-N-((R)-1-cyclobutylethyl)-[1,2,4]triazolo[1,5-a]pyrimidin-7-amine 61

Using intermediate **60** (131.9 mg) as the starting material, compound **61** (65.0 mg) was obtained as a white powder under the same conditions as described in Step 2 of Example 1.
MS ESI: m/z =475.1, [M+H]⁺.
¹H NMR (400 MHz, DMSO-*d*₆): δ 8.59 (s, 1H), 8.28 (s, 1H), 6.98 (s, 2H), 4.00 (d, *J=* 7.0 Hz, 2H), 3.08 (d, *J* = 11.3 Hz, 2H), 2.95 (d, *J =* 11.1 Hz, 2H), 1.85 (ddt, *J =* 22.9, 11.0, 3.7 Hz, 3H), 1.72 (dt, *J =* 10.8, 8.2 Hz, 2H), 1.61 (s, 3H), 1.46 (t, *J* = 9.2 Hz, 1H), 1.32 (dd, *J =* 7.2, 4.0 Hz, 2H), 0.96 (d, *J=* 6.4 Hz, 3H).

### Example 27

### 5-Chloro-N-((R)-1-cyclobutylethyl)-6-(2,6-difluoro-4-(((1R,5S,6r)-3-methyl-3-azabicyclo[3.1.0]hexan-6-yl)methoxy)phenyl)-[1,2,4]triazolo[1,5-a]pyrimidin-7-amine 62

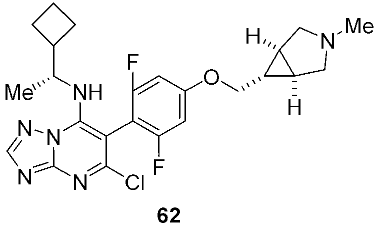

Using compound **61** (47.2 mg) as the starting material, sodium triacetoxyborohydride (63.26 mg), and aqueous formaldehyde solution (14.4 µL, wt. 37%) as reagents, the target compound **62** (30.0 mg) was obtained as a white powder via the same method as described in Example 2.
MS ESI: m/z =489.2, [M+H]⁺.
¹H NMR (400 MHz, DMSO-*d*₆): δ 8.59 (s, 1H), 7.84 (s, 1H), 7.03 - 6.94 (m, 2H), 3.94 (d, *J* = 7.4 Hz, 2H), 2.95 (d, *J =* 8.8 Hz, 2H), 2.47 (d, *J =* 8.3 Hz, 1H), 2.26 (d, *J* = 21.8 Hz, 5H), 1.94 - 1.53 (m, 6H), 1.47 (q, *J =* 7.6, 5.2 Hz, 3H), 1.31 (t, *J =* 9.4 Hz, 1H), 0.96 (d, *J =* 6.4 Hz, 3H).

### Example 28

### 6-(4-(((1R,5S,6r)-3-Azabicyclo[3.1.0]hexan-6-yl)methoxy)-2,6-difluorophenyl)-5-methyl-N-((R)-1-cyclobutylethyl)-[1,2,4]triazolo[1,5-a]pyrimidin-7-amine 61A

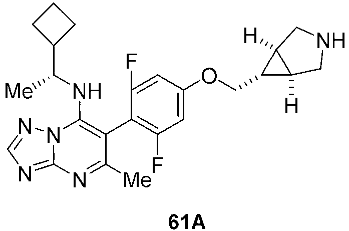

### Step 1: (1R,5S,6r)-6-((4-(5-Methyl-7-(((R)-1-cyclobutylethyl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 60A

Using intermediate **6A** (100.0 mg) and (1R,5S,6r)-6-(hydroxymethyl)-3-azabicyclo[3.1.0]hexane-3-carboxylic acid tert-butyl ester 53 (236.3 mg) as starting materials, and sodium hydride (44.4 mg, 60% wt. in mineral oil) as the base, intermediate **60A** (72.4 mg) was obtained under the same conditions as described in Step 1 of Example 1.

### Step 2: 6-(4-(((1R,5S,6r)-3-Azabicyclo[3.1.0]hexan-6-yl)methoxy)-2,6-difluorophenyl)-5-methyl-N-((R)-1-cyclobutylethyl)-[1,2,4]triazolo[1,5-a]pyrimidin-7-amine 61

Using intermediate **60A** (72.4 mg) as the starting material, compound **61A** (30.0 mg) was obtained as a white powder under the same conditions as described in Step 2 of Example 1.
MS ESI: m/z =455.2, [M+H]⁺.
¹H NMR (400 MHz, DMSO-*d*₆): δ 8.47 (s, 1H), 8.31 (s, 1H), 7.20 (d, *J =* 10.0 Hz, 1H), 6.99 (t, *J* = 11.6 Hz, 2H), 4.00 (d, *J* = 7.1 Hz, 2H), 3.08 (d, *J =* 11.2 Hz, 2H), 2.95 (d, *J =* 11.2 Hz, 2H), 2.46 - 2.39 (m, 1H), 2.12 (s, 3H), 1.85 (dqd, *J =* 23.0, 7.9, 4.0 Hz, 2H), 1.71 (dt, *J =* 10.7, 8.4 Hz, 1H), 1.60 (d, *J =* 13.0 Hz, 4H), 1.45 (p, *J* = 8.7 Hz, 1H), 1.35 - 1.25 (m, 2H), 0.93 (d, *J* = 6.3 Hz, 3H).

### Example 29

### 6-(4-(((1R,5S,6s)-3-Azabicyclo[3.1.0]hexan-6-yl)methoxy)-2,6-difluorophenyl)-5-chloro-N-((R)-3-methylbutan-2-yl)-[1,2,4]triazolo[1,5-a]pyrimidin-7-amine 65

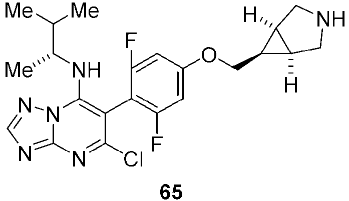

### Step 1: (1R,5S,6s)-6-((4-(5-Chloro-7-(((R)-3-methylbutan-2-yl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 64

Using intermediate **4** (100.0 mg) and (1R,5S,6s)-6-(hydroxymethyl)-3-azabicyclo[3.1.0]hexane-3-carboxylic acid tert-butyl ester **63** (231.18 mg) as starting materials, with sodium hydride (43.35 mg, 60% wt. in mineral oil) as the base, intermediate **64** (111.3 mg) was obtained under the same conditions as described in Step 1 of Example 1.

### Step 2: 6-(4-(((1R,5S,6s)-3-Azabicyclo[3.1.0]hex-6-yl)methoxy)-2,6-difluorophenyl)-5-chloro-N-((R)-3-methylbutan-2-yl)-[1,2,4]triazolo[1,5-a]pyrimidin-7-amine 65

Using intermediate **64** (113.3 mg) as the starting material, compound **65** (56.0 mg) was obtained as a white powder under the same conditions as described in Step 2 of Example 1.
MS ESI: m/z =463.1, [M+H]⁺.
¹H NMR (400 MHz, DMSO-*d*₆): δ 8.60 (s, 1H), 7.89 (s, 1H), 7.00 (dd, *J* = 9.8, 2.8 Hz, 2H), 4.24 (d, *J =* 7.4 Hz, 2H), 3.27 (d, *J =* 11.2 Hz, 2H), 3.05 (d, *J =* 11.4 Hz, 2H), 1.90 - 1.72 (m, 4H), 1.43 (p, *J =* 7.8 Hz, 1H), 1.06 (d, *J =* 6.5 Hz, 3H), 0.71 (d, *J =* 6.6 Hz, 6H).

### Example 30

### 5-Chloro-6-(2,6-difluoro-4-(((1R,5S,6s)-3-methyl-3-azabicyclo[3.1.0]hex-6-yl)methoxy)phenyl)-N-((R)-3-methylbutan-2-yl)-[1,2,4]triazolo[1,5-a]pyrimidin-7-amine 66

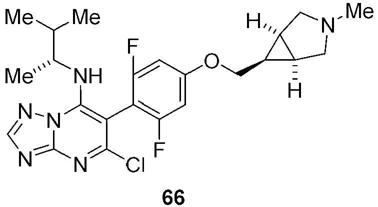

Using compound **65** (30.3 mg) as the starting material, with sodium triacetoxyborohydride (41.685 mg) and aqueous formaldehyde solution (8.5 µL, wt. 37%) as reagents, the target compound **66** (15.0 mg) was obtained as a white powder via the same method as described in Example 2.
MS ESI: m/z =477.2, [M+H]⁺.
¹H NMR (400 MHz, DMSO-*d*₆): δ 8.59 (s, 1H), 7.87 (s, 1H), 6.98 - 6.90 (m, 2H), 4.46 (d, *J* = 7.1 Hz, 2H), 2.98 (d, *J* = 9.4 Hz, 2H), 2.21 (s, 3H), 1.77 (q, *J* = 6.9 Hz, 2H), 1.67 - 1.60 (m, 2H), 1.28 - 1.18 (m, 2H), 1.06 (d, *J =* 6.5 Hz, 4H), 0.70 (d, *J =* 6.6 Hz, 6H).

### Example 31

### 6-(4-(((1R,5S,6s)-3-Azabicyclo[3.1.0]hex-6-yl)methoxy)-2,6-difluorophenyl)-5-chloro-N-((S)-1,1,1-trifluoropropan-2-yl)-[1,2,4]triazolo[1,5-a]pyrimidin-7-amine 68

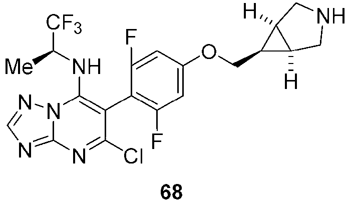

### Step 1: (1R,5S,6s)-6-((4-(5-Chloro-7-(((S)-1,1,1-trifluoropropan-2-yl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 67

Using intermediate **5** (100.0 mg) and (1R,5S,6s)-6-(hydroxymethyl)-3-azabicyclo[3.1.0]hexane-3-carboxylic acid tert-butyl ester **63** (215.93 mg) as starting materials, with sodium hydride (40.5 mg, 60% wt. in mineral oil) as base, intermediate **67** (106.7 mg) was obtained under the same conditions as described in Step 1 of Example 1.

### Step 2: 6-(4-(((1R,5S,6s)-3-azabicyclo[3.1.0]hexan-6-yl)methoxy)-2,6-difluorophenyl)-5-chloro-N-((S)-1,1,1-trifluoropropan-2-yl)-[1,2,4]triazolo[1,5-a]pyrimidin-7-amine 68

Using intermediate **67** (106.7 mg) as starting material, compound **68** (20.0 mg) was obtained as a white powder under the same conditions as described in Step 2 of Example 1.
MS ESI: m/z =489.1, [M+H]⁺.
¹H NMR (400 MHz, DMSO-*d*₆): δ 8.37 (s, 1H), 8.15 (s, 1H), 6.83 (d, *J =* 10.6 Hz, 2H), 5.81 (t, *J* = 7.5 Hz, 1H), 4.12 (d, *J* = 7.6 Hz, 2H), 3.36 (d, *J =* 10.6 Hz, 2H), 3.05 (d, *J =* 11.8 Hz, 2H), 1.93 (dd, *J =* 8.1, 3.3 Hz, 2H), 1.46 (p, *J =* 7.9 Hz, 1H), 1.27 (d, *J* = 6.8 Hz, 3H).

### Example 32

### 5-Chloro-6-(2,6-difluoro-4-(((1R,5S,6s)-3-methyl-3-azabicyclo[3.1.0]hexan-6-yl)methoxy)phenyl)-N-((S)-1,1,1-trifluoropropan-2-yl)-[1,2,4]triazolo[1,5-a]pyrimidin-7-amine 69

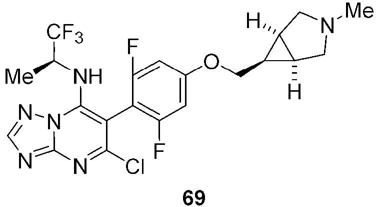

Using compound **68** (10.7 mg) as starting material, with sodium triacetoxyborohydride (14.0 mg) and formaldehyde aqueous solution (2.53 µL, 37% wt.) as reagents, target compound **69** (7.0 mg) was obtained as a white powder via the same method as described in Example 2.
MS ESI: m/z =503.1, [M+H]⁺.

### Example 33

### 6-(4-(((1R,5S,6r)-3-azabicyclo[3.1.0]hexan-6-yl)methoxy)-2,6-difluorophenyl)-5-chloro-N-(2,2,2-trifluoroethyl)-[1,2,4]triazolo[1,5-a]pyrimidin-7-amine 71

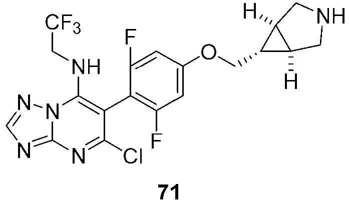

### Step 1 : (1R,5S,6r)-6-((4-(5-chloro-7-((2,2,2-trifluoroethyl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 70

Using intermediate **7** (100.0 mg) and (1R,5S,6r)-6-(hydroxymethyl)-3-azabicyclo[3.1.0]hexane-3-carboxylic acid tert-butyl ester **53** (224.0 mg) as starting materials, with sodium hydride (42.0 mg, 60% wt. in mineral oil) as base, intermediate **70** (134.8 mg) was obtained under the same conditions as described in Step 1 of Example 1.

### Step 2: 6-(4-(((1R,5S,6r)-3-Azabicyclo[3.1.0]hexan-6-yl)methoxy)-2,6-difluorophenyl)-5-chloro-N-(2,2,2-trifluoroethyl)-[1,2,4]triazolo[1,5-a]pyrimidin-7-amine 71

Using intermediate **70** (126.1 mg) as starting material, compound **71** (41.0 mg) was obtained as a white powder under the same conditions as described in Step 2 of Example 1.
MS ESI: m/z =475.0, [M+H]⁺.
¹H NMR (400 MHz, DMSO-*d*₆): δ 8.49 (s, 1H), 8.25 (s, 1H), 6.89 (d, *J =* 9.6 Hz, 2H), 4.72 (q, *J* = 9.2 Hz, 2H), 3.98 (d, *J =* 6.8 Hz, 2H), 3.20 (d, *J =* 11.2 Hz, 2H), 3.11 (d, *J =* 11.2 Hz, 2H), 1.71 (s, 2H), 1.39 - 1.33 (m, 1H).

### Example 34

### 5-Chloro-6-(2,6-difluoro-4-(((1R,5S,6r)-3-methyl-3-azabicyclo[3.1.0]hexan-6-yl)methoxy)phenyl)-N-(2,2,2-trifluoroethyl)-[1,2,4]triazolo[1,5-a]pyrimidin-7-amine 72

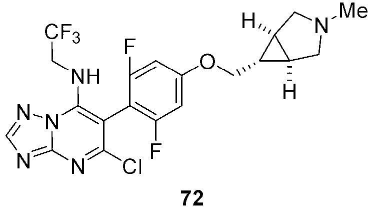

Using compound **71** (23.1 mg) as the starting material, sodium triacetoxyborohydride (30.98 mg), and aqueous formaldehyde solution (5.61 µL, wt. 37%) as reagents, the target compound **72** (17.0 mg) was obtained as a white powder via the same method as described Example 2.
MS ESI: m/z =489.1, [M+H]⁺.
¹H NMR (400 MHz, DMSO-*d*₆): δ 8.52 (s, 1H), 6.86 (d, *J=* 9.8 Hz, 2H), 4.67 (q, *J =* 9.0 Hz, 2H), 3.84 (d, *J = 7.2* Hz, 2H), 2.91 (d, *J =* 8.9 Hz, 2H), 2.23 (d, *J =* 8.8 Hz, 2H), 2.18 (s, 3H), 1.50 (d, *J* = 3.2 Hz, 1H), 1.41 (d, *J* = 2.9 Hz, 2H).

### Example 35

### 6-(4-(((1R,5S,6r)-3-Azabicyclo[3.1.0]hexan-6-yl)methoxy)-2,6-difluorophenyl)-5-chloro-N-(2,2,2-trifluoroethyl)-[1,2,4]triazolo[1,5-a]pyrimidin-7-amine 71A

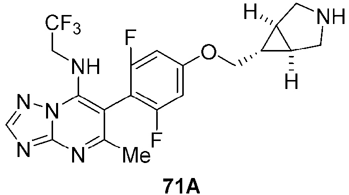

### Step 1: (1R,5S,6r)-6-((4-(5-Methyl-7-((2,2,2-trifluoroethyl)amino)-[1,2,4]triazolo[1,5-a]pyrimidin-6-yl)-3,5-difluorophenoxy)methyl)-3-azabicyclo[3.1.0]hexane-3-carboxylic acid tert-butyl ester 70A

Using intermediate **7A** (100.0 mg) and (1R,5S,6r)-6-(hydroxymethyl)-3-azabicyclo[3.1.0]hexane-3-carboxylic acid tert-butyl ester **53** (236.08 mg) as starting materials, and sodium hydride (44.3 mg, 60% wt. in mineral oil) as the base, the same conditions as described in Step 1 of Example 1 were applied to obtain intermediate **70A** (132.8 mg).

### Step 2: 6-(4-(((1R,5S,6r)-3-Azabicyclo[3.1.0]hexan-6-yl)methoxy)-2,6-difluorophenyl)-5-methyl-N-(2,2,2-trifluoroethyl)-[1,2,4]triazolo[1,5-a]pyrimidin-7-amine 71A

Using intermediate **70A** (125.1 mg) as the starting material, compound **71A** (70.0mg) was obtained as a white powder under the same conditions as described in Step 2 of Example 1.
MS ESI: m/z =469.2, [M+H]⁺.
¹H NMR (400 MHz, DMSO-*d*₆): δ 8.49 (s, 1H), 8.23 (s, 1H), 6.90 (d, *J =* 9.6 Hz, 2H), 4.63 (q, *J* = 9.0 Hz, 2H), 3.92 (d, *J =* 6.9 Hz, 2H), 3.11 (d, *J* = 11.3 Hz, 2H), 3.00 (d, *J =* 11.3 Hz, 2H), 2.10 (s, 3H), 1.62 (s, 2H), 1.30 (dq, *J =* 7.3, 3.5 Hz, 1H).

### Example 36

### 5-Chloro-6-(2,6-difluoro-4-(((3aR,5r,6aS)-octahydrocyclopenta[c]pyrrol-5-yl)oxy)phenyl)-N-((R)-3-methylbutan-2-yl)-[1,2,4]triazolo[1,5-a]pyrimidin-7-amine 74

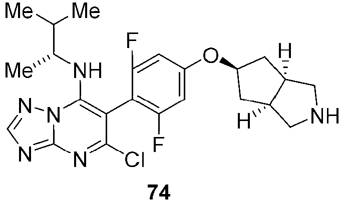

### Step 1: (3aR,5r,6aS)-5-(4-(5-Chloro-7-(((R)-3-methylbutan-2-yl)amino)-[1,2,4]triazolo[1,5-a]pyrimidin-6-yl)-3,5-difluorophenoxy)hexahydrocyclopenta[c]pyrrole-2(1H)-carboxylic acid tert-butyl ester 73

Using intermediate **4** (200.0 mg) and (3aR,5r,6aS)-5-hydroxyhexahydrocyclopenta[c]pyrrole-2(1H)-carboxylic acid tert-butyl ester **17** (492.46 mg) as starting materials, and sodium hydride (87.0 mg, 60% wt. in mineral oil) as the base, intermediate **73** (270 mg) was obtained under the same conditions as described in Step 1 of Example 1.

### Step 2: 5-Chloro-6-(2,6-difluoro-4-(((3aR,5r,6aS)-octahydrocyclopenta[c]pyrrol-5-yl)oxy)phenyl)-N-((R)-3-methylbutan-2-yl)-[1,2,4]triazolo[1,5-a]pyrimidin-7-amine 74

Using intermediate **73** (270 mg) as the starting material, compound **74** (90.0 mg) was obtained as a white powder under the same conditions as described in Step 2 of Example 1.
MS ESI: m/z =477.1, [M+H]⁺.
¹H NMR (400 MHz, DMSO-*d*₆): δ 8.60 (s, 1H), 8.36 (s, 1H), 7.04 - 7.00 (m, 2H), 4.97 (t, *J* = 5.2 Hz, 1H), 3.26 - 3.21 (m, 2H), 2.92 (dd, *J* = 11.7, 4.4 Hz, 2H), 2.81 - 2.75 (m, 2H), 2.26 - 2.19 (m, 2H), 1.74 (tt, *J =* 13.8, 5.7 Hz, 4H), 1.05 (d, *J =* 6.5 Hz, 3H), 0.69 (d, *J =* 6.6 Hz, 7H).

### Example 37

### 5-Chloro-6-(2,6-difluoro-4-(((3aR,5r,6aS)-2-methyloctahydrocyclopenta[c]pyrrol-5-yl)oxy)phenyl)-N-((R)-3-methylbutan-2-yl)-[1,2,4]triazolo[1,5-a]pyrimidin-7-amine 75

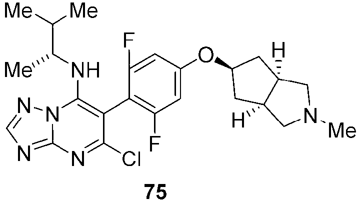

Using compound **74** (40.0 mg) as the starting material, sodium triacetoxyborohydride (53.4 mg), and aqueous formaldehyde solution (14.34 µL, wt. 37%) as reagents, the target compound **75** (33.1 mg) was obtained as a white powder via the same method as described in Example 2.
MS ESI: m/z =491.2, [M+H]⁺.
¹H NMR (400 MHz, DMSO-*d*₆): δ 8.59 (s, 1H), 7.94 (s, 1H), 6.97 (dd, *J =* 11.4, 5.4 Hz, 2H), 4.85 - 4.77 (m, 1H), 2.55 (s, 2H), 2.43 - 2.35 (m, 4H), 2.24 (s, 6H), 1.77 (q, *J =* 6.9 Hz, 1H), 1.53 (dt, *J =* 13.1, 6.1 Hz, 2H), 1.05 (d, *J =* 6.5 Hz, 3H), 0.69 (d, *J =* 6.7 Hz, 6H).

### Example 38

### 5-Chloro-6-(2,6-difluoro-4-(((3aR,5r,6aS)-octahydrocyclopenta[c]pyrrol-5-yl)oxy)phenyl)-N-((S)-1,1,1-trifluoropropan-2-yl)-[1,2,4]triazolo[1,5-a]pyrimidin-7-amine 77

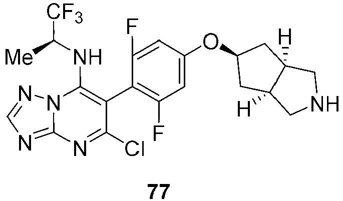

### Step 1: (3aR,5r,6aS)-5-(((S)-1,1,1-Trifluoropropan-2-yl)amino)-[1,2,4]triazolo[1,5-a]pyrimidin-6-yl)-3,5-difluorophenoxy)hexahydrocyclopenta[c]pyrrole-2(1H)-carboxylic acid tert-butyl ester 76

Using intermediate **5** (200.0 mg) and (3aR,5r,6aS)-5-hydroxyhexahydrocyclopenta[c]pyrrole-2(1H)-carboxylic acid tert-butyl ester **17** (459.75 mg) as starting materials, and sodium hydride (81.0 mg, 60% wt. in mineral oil) as the base, intermediate **76** (280.7mg) was obtained under the same conditions as described in Step 1 of Example 1.

### Step 2: 5-Chloro-6-(2,6-difluoro-4-(((3aR,5r,6aS)-octahydrocyclopenta[c]pyrrol-5-yl)oxy)phenyl)-N-((S)-1,1,1-trifluoropropan-2-yl)-[1,2,4]triazolo[1,5-a]pyrimidin-7-amine 77

Using intermediate**76** (280.7 mg) as starting material, compound **77** (120.0 mg) was obtained as a white powder under the same conditions as described in Step 2 of Example 1.
MS ESI: m/z =503.0 [M+H]⁺.
¹H NMR (400 MHz, DMSO-*d*₆): δ 8.32 (s, 1H), 8.24 (d, *J* = 4.7 Hz, 1H), 6.85 (d, *J* = 10.7 Hz, 2H), 5.78 (q, *J =* 7.2 Hz, 1H), 4.95 (p, *J* = 4.5, 4.1 Hz, 1H), 3.36 (dt, *J* = 12.5, 4.3 Hz, 2H), 3.01 (dd, *J* = 11.3, 5.0 Hz, 2H), 2.84 (s, 2H), 2.22 (dd, *J =* 13.8, 6.8 Hz, 2H), 1.78 (dd, *J =* 11.8, 7.0 Hz, 2H), 1.29 (d, *J =* 5.8 Hz, 3H).

### Example 39

### 5-Chloro-6-(2,6-difluoro-4-(((3aR,5r,6aS)-2-methyloctahydrocyclopenta[c]pyrrol-5-yl)oxy)phenyl)-N-((S)-1,1,1-trifluoropropan-2-yl)-[1,2,4]triazolo[1,5-a]pyrimidin-7-amine 78

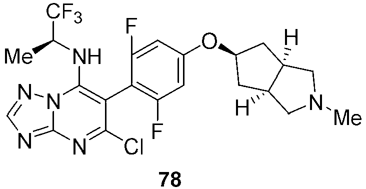

Using compound **77** (38.8 mg) as starting material, sodium triacetoxyborohydride (49.1 mg), and aqueous formaldehyde solution (13.2µL, wt.37 %) as reagents, the target compound **78** (35.0 mg) was obtained as a white powder via the same method as described in Example 2.
MS ESI: m/z =517.1, [M+H]⁺.
¹H NMR (400 MHz, DMSO-*d*₆): δ 8.50 (s, 1H), 8.19 (s, 1H), 6.90 (d, *J =* 10.6 Hz, 2H), 5.88 - 5.80 (m, 1H), 4.82 (t, *J =* 6.4 Hz, 1H), 2.64 (s, 6H), 2.41 (s, 3H), 2.29 - 2.24 (m, 2H), 1.61 (d, *J =* 10.5 Hz, 2H), 1.36 (d, *J =* 6.7 Hz, 3H).

### Example 40

### (2,6-Difluoro-4-(((3aR,5s,6aS)-octahydrocyclopenta[c]pyrrol-5-yl)oxy)phenyl)-N-((R)-3-methylbutan-2-yl)-[1,2,4]triazolo[1,5-a]pyrimidin-7-amine 80

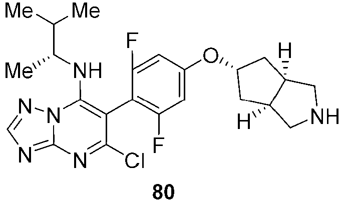

### Step 1: (3aR,5s,6aS)-5-(4-(5-Chloro-7-(((R)-3-methylbutan-2-yl)amino)-[1,2,4]triazolo[1,5-a]pyrimidin-6-yl)-3,5-difluorophenoxy)hexahydrocyclopenta[c]pyrrole-2(1H)-carboxylic acid tert-butyl ester 79

Using intermediate **4** (100.0 mg) and (3aR,5s,6aS)-5-hydroxyhexahydrocyclopenta[c]pyrrole-2(1H)-carboxylic acid tert-butyl ester **19** (246.23 mg) as starting materials, and sodium hydride (43.36 mg, 60% wt. in mineral oil) as the base, intermediate **79** (150.0 mg) was obtained under the same conditions as described in Step 1 of Example 1.

### Step 2:(2,6-Difluoro-4-(((3aR,5s,6aS)-octahydrocyclopenta[c]pyrrol-5-yl)oxy)phenyl)-N-((R)-3-methylbutan-2-yl)-[1,2,4]triazolo[1,5-a]pyrimidin-7-amine 80

Using intermediate **79** (150 mg) as the starting material, compound **80** (75.0 mg) was obtained as a white powder under the same conditions as described in Step 2 of Example 1.
MS ESI: m/z =477.1, [M+H]⁺.
¹H NMR (400 MHz, DMSO-*d*₆): δ 8.60 (s, 1H), 8.37 (s, 1H), 6.99 (dd, *J =* 10.5, 4.7 Hz, 2H), 5.11 - 5.07 (m, 1H), 3.10 (dd, *J =* 11.5, 6.4 Hz, 2H), 2.94 (d, *J =* 10.9 Hz, 2H), 2.85 (s, 2H), 2.03 (dq, *J =* 13.0, 3.7 Hz, 2H), 1.90 - 1.71 (m, 4H), 1.05 (d, *J =* 6.5 Hz, 3H), 0.70 (d, *J =* 6.6 Hz, 6H).

### Example 41

### 5-Chloro-6-(2,6-difluoro-4-(((3aR,5s,6aS)-2-methyloctahydrocyclopenta[c]pyrrol-5-yl)oxy)phenyl)-N-((R)-3-methylbutan-2-yl)-[1,2,4]triazolo[1,5-a]pyrimidin-7-amine 81

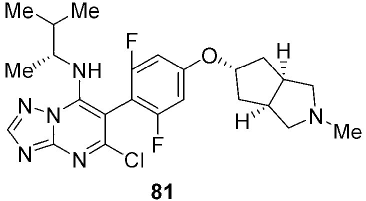

Using compound **80** (25.6 mg) as the starting material, sodium triacetoxyborohydride (34.2 mg), and aqueous formaldehyde solution (6.19 µL, wt.37 %) as reagents, the target compound **81** (25.0 mg) was obtained as a white powder via the same method as described in Example 2.
MS ESI: m/z =491.1, [M+H]⁺.
¹H NMR (400 MHz, CDCl₃): δ 8.32 (s, 1H), 6.60 - 6.55 (m, 2H), 6.28 (d, *J =* 10.6 Hz, 1H), 4.91 (d, *J =* 4.6 Hz, 1H), 3.31 (s, 1H), 2.93 - 2.86 (m, 2H), 2.75 (d, *J* = 9.7 Hz, 2H), 2.43 (d, *J* = 9.4 Hz, 4H), 2.25 - 2.19 (m, 2H), 1.87 (dt, *J =* 14.5, 5.4 Hz, 2H), 1.65 (dt, *J =* 12.9, 6.6 Hz, 1H), 1.25 (s, 1H), 1.06 (d, *J =* 6.6 Hz, 3H), 0.80 (t, *J =* 6.6 Hz, 6H).

### Example 42

### 5-Chloro-6-(2,6-difluoro-4-(((3aR,5s,6aS)-octahydrocyclopenta[c]pyrrol-5-yl)oxy)phenyl)-N-((S)-1,1,1-trifluoropropan-2-yl)-[1,2,4]triazolo[1,5-a]pyrimidin-7-amine 83

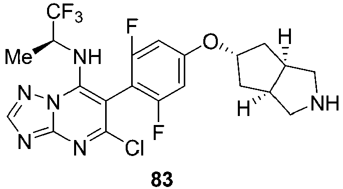

### Step 1: (3aR,5s,6aS)-5-(4-(5-Chloro-7-(((S)-1,1,1-trifluoropropan-2-yl)amino)-[1,2,4]triazolo[1,5-a]pyrimidin-6-yl)-3,5-difluorophenoxy)hexahydrocyclopenta[c]pyrrole-2(1H)-carboxylic acid tert-butyl ester 82

Using intermediate **5** (88.9 mg) and (3aR,5s,6aS)-5-hydroxyhexahydrocyclopenta[c]pyrrole-2(1H)-carboxylic acid tert-butyl ester**19** (204.5 mg) as starting materials, with sodium hydride (36.0 mg, 60% wt. in mineral oil) as the base, intermediate **82** (115.0 mg) was obtained under the same conditions as described in Step 1 of Example 1.

### Step 2: 5-Chloro-6-(2,6-difluoro-4-(((3aR,5s,6aS)-octahydrocyclopenta[c]pyrrol-5-yl)oxy)phenyl)-N-((S)-1,1,1-trifluoropropan-2-yl)-[1,2,4]triazolo[1,5-a]pyrimidin-7-amine 83

Using intermediate **82** (95.1 mg) as the starting material, compound 83 (50.0 mg) was obtained as a white powder under the same conditions as described in Step 2 of Example 1.
MS ESI: m/z =503.1, [M+H]⁺.
¹H NMR (400 MHz, DMSO-*d*₆): δ 8.29 (d, *J =* 21.9 Hz, 2H), 6.83 (d, *J =* 10.7 Hz, 2H), 5.86 - 5.75 (m, 1H), 5.04 (t, *J =* 4.5 Hz, 1H), 3.24 (dd, *J =* 11.8, 6.4 Hz, 2H), 3.05 (d, *J =* 11.6 Hz, 2H), 2.93 (s, 2H), 2.08 - 1.99 (m, 2H), 1.90 (d, *J =* 13.3 Hz, 2H), 1.29 (d, *J =* 6.6 Hz, 3H).

### Example 43

### 5-Chloro-6-(2,6-difluoro-4-(((3aR,5s,6aS)-2-methyloctahydrocyclopenta[c]pyrrol-5-yl)oxy)phenyl)-N-((S)-1,1,1-trifluoropropan-2-yl)-[1,2,4]triazolo[1,5-a]pyrimidin-7-amine 84

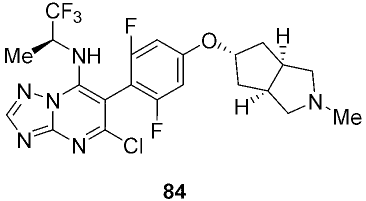

Using compound **83** (40.9 mg) as the starting material, sodium triacetoxyborohydride (51.5 mg), and aqueous formaldehyde solution (8.9 µL, wt.37 %) as reagents, the target compound **84** (33.0 mg) was obtained as a white powder via the same method as described in Example 2.
MS ESI: m/z =517.1, [M+H]⁺.
¹H NMR (400 MHz, DMSO-*d*₆): δ 8.51 (s, 1H), 6.85 (d, *J =* 10.8 Hz, 2H), 5.84 (q, *J =* 7.6 Hz, 1H), 5.05 - 5.00 (m, 1H), 2.75 (d, *J =* 9.5 Hz, 2H), 2.61 (d, *J =* 9.4 Hz, 2H), 2.45 (d, *J =* 7.2 Hz, 2H), 2.34 (s, 3H), 2.04 (dq, *J =* 13.2, 3.5 Hz, 2H), 1.77 (dt, *J =* 13.7, 5.0 Hz, 2H), 1.36 (d, *J =* 6.8 Hz, 3H).

### Example 44

### 6-(4-(((1R,5S,6s)-3-Azabicyclo[3.1.0]hexan-6-yl)ethynyl)-2,6-difluorophenyl)-5-chloro-N-((R)-3-methylbutan-2-yl)-[1,2,4]triazolo[1,5-a]pyrimidin-7-amine hydrochloride86

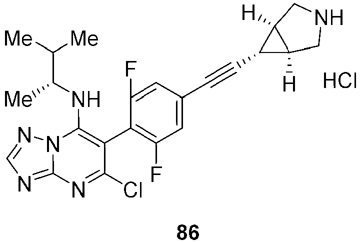

### Step 1: tert-Butyl (1R,5S,6s)-6-(((R)-3-methylbutan-2-yl)amino)-[1,2,4]triazolo[1,5-a]pyrimidin-6-yl)-3,5-difluorophenyl)ethynyl)-3-azabicyclo[3.1.0]hexane-3-carboxylate 85

Intermediate **4B** (100.0 mg) was dissolved in N,N-dimethylformamide (1.24 mL), followed by sequential addition of intermediate**19A**(130.3 mg), tetrakis(triphenylphosphine)palladium (24.22 mg), copper(I) iodide (5.98 mg), and triethylamine (88.0 µL). The reaction was carried out at 80°C under inert atmosphere for 20 minutes, then at room temperature for 8 hours. Saturated sodium chloride solution and ethyl acetate were added for extraction. The organic phase was dried over anhydrous sodium sulfate, filtered, and concentrated to afford intermediate **85** (116.9 mg) as a yellow oil.

### Step 2: 6-(4-(((1R,5S,6s)-3-Azabicyclo[3.1.0]hex-6-yl)ethynyl)-2,6-difluorophenyl)-5-chloro-N-((R)-3-methylbutan-2-yl)-[1,2,4]triazolo[1,5-a]pyrimidin-7-amine hydrochloride 86

Intermediate **85** (116.9 mg) was dissolved in methanol (2.1 mL) at room temperature, and 4.0 M hydrogen chloride in dioxane solution (0.95 mL) was added. The mixture was stirred at room temperature for 5 hours. The solvent was evaporated, and the residue was purified by Pre-HPLC(eluent: acetonitrile and water containing 1‰ formic acid) to afford compound **86** (40.0 mg) as a white powder.
MS ESI: m/z =457.1, [M+H]⁺.
¹H NMR (400 MHz, DMSO-*d*₆): δ 8.60 (d, J = 2.2 Hz, 1H), 8.31 - 8.18 (m, 1H), 7.41 - 7.36 (m, 2H), 3.06 - 3.00 (m, 2H), 2.82 (d, J = 11.4 Hz, 2H), 1.94 - 1.90 (m, 2H), 1.76 (q, J = 6.9 Hz, 1H), 1.59 (q, J = 3.1 Hz, 1H), 1.05 (d, J = 6.5 Hz, 4H), 0.69 (d, J = 6.6 Hz, 6H).

### Example 45

### 6-(4-(((1R,5S,6s)-3-Azabicyclo[3.1.0]hex-6-yl)ethynyl)-2,6-difluorophenyl)-5-chloro-N-((R)-1-cyclobutylethyl)-[1,2,4]triazolo[1,5-a]pyrimidin-7-amine hydrochloride 88

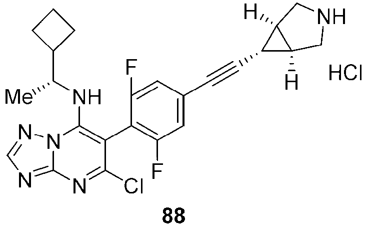

### Step 1: tert-Butyl (1R,5S,6s)-6-((4-(5-chloro-7-(((R)-1-cyclobutylethyl)amino)-[1,2,4]triazolo[1,5-a]pyrimidin-6-yl)-3,5-difluorophenyl)ethynyl)-3-azabicyclo[3.1.0]hexane-3-carboxylate 87

Intermediate **6B** (100.0 mg) was dissolved in N,N-dimethylformamide (1.20 mL), followed by sequential addition of intermediate **19A** (127.1 mg), tetrakis(triphenylphosphine)palladium (23.63 mg), copper(I) iodide (5.84 mg), and triethylamine (85.3 µL). The reaction was carried out at room temperature for 8 hours. Saturated sodium chloride solution and ethyl acetate were added for extraction. The organic phase was dried over anhydrous sodium sulfate, filtered, and concentrated to afford intermediate **87** (109.5 mg) as a yellow oil.

### Step 2: 6-(4-(((1R,5S,6s)-3-Azabicyclo[3.1.0]hex-6-yl)ethynyl)-2,6-difluorophenyl)-5-chloro-N-((R)-1-cyclobutylethyl)-[1,2,4]triazolo[1,5-a]pyrimidin-7-amine hydrochloride 88

Intermediate **87** (109.5 mg) was dissolved in methanol (1.92 mL) at room temperature, and 4.0 M hydrogen chloride in dioxane solution (0.86 mL) was added. The mixture was stirred at room temperature for 5 hours. The solvent was evaporated, and the residue was purified by Pre-HPLC (eluent: acetonitrile and water containing 1‰ formic acid) to afford compound **88** (51.0 mg) as a white powder.
MS ESI: m/z =469.1, [M+H]⁺.
¹H NMR (400 MHz, DMSO-*d*₆): δ 8.54 (s, 1H), 8.17 (s, 1H), 7.34 (t, *J =* 10.9 Hz, 2H), 2.97 (d, *J* = 11.6 Hz, 2H), 2.76 (d, *J =* 11.5 Hz, 2H), 2.39 (d, *J =* 8.4 Hz, 1H), 1.88 - 1.61 (m, 6H), 1.53 (h, *J =* 6.6, 5.5 Hz, 2H), 1.39 (t, *J =* 9.1 Hz, 1H), 1.25 (d, *J =* 13.7 Hz, 1H), 0.90 (d, *J =* 6.3 Hz, 3H).

### Example 46

### 5-Chloro-N-((R)-1-cyclobutylethyl)-6-(2,6-difluoro-4-(((1R,5S,6s)-3-methyl-3-azabicyclo[3.1.0]hexan-6-yl)ethynyl)phenyl)-[1,2,4]triazolo[1,5-a]pyrimidin-7-amine 89

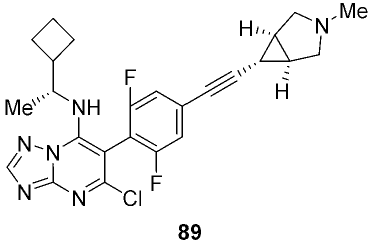

Using compound **88** (22.7 mg) as the starting material, sodium triacetoxyborohydride (28.5 mg), and aqueous formaldehyde solution (5.2 µL, wt. 37%) as reagents, the target compound **89** (20.0 mg) was obtained as a white powder via the same method as Example 2.
MS ESI: m/z =483.2, [M+H]⁺.
¹H NMR (400 MHz, DMSO-*d*₆): δ 8.60 (s, 1H), 7.90 (s, 1H), 7.39 (t, *J =* 10.8 Hz, 2H), 2.99 (d, *J* = 9.2 Hz, 2H), 2.45 (d, *J =* 10.1 Hz, 1H), 2.25 (d, *J =* 24.4 Hz, 5H), 1.94 - 1.53 (m, 8H), 1.46 (q, *J* = 9.1 Hz, 1H), 1.37 - 1.25 (m, 1H), 0.96 (d, *J =* 6.3 Hz, 3H).

### Example 47

### (6-(4-(((1R,5S,6s)-3-Azabicyclo[3.1.0]hex-6-yl)ethynyl)-2,6-difluorophenyl)-5-chloro-N-((S)-1,1,1-trifluoropropan-2-yl)-[1,2,4]triazolo[1,5-a]pyrimidin-7-amine hydrochloride91

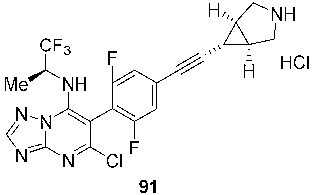

### Step 1: tert-Butyl (1R,5S,6s)-6-((4-(5-chloro-7-(((S)-1,1,1-trifluoropropan-2-yl)amino)-[1,2,4]triazolo[1,5-a]pyrimidin-6-yl)-3,5-difluorophenyl)ethynyl)-3-azabicyclo[3.1.0]hexane-3-carboxylate 90

Using intermediate **5B** (100.0 mg) and intermediate **19A** (123.5 mg) as starting materials, tetrakis(triphenylphosphine)palladium (22.97 mg) and copper(I) iodide (5.675 mg) as catalysts, and triethylamine (83.0µL) as the base, intermediate **90** (122.9 mg) was obtained as a yellow oil via the same synthetic method as described in Step 1 of Example 45.

### Step 2: (6-(4-(((1R,5S,6s)-3-Azabicyclo[3.1.0]hex-6-yl)ethynyl)-2,6-difluorophenyl)-5-chloro-N-((S)-1,1,1-trifluoropropan-2-yl)-[1,2,4]triazolo[1,5-a]pyrimidin-7-amine hydrochloride 91

Using intermediate **90** (122.9 mg) as the starting material, compound **91** (60.0 mg) was obtained as a white powder via the same synthetic method as described in Step 2 of Example 45. MS ESI: m/z =483.1, [M+H]⁺.
¹H NMR (400 MHz, DMSO-*d*₆): δ 8.37 (s, 1H), 8.18 (s, 1H), 7.24 (t, *J =* 7.8 Hz, 2H), 5.98 - 5.87 (m, 1H), 3.21 (d, *J =* 11.6 Hz, 2H), 3.05 (d, *J =* 11.4 Hz, 2H), 2.04 (t, *J =* 2.8 Hz, 2H), 1.67 (t, *J =* 3.6 Hz, 1H), 1.30 (d, *J =* 6.8 Hz, 3H).

### Example 48

### 5-Chloro-6-(2,6-difluoro-4-(((1R,5S,6s)-3-methyl-3-azabicyclo[3.1.0]hexan-6-yl)ethynyl)phenyl)-N-((S)-1,1,1-trifluoropropan-2-yl)-[1,2,4]triazolo[1,5-a]pyrimidin-7-amine 92

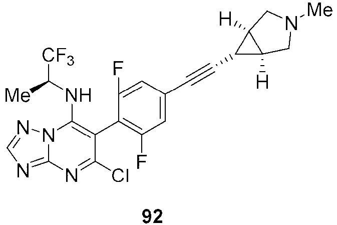

Using **Example 47** (29.6 mg) as the starting material, sodium triacetoxyborohydride (36.2 mg), and aqueous formaldehyde solution (6.6 µL, wt. 37%) as reagents, the target compound **92** (25.0 mg) was obtained as a white powder via the same method as Example 2.
MS ESI: m/z =497.1, [M+H]⁺.
¹H NMR (400 MHz, DMSO-*d*₆): δ 8.67 (s, 1H), 8.14 (s, 1H), 7.33 (s, 2H), 6.04 (s, 1H), 3.05 (d, *J* = 9.4 Hz, 2H), 2.37 (d, *J =* 9.4 Hz, 4H), 2.27 (s, 4H), 1.91 (s, 2H), 1.86 (d, *J =* 3.4 Hz, 2H), 1.42 - 1.38 (m, 3H).

### Example 49

### 6-(4-(((1R,5S,6s)-3-Azabicyclo[3.1.0]hexan-6-yl)ethynyl)-2,6-difluorophenyl)-5-chloro-N-(2,2,2-trifluoroethyl)-[1,2,4]triazolo[1,5-a]pyrimidin-7-amine hydrochloride 94

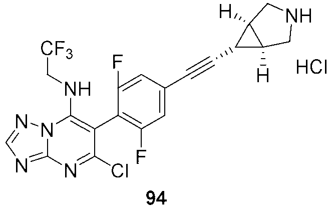

### Step 1: tert-Butyl (1R,5S,6s)-6-((4-(5-chloro-7-((2,2,2-trifluoroethyl)amino)-[1,2,4]triazolo[1,5-a]pyrimidin-6-yl)-3,5-difluorophenyl)ethynyl)-3-azabicyclo[3.1.0]hexane-3-carboxylate 93

Using intermediate **7B** (100.0mg) and intermediate **19A** (127.1 mg) as starting materials, tetrakis(triphenylphosphine)palladium (23.63 mg) and copper(I) iodide (5.84 mg) as catalysts, and triethylamine (85.3µL) as the base, intermediate **93** (88.6 mg) was obtained as a yellow oil via the same synthetic method as described in Step 1 of Example 45.

### Step 2: 6-(4-(((1R,5S,6s)-3-Azabicyclo[3.1.0]hexan-6-yl)ethynyl)-2,6-difluorophenyl)-5-chloro-N-(2,2,2-trifluoroethyl)-[1,2,4]triazolo[1,5-a]pyrimidin-7-amine hydrochloride 94

Using Intermediate **93** (88.6 mg) as the starting material, compound **94** (40.0 mg) was obtained as a white powder via the same synthetic method as described in Step 2 of Example 45.
MS ESI: m/z =469.0, [M+H]⁺.
¹H NMR (400 MHz, DMSO-*d*₆): δ 8.44 (s, 1H), 8.20 (s, 1H), 7.29 (d, *J =* 7.5 Hz, 2H), 4.76 (q, *J* = 9.4 Hz, 2H), 3.13 (d, *J =* 11.6 Hz, 2H), 2.94 (d, *J =* 11.4 Hz, 2H), 2.00 - 1.96 (m, 2H), 1.63 (t, *J* = 3.6 Hz, 1H).

### Example 50

### 5-Chloro-6-(2,6-difluoro-4-(((1R,5S,6s)-3-methyl-3-azabicyclo[3.1.0]hexan-6-yl)ethynyl)phenyl)-N-(2,2,2-trifluoroethyl)-[1,2,4]triazolo[1,5-a]pyrimidin-7-amine

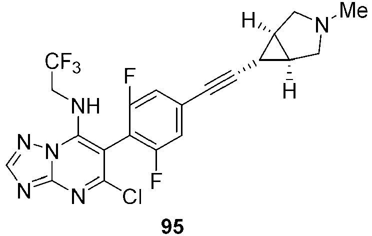

Using compound **94** (22.7 mg) as the starting material, sodium triacetoxyborohydride (28.5 mg), and aqueous formaldehyde solution (5.2 µL, wt. 37%) as reagents, the target compound **95** (16.0 mg) was obtained as a white powder via the same method as Example 2.
MS ESI: m/z =483.1, [M+H]⁺.
¹H NMR (400 MHz, DMSO-*d*₆): δ 8.61 (s, 1H), 8.15 (s,1H), 7.34 (d, *J =* 7.9 Hz, 2H), 4.77 (q, *J* = 9.1 Hz, 2H), 3.02 (d, *J* = 9.2 Hz, 2H), 2.30 (d, *J* = 9.1 Hz, 2H), 2.24 (s, 3H), 1.87 (dt, *J* = 17.6, 2.9 Hz, 3H).

### Example 51

### 6-(4-((E)-2-((1R,5S,6s)-3-Azabicyclo[3.1.0]hexan-6-yl)vinyl)-2,6-difluorophenyl)-5-chloro-N-((R)-3-methylbutan-2-yl)-[1,2,4]triazolo[1,5-a]pyrimidin-7-amine hydrochloride 97

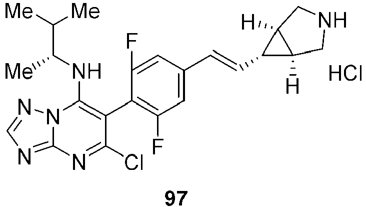

### Step 1: tert-Butyl (1R,5S,6s)-6-((E)-4-(5-chloro-7-(((R)-3-methylbutan-2-yl)amino)-[1,2,4]triazolo[1,5-a]pyrimidin-6-yl)-3,5-difluorostyryl)-3-azabicyclo[3.1.0]hexane-3-carboxylate 96

Intermediate **4B** (100.0 mg) was dissolved in N-methylpyrrolidone (3.0 mL), followed by sequential addition of intermediate **19B** (209.28 mg), tetrakis(triphenylphosphine)palladium (24.2 mg), and copper(I) iodide (4.0 mg). The reaction was carried out at 80°C for 1 hour. Saturated sodium chloride and ethyl acetate were added for extraction. The organic phase was dried over anhydrous sodium sulfate, filtered, and concentrated to afford intermediate **96** (110.9 mg) as a yellow oil.

### Step 2: 6-(4-((E)-2-((1R,5S,6s)-3-Azabicyclo[3.1.0]hexan-6-yl)vinyl)-2,6-difluorophenyl)-5-chloro-N-((R)-3-methylbutan-2-yl)-[1,2,4]triazolo[1,5-a]pyrimidin-7-amine hydrochloride 97

Using intermediate **96** (110.9 mg) as the starting material, the target compound **97** (70.0 mg) was obtained as a white powder via the same synthetic method as described in Step 2 of Example 45.
MS ESI: m/z =459.1, [M+H]⁺.
¹H NMR (400 MHz, DMSO-*d*₆): δ 8.54 (d, *J =* 3.6 Hz, 1H), 8.24 (s, 1H), 7.29 (d, *J =* 10.0 Hz, 2H), 6.38 (d, *J* = 15.9 Hz, 1H), 6.22 (dd, *J =* 15.9, 9.0 Hz, 1H), 3.00 (d, *J =* 11.4 Hz, 2H), 2.86 (d, *J =* 11.2 Hz, 2H), 1.73 - 1.66 (m, 3H), 1.56 (dt, *J =* 6.8, 3.3 Hz, 1H), 0.98 (d, *J =* 6.6 Hz, 4H), 0.62 (dt, *J =* 6.8, 4.4 Hz, 6H).

### Example 52

### 6-(4-((E)-2-((1R,5S,6s)-3-Azabicyclo[3.1.0]hexan-6-yl)vinyl)-2,6-difluorophenyl)-5-chloro-N-((R)-1-cyclobutylethyl)-[1,2,4]triazolo[1,5-a]pyrimidin-7-amine hydrochloride 99

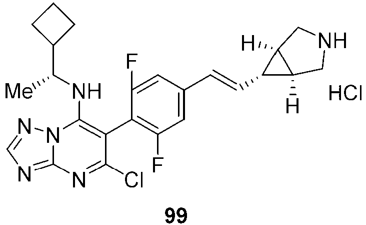

### Step 1: tert-Butyl (1R,5S,6s)-6-((E)-4-(5-chloro-7-(((R)-1-cyclobutylethyl)amino)-[1,2,4]triazolo[1,5-a]pyrimidin-6-yl)-3,5-difluorostyryl)-3-azabicyclo[3.1.0]hexane-3-carboxylate 98

Using intermediate **6B** (100.0 mg) and intermediate **19B** (204.2 mg) as starting materials, with tetrakis(triphenylphosphine)palladium (23.6 mg) and copper(I) iodide (4.0 mg) as catalysts, intermediate **98** (127.9 mg) was obtained as a yellow oil via a similar method as described in Step 1 of Example 51.

### Step 2: 6-(4-((E)-2-((1R,5S,6s)-3-Azabicyclo[3.1.0]hexan-6-yl)vinyl)-2,6-difluorophenyl)-5-chloro-N-((R)-1-cyclobutylethyl)-[1,2,4]triazolo[1,5-a]pyrimidin-7-amine hydrochloride 99

Using intermediate **98** (127.9 mg) as the starting material, the target compound **99** (90.0 mg) was obtained as a white powder via the same synthetic method as described in Step 2 of Example 45.
MS ESI: m/z =471.1, [M+H]⁺.
¹H NMR (400 MHz, DMSO-*d*₆): δ 8.60 (s, 1H), 8.31 (s, 1H), 7.38 (t, *J =* 10.8 Hz, 2H), 6.46 (d, *J* = 15.9 Hz, 1H), 6.30 (dd, *J =* 15.9, 9.1 Hz, 1H), 3.08 (d, *J =* 11.4 Hz, 2H), 2.94 (d, *J =* 11.2 Hz, 2H), 2.44 (d, *J =* 8.1 Hz, 1H), 1.91 - 1.79 (m, 2H), 1.76 - 1.54 (m, 6H), 1.45 (p, *J =* 8.7 Hz, 1H), 1.25 (s, 1H), 0.95 (d, *J =* 6.4 Hz, 3H).

### Example 53

### 6-(4-((E)-2-((1R,5S,6s)-3-Azabicyclo[3.1.0]hexan-6-yl)vinyl)-2,6-difluorophenyl)-5-chloro-N-((S)-1,1,1-trifluoropropan-2-yl)-[1,2,4]triazolo[1,5-a]pyrimidin-7-amine hydrochloride 101

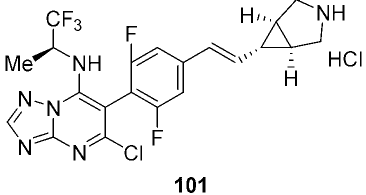

### Step 1: tert-Butyl (1R,5S,6s)-6-((E)-4-(5-chloro-7-(((S)-1,1,1-trifluoropropan-2-yl)amino)-[1,2,4]triazolo[1,5-a]pyrimidin-6-yl)-3,5-difluorostyryl)-3-azabicyclo[3.1.0]hexane-3-carboxylate 100

Using intermediate **5B** (70.0 mg) and intermediate **19B** (138.99 mg) as starting materials, with tetrakis(triphenylphosphine)palladium (16.1 mg) and copper(I) iodide (2.65 mg) as catalysts, intermediate **100** (78.1 mg) was obtained as a yellow oil via a similar method as described in Step 1 of Example 51.

### Step 2: 6-(4-((E)-2-((1R,5S,6s)-3-Azabicyclo[3.1.0]hexan-6-yl)vinyl)-2,6-difluorophenyl)-5-chloro-N-((S)-1,1,1-trifluoropropan-2-yl)-[1,2,4]triazolo[1,5-a]pyrimidin-7-amine hydrochloride 101

Using intermediate **100** (78.1 mg) as the starting material, compound **101** (40.0 mg) was obtained as a white powder via the same method as described in Step 2 of Example 45.
MS ESI: m/z =485.1, [M+H]⁺.

### Example 54

### 6-(4-((E)-2-((1R,5S,6s)-3-Azabicyclo[3.1.0]hexan-6-yl)vinyl)-2,6-difluorophenyl)-5-chloro-N-(2,2,2-trifluoroethyl)-[1,2,4]triazolo[1,5-a]pyrimidin-7-amine hydrochloride103

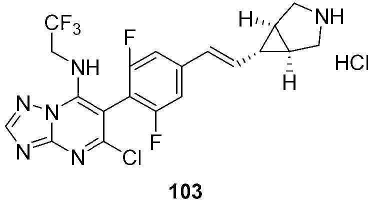

### Step 1: tert-Butyl (1R,5S,6s)-6-((E)-4-(5-chloro-7-((2,2,2-trifluoroethyl)amino)-[1,2,4]triazolo[1,5-a]pyrimidin-6-yl)-3,5-difluorostyryl)-3-azabicyclo[3.1.0]hexane-3-carboxylate102

Using intermediate**7B** (120.0 mg) and intermediate **19B** (245.1 mg) as starting materials, with tetrakis(triphenylphosphine)palladium (28.36 mg) and copper(I) iodide (4.67 mg) as catalysts, intermediate **102** (235.7 mg) was obtained as a yellow oil via the similar method as described in Step 1 of Example 51.

### Step 2: 6-(4-((E)-2-((IR,5S,6s)-3-Azabicyclo[3.1.0]hexan-6-yl)vinyl)-2,6-difluorophenyl)-5-chloro-N-(2,2,2-trifluoroethyl)-[1,2,4]triazolo[1,5-a]pyrimidin-7-amine hydrochloride 103

Using intermediate **102** (235.7 mg) as the starting material, compound **103** (160.0 mg) was obtained as a white powder via the synthetic method in Step 2 of Example 45.
MS ESI: m/z =471.1, [M+H]⁺.
¹H NMR (400 MHz, DMSO-*d*₆): δ 8.42 (s, 1H), 8.25 (s, 1H), 7.26 (d, *J =* 8.8 Hz, 2H), 6.45 (d, *J* = 15.8 Hz, 1H), 6.22 (dd, *J =* 15.9, 8.9 Hz, 1H), 4.71 (q, *J =* 9.3 Hz, 2H), 3.21 (d, *J =* 11.5 Hz, 2H), 3.11 (d, *J* = 11.3 Hz, 2H), 1.83 (t, *J =* 2.6 Hz, 2H), 1.67 (dt, *J =* 9.4, 3.3 Hz, 1H).

### Example 55

### 5-Chloro-6-(2,6-difluoro-4-(((1R,5S,6s)-3-methyl-3-azabicyclo[3.1.0]hexan-6-yl)ethynyl)phenyl)-N-((R)-3-methylbutan-2-yl)-[1,2,4]triazolo[1,5-a]pyrimidin-7-amine

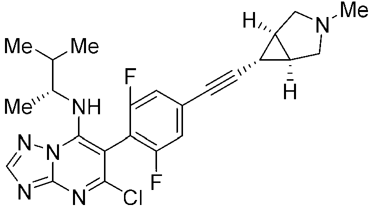

Using Example 44 (24.7 mg) as the starting material, with sodium triacetoxyborohydride (31.9 mg) and aqueous formaldehyde solution (5.83 µL, wt. 37%) as reagents, the target compound (12.0 mg) was obtained as a white powder via the same method as described in Example 2.
MS ESI: m/z =471.1, [M+H]⁺.

### Example 56

### 5-Chloro-N-((R)-1-cyclobutylethyl)-6-(2,6-difluoro-4-((E)-2-((1R,5S,6s)-3-methyl-3-azabicyclo[3.1.0]hexan-6-yl)vinyl)phenyl)-[1,2,4]triazolo[1,5-a]pyrimidin-7-amine

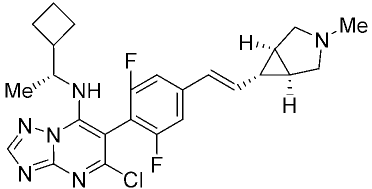

Using Example 52 (27.1 mg) as the starting material, sodium triacetoxyborohydride (34.04 mg), and aqueous formaldehyde solution (6.2 µL, wt. 37 %) as reagents, the target compound (20.0 mg) was obtained as a white powder via the same method as described in Example 2.
MS ESI: m/z =485.2, [M+H]⁺.

### Example 57

### 6-(2,6-Difluoro-4-(((1R,5S,6r)-3-methyl-3-azabicyclo[3.1.0]hexan-6-yl)methoxy)phenyl)-5-methyl-N-((R)-3-methylbutan-2-yl)-[1,2,4]triazolo[1,5-a]pyrimidin-7-amine

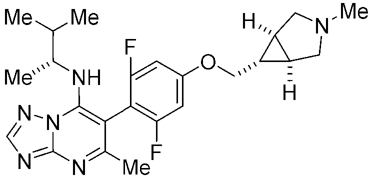

Using Example 23 (20.1 mg) as the starting material, sodium triacetoxyborohydride (29.0 mg), and aqueous formaldehyde solution (5.3 µL, wt. 37 %) as reagents, the target compound (13.0 mg) was obtained as a white powder via the same method as described in Example 2.
MS ESI: m/z =457.2, [M+H]⁺.

### Example 58

### 5-Chloro-6-(difluoro-4-((E)-2-((1R,5S,6s)-3-methyl-3-azabicyclo[3.1.0]hexan-6-yl)vinyl)phenyl)-N-(2,2,2-trifluoroethyl)-[1,2,4]triazolo[1,5-a]pyrimidin-7-amine

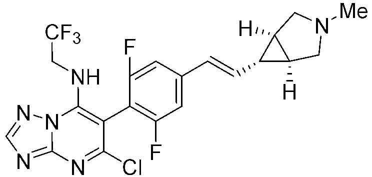

Using Example 54 (34.4 mg) as the starting material, sodium triacetoxyborohydride (43.2 mg), and aqueous formaldehyde solution (7.8 µL, wt. 37 %) as reagents, the target compound (22.0 mg) was obtained as a white powder via the same method as described in Example 2.
MS ESI: m/z =485.1, [M+H]⁺.

### Example 59

### 5-Chloro-6-(2,6-difluoro-4-((E)-2-((1R,5S,6s)-methyl-3-azabicyclo[3.1.0]hexan-6-yl)vinyl)phenyl)-N-((R)-3-methylbutan-2-yl)-[1,2,4]triazolo[1,5-a]pyrimidin-7-amine

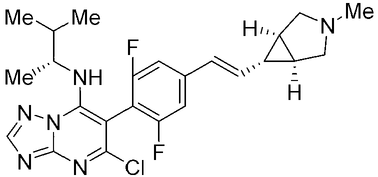

Using Example 51 (28.5 mg) as the starting material, sodium triacetoxyborohydride (36.7 mg), and aqueous formaldehyde solution (6.7 µL, wt. 37 %) as reagents, the target compound (15.0 mg) was obtained as a white powder via the same method as described in Example 2.
MS ESI: m/z =473.1, [M+H]⁺.

### Example 60

### 6-(2,6-Difluoro-4-(((1R,5S,6r)-methyl-3-azabicyclo[3.1.0]hexan-6-yl)methoxy)phenyl)-5-methyl-N-(2,2,2-trifluoroethyl)-[1,2,4]triazolo[1,5-a]pyrimidin-7-amine

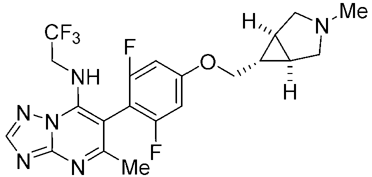

Using Example 35 (41.6 mg) as the starting material, sodium triacetoxyborohydride (58.2 mg), and aqueous formaldehyde solution (10.7 µL, wt. 37%) as reagents, the target compound (23.0 mg) was obtained as a white powder via the same method as Example 2.
MS ESI: m/z =469.1, [M+H]⁺.

### Example 61

### 5-Chloro-6-(2,6-difluoro-4-(((1R,5S,6s)-3-ethyl-3-azabicyclo[3.1.0]hexan-6-yl)ethynyl)phenyl)-N-((S)-1,1,1-trifluoropropan-2-yl)-[1,2,4]triazolo[1,5-a]pyrimidin-7-amine 92

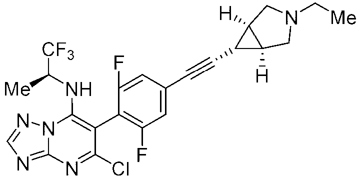

Using Example 47 (40 mg) as the starting material, dissolved in anhydrous N,N-dimethylformamide (2 mL), ethyl bromide (7 µL) was added under ice bath cooling, followed by stirring for 30 minutes, and then cesium carbonate (36 mg) was added. The reaction mixture was stirred at 60°C in an oil bath for 4 hours, then cooled to room temperature, quenched with saturated brine, and extracted with ethyl acetate (10 mL × 3). The combined organic phases were washed with saturated brine, dried over anhydrous sodium sulfate, filtered, concentrated, and purified by HPLC to afford the target product T-1: 5-chloro-6-(2,6-difluoro-4-(((1R,5S,6s)-3-ethyl-3-azabicyclo[3.1.0]hexan-6-yl)ethynyl)phenyl)-N-((S)-1,1,1-trifluoropropan-2-yl)-[1,2,4]triazolo[1,5-a]pyrimidin-7-amine (8 mg).
MS-ESI: m/z =511.0 [M+H]⁺.

### Example 62

### 5-Chloro-6-(2,6-difluoro-4-(((1R,5S,6s)-3-isopropyl-3-azabicyclo[3.1.0]hexan-6-yl)ethynyl)phenyl)-N-((S)-1,1,1-trifluoropropan-2-yl)-[1,2,4]triazolo[1,5-a]pyrimidin-7-amine 92

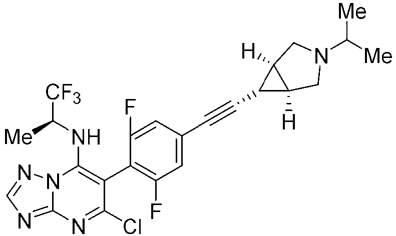

Using Example 47 (40 mg) as the starting material, isopropyl iodide (10 µL) and Cs₂CO₃ (35 mg) as reagents, the target compound (6.0 mg) was obtained via the same method as described in Example 61.
MS ESI: m/z =525.0, [M+H]⁺.

### Example 63

### 5-Chloro-6-(2,6-difluoro-4-(((1R,5S,6s)-3-methyl-d₃-3-azabicyclo[3.1.0]hexan-6-yl)ethynyl)phenyl)-N-((S)-1,1,1-trifluoropropan-2-yl)-[1,2,4]triazolo[1,5-a]pyrimidin-7-amine 92

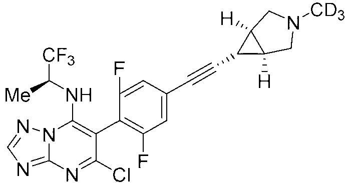

Using Example 47 (80 mg) as the starting material, dissolved in anhydrous N,N-dimethylformamide (2 mL), CD₃I (15 µL) and Cs₂CO₃ (60.1 mg) as reagents, the target compound (16.0 mg) was obtained via the same method as described in Example 61.
MS-ESI: m/z =500.0 [M+H]⁺.

### Example 64

### Microtubule-stabilizing Activity Assay

**Compound solvent:** 100% dimethyl sulfoxide (DMSO).
**Cell line:** HEK293T (ATCC, CRL3216).
**Culture conditions:** Cells were cultured in Dulbecco's Modified Eagle's Medium (DMEM) containing 10% fetal bovine serum and 1% penicillin/streptomycin. Cells were cultured at 37°C in a Thermo-Fisher incubator with a humidified atmosphere of 5% carbon dioxide.

### Test method:

One day prior to the experiment, 100,000 cells were seeded at a density of 200,000 cells/ml in a 24-well plate.

On the day of the experiment, 20 µl of PBS phosphate buffer containing the test compound at experimental concentrations or an equivalent volume of DMSO was added to each well. After incubating at 37°C for 1 hour, colchicine was added to each well at a final concentration of 1 µM, followed by an additional 3-hour incubation at 37°C. The plate was then placed on ice. The medium in each well was removed, and the cells were washed with ice-cold PBS. After aspirating the PBS, 0.1 mL of ice-cold RIPA lysis buffer (containing 0.5% sodium deoxycholate, 0.1% SDS, 1% NP-40, 5 mM EDTA, pH 8.0) was quickly added. Prior to use, a commercial protease inhibitor cocktail (1:500) and 1 µM deacetylase inhibitor Trichostatin A (Sangon Biotech Co., Ltd.) were added to the cell lysis buffer. The cell lysate were transferred to a 1.5 ml centrifuge tube, sonicated, and centrifuged at 13,000 rpm for 10 minutes. The supernatant was collected, and protein concentration was determined using the BCA assay.

Based on existing literature (Black, 1989; Kurt, 2011), acetylated microtubule was used as an indicator of microtubule polymerization to assess the microtubule-stabilizing efficacy of the compounds. HEK293T cell lysates were analyzed by SDS-PAGE (7.5% gel) and immunoblotted using acetylated microtubule antibody (1:20,000, Sigma-Aldrich, T7451) and α-microtubule antibody (1:2,000, Proteintech, 11224-1-AP). After incubation with fluorescent dye-conjugated IgG (1:40,000, Licor), the blots were visualized using an Odyssey-Dlx imaging system. Grayscale analysis was performed using ImageJ.

The compound activity was quantified with the microtubule-stabilizing activity of CNDR-51657 (C5) (Jane, 2016) at 500 nM as 1.00. The test results of microtubule-stabilizing activity for each embodiment are shown in Table 1.

**Table 1 Microtubule-Stabilizing Activity Test Results**

| Example | Microtubule-Stabilizing Activity | |
|---|---|---|
| | 500 nM | 50 nM |
| Intermediate **4** (CNDR-51657) | 1.00 | 0.2-0.3 |
| Cevipubulin | 1.68 | 1.65 |
| **1** | 0.71 | 0.36 |
| **2** | 0.71 | 0.29 |
| **3** | 0.49 | 0.36 |
| **4** | 0.87 | 0.36 |
| **5** | 0.76 | 0.41 |
| **6** | 0.38 | 0.25 |
| **7** | 0.81 | 0.42 |
| **8** | 0.31 | 0.43 |
| **9** | 1.74 | 0.53 |
| **10** | 0.28 | 0.26 |
| **11** | 1.45 | 0.60 |
| **12** | 0.78 | 0.30 |
| **13** | 1.39 | 0.35 |
| **14** | 1.98 | 0.23 |
| **15** | 1.60 | 0.15 |
| **16** | 1.66 | 0.07 |
| **17** | 0.89 | 0.21 |
| **18** | 0.68 | 0.07 |
| **19** | 0.62 | 0.16 |
| **20** | 1.32 | 0.05 |
| **21** | 1.60 | 1.27 |
| **22** | 1.61 | 1.88 |
| **23** | 0.99 | 0.80 |
| **24** | 1.63 | 0.86 |
| **25** | 1.31 | 0.96 |
| **26** | 1.41 | 1.35 |
| **27** | 1.37 | 1.64 |
| **28** | 1.43 | 1.65 |
| **29** | 1.69 | 0.32 |
| **30** | 0.97 | 0.09 |
| **31** | 1.25 | 0.35 |
| **32** | 2.51 | 0.07 |
| **33** | 1.64 | 1.46 |
| **34** | 2.10 | 1.05 |
| **35** | 1.80 | 0.87 |
| **36** | 0.37 | 0.47 |
| **37** | 0.17 | 0.16 |
| **38** | 0.95 | 0.31 |
| **39** | 0.45 | 0.29 |
| **40** | 0.69 | 0.17 |
| **41** | 1.93 | 0.11 |
| **42** | 0.73 | 0.25 |
| **43** | 2.69 | 0.09 |
| **44** | 1.6 | |
| **45** | 1.3 | 1.1 |
| **46** | 1.44 | 1.76 |
| **47** | 1.38 | 1.23 |
| **48** | 1.66 | 1.42 |
| **49** | 1.74 | 1.04 |
| **50** | 2.78 | 2.0 |
| **51** | 1.85 | |
| **52** | 1.69 | 1.41 |
| **53** | 1.38 | 1.23 |
| **54** | 3.12 | 1.56 |
| **55** | 1.62 | 1.8 |
| **56** | 1.69 | 1.47 |
| **57** | 1.90 | |
| **58** | 1.63 | 1.09 |
| **59** | 1.87 | 0.61 |
| **60** | 1.24 | 0.95 |

### Example 65

### Mouse oral pharmacokinetic study:

Healthy male CD1 mice were used as experimental animals. The drug was administered by oral gavage at a dose of 10 mg/kg with a dosing volume of 10 mL/kg. Drug preparation: 10 mg of the compound was placed in 10 mL of 50% PEG400 or 20% HPb-cyclodextrin, ground and shaken to ensure uniform distribution of the compound in small particle size, followed by oral administration. Blood samples were collected at 30 min, 1 h, 2 h, and 4 h post-dose, and brain tissues were sampled at 4 h. EDTA dipotassium salt was added to the blood samples, and plasma was obtained after centrifugation. The supernatant was analyzed after dilution with 5 volumes of acetonitrile and centrifugation. The brain tissue homogenate was diluted with 3.5 volumes of acetonitrile, centrifuged, and the supernatant was collected for analysis. The samples were then analyzed by liquid chromatography-tandem mass spectrometry (LC-MS/MS): the instrument was AB Sciex 3500; the mobile phase consisted of 0.1% formic acid in water (A)/0.1% formic acid in acetonitrile (B), with gradient elution.

**Table 2 PK Test Results of Partial Compounds**

| Example | Tₘₐₓ h | Cₘₐₓ ng/mL | AUC₀₋₄ₕ ng*h/mL | C_{brain@4h} ng/mL | C_{pasma@4h} ng/mL | C_{brain}/Cₚₗₐₛₘₐ |
|---|---|---|---|---|---|---|
| **Cevipubulin** | 0.9 | 660 | 1826 | 38 | 376 | 0.10 |
| **CNDR-51657** | 0.5 | 49 | 57 | 0 | 3 | 0 |
| **11** | 0.75 | 4135 | 11156 | 252 | 2195 | 0.11 |
| **24** | 0.75 | 2645 | 5955 | 147 | 874 | 0.17 |
| **25** | 0.75 | 399 | 939 | 198 | 137 | 1.45 |
| **46** | 0.5 | 572 | 1598 | 356 | 325 | 1.09 |
| **48** | 0.75 | 591 | 1806 | 1018 | 359 | 2.83 |
| **55** | 0.5 | 496 | 1450 | 375 | 306 | 1.22 |
| **57** | 0.5 | 161 | 525 | 102 | 106 | 0.96 |
| **58** | 1.0 | 115 | 368 | 222 | 62 | 3.58 |
| **60** | 0.5 | 379 | 696 | 62 | 47 | 1.32 |

The above results indicate that the compounds of the present invention exhibit microtubule-stabilizing activity. Furthermore, compared with the controls Cevipubulin and CNDR-51657, the compounds of the present invention unexpectedly demonstrated significantly improved exposure in brain tissue.

All documents mentioned in the present disclosure are incorporated herein by reference as if each document were individually incorporated by reference. Furthermore, it should be understood that after reading the above teachings of the present disclosure, those skilled in the art may make various modifications or alterations to the present disclosure, and such equivalent forms shall also fall within the scope defined by the appended claims of this application.

## Claims

1. A compound as shown in general formula I or a pharmaceutically acceptable salt, stereoisomer, tautomer, or prodrug thereof: 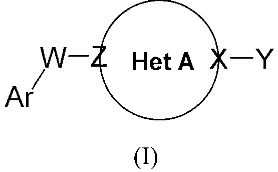 wherein,
Ar is: 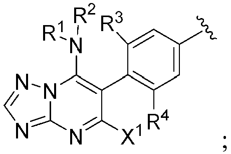
X¹ is selected from the group consisting of: Cl, CN, vinyl, -CH=CHC₁₋₆ alkyl, -CH=CHC₃₋₆ cycloalkyl, -C=CH, -C=C₁₋₆ alkyl, -C-CC₃₋₆ cycloalkyl, C₁₋₆ alkyl, C₁₋₆ haloalkyl, C₃₋₆ cycloalkyl, C₃₋₆ halocycloalkyl, OC₁₋₆ alkyl, OC₁₋₆ haloalkyl, SC₁₋₆ alkyl;
R¹ is selected from the group consisting of: C₁-C₁₀ alkyl, C₁-C₁₀ haloalkyl, C₃-C₁₀ cycloalkyl, C₃-C₁₀ heterocyclyl, C₃-C₁₀ halocycloalkyl, or R¹ is: 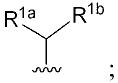
R^{1a} and R^{1b} are each independently hydrogen, C₁-C₁₀ alkyl, C₁-C₁₀ haloalkyl, C₃-C₁₀ cycloalkyl, C₃-C₁₀ halocycloalkyl, C₇-C₁₁ spirocycloalkane, C₅-C₁₀ heterospirocycle, aryl, heteroaryl;
R² is hydrogen;
R³ and R⁴ are independently hydrogen, F, Cl, Br;
W is a chemical bond, -O(CR^{a}R^{b})ₙ-, -(CR^{a}R^{b})ₙO-, -S(CR^{a}R^{b})ₙ₋, -(CR^{a}R^{b})ₙS-, -(CR^{a}R^{b})ₙ-NR^{e}-, - N(R^{e})-(CR^{a}R^{b})ₙ-,-C(O)N(R^{e})-(CR^{a}R^{b})ₙ-, -N(R^{e})C(O)-(CR^{a}R^{b})ₙ-,-(CR^{a}R^{b})ₙ-, -C(O)(CR^{a}R^{b})ₙ-, - (CR^{a}R^{b})ₙC(O)-, arylene,(Z)-CH=CH-,(E)-CH=CH-,-C=C-, C₅-C₉ fused heteroarylene, 5 or 6membered heteroarylene;
R^{a} and R^{b} are each independently hydrogen, substituted or unsubstituted C₁-C₁₀ alkyl, substituted or unsubstituted C₃-C₁₀ cycloalkyl, substituted or unsubstituted C₂-C₁₀ alkenyl, substituted or unsubstituted C₆-C₂₀ aryl, or substituted or unsubstituted C₃-C₁₄ heteroaryl; R^{a} and R^{b}, together with the carbon atom to which they are attached, may form a 3-8 membered ring or a 4-8 membered heterocycle, wherein the heteroatom may be sulfur, oxygen, NH, or NR^{e};
R^{c} and R^{d} are each independently hydrogen, C₁-C₁₀ alkyl, C₃-C₁₀ cycloalkyl, C₂-C₁₀ alkenyl, C₆-C₂₀ aryl, C₃-C₁₄ heteroaryl; R^{c} and R^{d} may be substituted by one or more groups selected from the group consisting of: halogen, hydroxyl, amino, nitro, cyano, aldehyde, carboxyl, alkoxy, -CF₃, - SF₅. R^{c} and R^{d}, together with the nitrogen atom to which they are attached, may form a3-8 membered ring or a 4-8membered heterocycle, wherein the heteroatom may be sulfur, oxygen, NH, or NR^{e};
R^{e} is hydrogen, C₁-C₆ alkyl, -(CR^{a}R^{b})ₙ-C₃-C₆ cycloalkyl, -(CR^{a}R^{b})ₙ₋aryl, -(CR^{a}R^{b})ₙ₋heteroaryl; R^{e} may be substituted by one or more groups selected from the group consisting of: halogen, hydroxyl, amino, nitro, cyano, aldehyde, carboxyl, alkoxy, -CF₃, -SF₅.
Y is H, halogen, OR^{e}, -(CR^{a}R^{b})ₘ-CO₂H, -(CR^{a}R^{b})ₘ-CO(CR^{a}R^{b})ₙ-NR^{a}R^{b}, C₁-C₆ alkyl, C₁-C₆ haloalkyl, -(CR^{a}R^{b})ₙ-C₃-C₆ cycloalkyl, -(CR^{a}R^{b})ₙ-C₃-C₆ halocycloalkyl, -(CR^{a}R^{b})ₙ-aryl, - (CR^{a}R^{b})ₙ-heteroaryl, -(CR^{a}R^{b})ₙ-NR^{c}R^{d}, -O(CR^{a}R^{b})ₙ-NR^{c}R^{d}, -S(CR^{a}R^{b})ₙ-NR^{c}R^{d}, -NR^{e}(CR^{a}R^{b})ₙ-NR^{c}R^{d}, -(CR^{a}R^{b})ₙ-P(O)Me₂, -(CR^{a}R^{b})ₙ-SO₂R^{a}, -(CR^{a}R^{b})ₙ-SO₂NR^{c}R^{d}, -(CR^{a}R^{b})ₙ-NR^{e}CONR^{c}R^{d}, - (CR^{a}R^{b})ₙ-CONR^{c}R^{d}.
m and n are independently 0, 1, 2, 3, 4, 5, or 6; 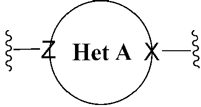
is monocyclic hydrocarbyl, spiro hydrocarbyl, fused hydrocarbyl, bridged hydrocarbyl, monocyclic heterocyclyl, spiro heterocyclyl, fused heterocyclyl, and bridged heterocyclyl structures;
X and Z are independently C(R⁵), N;
R⁵ is hydrogen, OH, CN, halogen, NR^{c}R^{d}, C₁-C₁₀ alkyl, C₁-C₁₀ haloalkyl, -(CR^{a}R^{b})ₙ-C₃-C₁₀ cycloalkyl, -(CR^{a}R^{b})ₙ-C₃-C₁₀ halocycloalkyl, -(CR^{a}R^{b})ₙ-CO₂H, -(CR^{a}R^{b})ₙ-CONR^{c}R^{d};
unless otherwise specified, the monocyclic hydrocarbyl, spiro hydrocarbyl, fused hydrocarbyl, bridged hydrocarbyl, monocyclic heterocyclyl, spiro heterocyclyl, fused heterocyclyl or bridged heterocyclyl has 5 to 20 ring skeletal atoms, and when the ring skeletal atoms contain heteroatoms, the heteroatoms may be 1 to 4 (such as 3) heteroatoms selected from nitrogen, sulfur or oxygen.

2. The compound of claim 1, or a pharmaceutically acceptable salt, stereoisomer, tautomer, or prodrug thereof, wherein the R^{1a} is: CF₃, 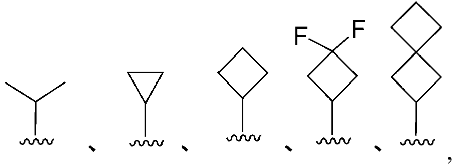 R^{1b} is hydrogen, methyl, or CF₃.

3. The compound of claim 1, or a pharmaceutically acceptable salt, stereoisomer, tautomer or prodrug thereof, wherein the Ar is:

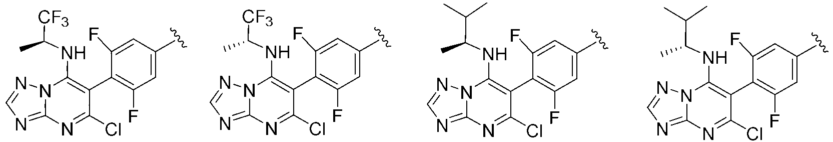

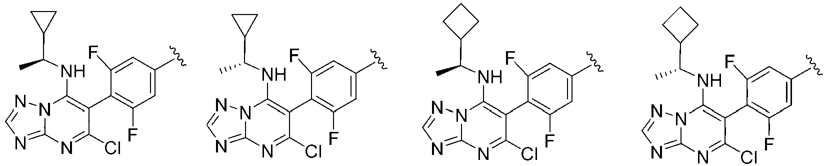

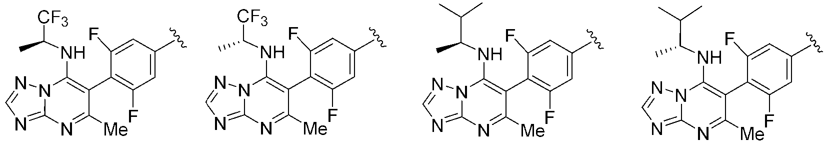

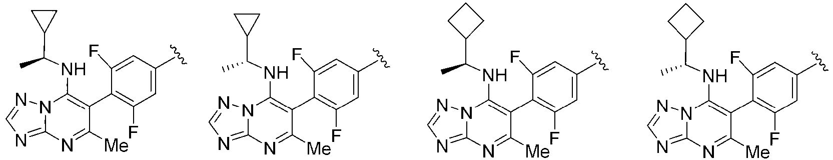

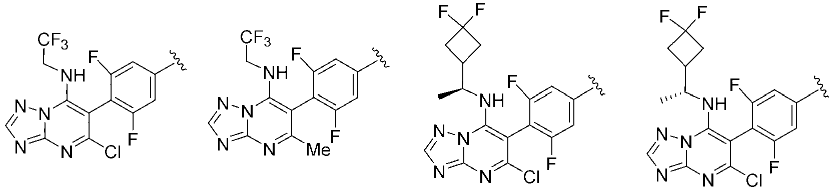

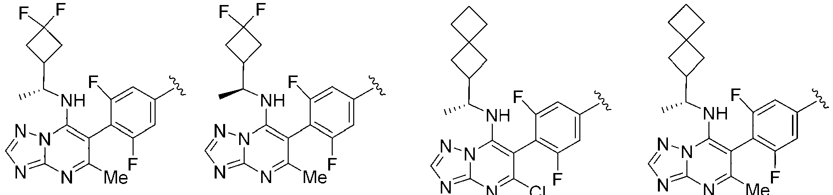

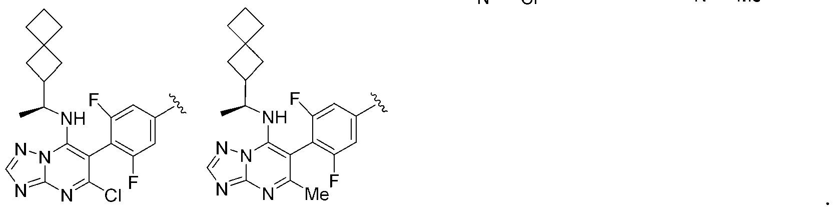

4. The compound of claim 1, or a pharmaceutically acceptable salt, stereoisomer, tautomer or prodrug thereof, wherein, 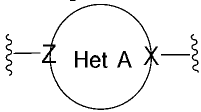 is:

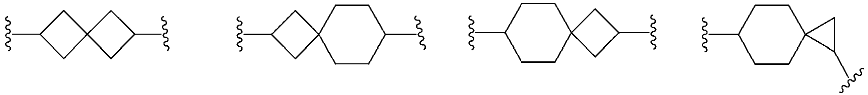

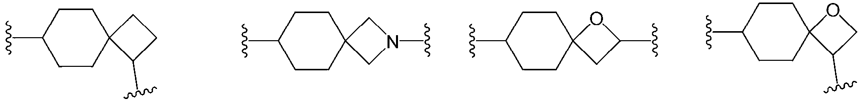

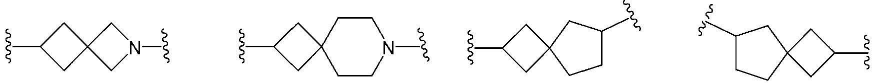

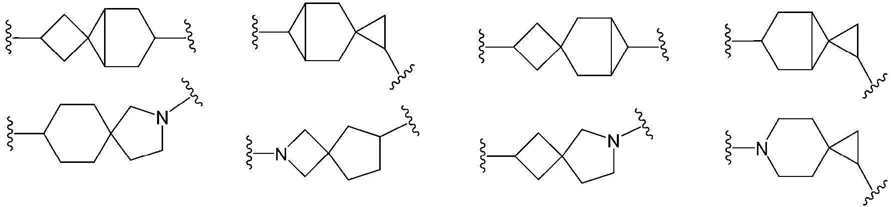

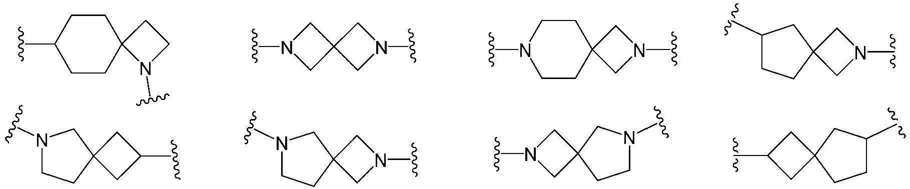

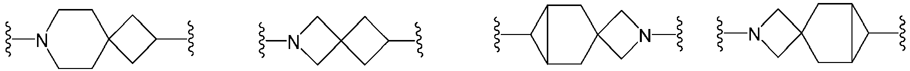

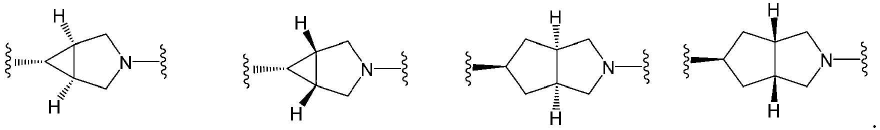

5. The compound of claim 1, or a pharmaceutically acceptable salt, stereoisomer, tautomer or prodrug thereof, wherein the compound has a structure as shown in the general formula selected from the group consisting of: 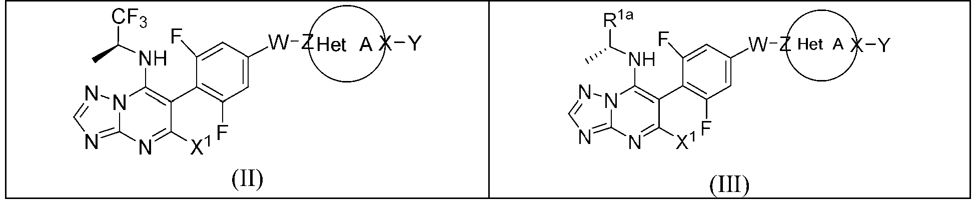 wherein each group is as defined in claim 1.

6. The compound of claim 1, or a pharmaceutically acceptable salt, stereoisomer, tautomer or prodrug thereof, wherein the compound are selected from the group consisting of: 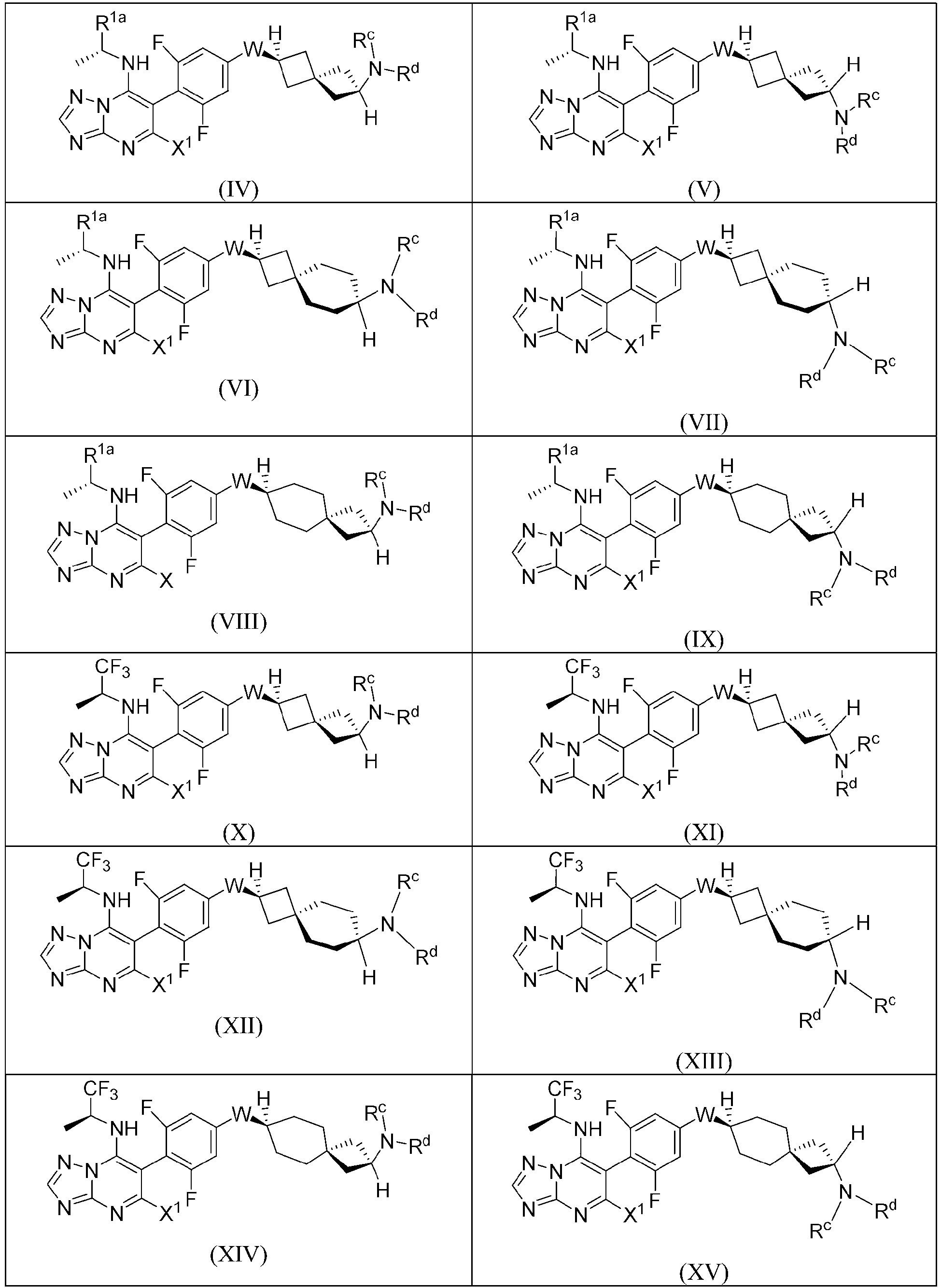 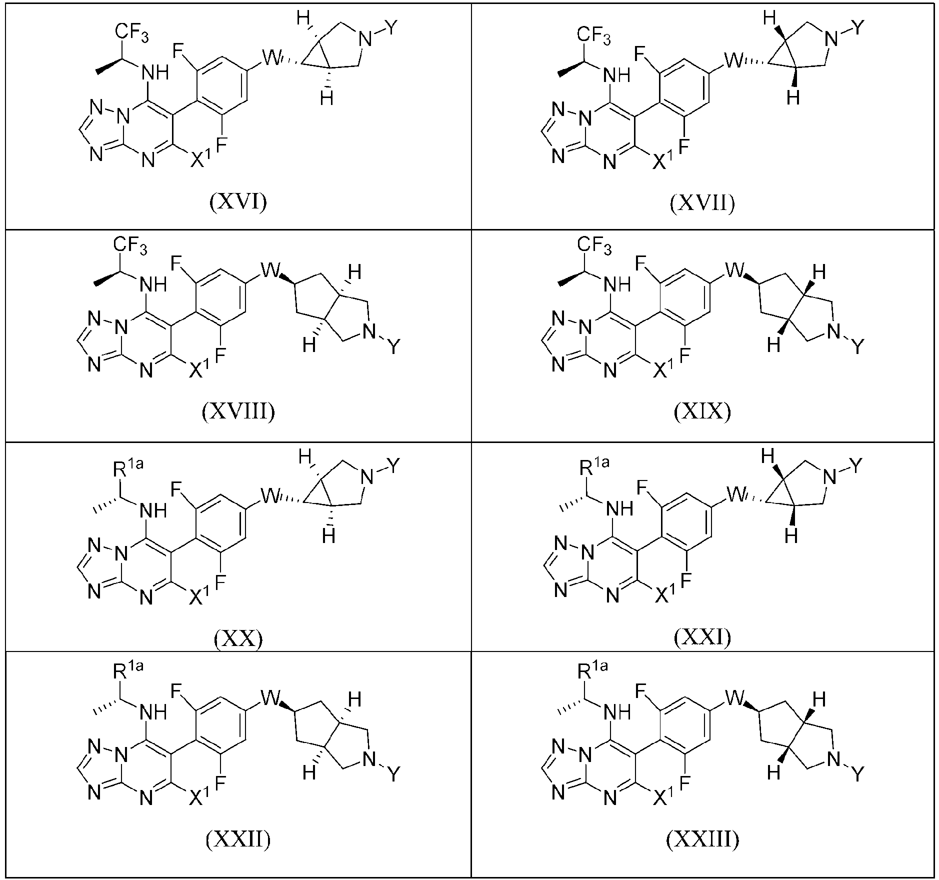 wherein W and each group are as defined in claim 1.

7. The compound of claim 1, or a pharmaceutically acceptable salt, stereoisomer, tautomer or prodrug thereof, wherein the compound is: 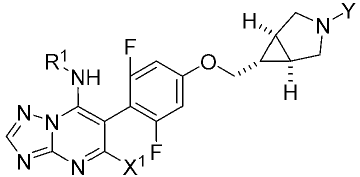 wherein
R¹ is selected from the following structures: 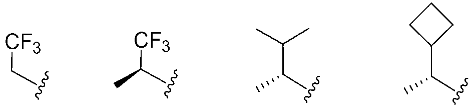
X¹ is selected from Cl, methyl, CF₃, ethyl, or cyclopropyl; Y is selected from H, C₁₋₆ alkyl, C₁₋₆fluoroalkyl, C₃₋₆ cycloalkyl, C₃₋₆ fluorocycloalkyl, -SO₂R^{a}, -SO₂NR^{c}R^{d}, -CONR^{c}R^{d}, aryl, or heteroaryl.

8. The compound of claim 1, or a pharmaceutically acceptable salt, stereoisomer, tautomer or prodrug thereof, wherein the compound is: 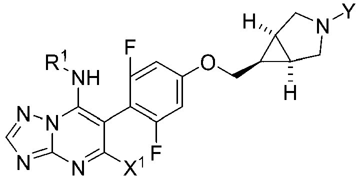 wherein
R¹ is selected from the following structures: 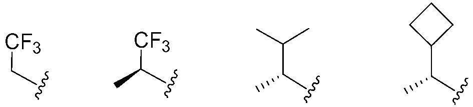
X¹ is selected from Cl, methyl, CF₃, ethyl, or cyclopropyl; Y is selected from H, C₁₋₆ alkyl, C₁₋₆ fluoroalkyl, C₃₋₆ cycloalkyl, C₃₋₆ fluorocycloalkyl, -SO₂R^{a}, -SO₂NR^{c}R^{d}, -CONR^{c}R^{d}, aryl, or heteroaryl.

9. The compound of claim 1, or a pharmaceutically acceptable salt, stereoisomer, tautomer or prodrug thereof, wherein the compound is: 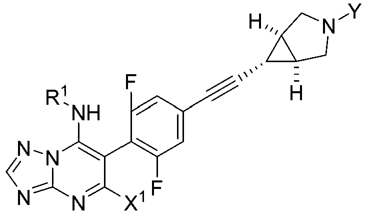 wherein
R¹ is selected from the following structures: 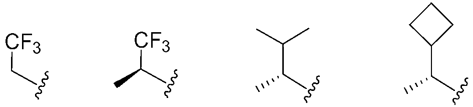
X¹ is selected from Cl, methyl, CF₃, ethyl, or cyclopropyl; Y is selected from H, C₁₋₆ alkyl, C₁₋₆ fluoroalkyl, C₃₋₆ cycloalkyl, C₃₋₆ fluorocycloalkyl, -SO₂R^{a}, -SO₂NR^{c}R^{d}, -CONR^{c}R^{d}, aryl, or heteroaryl.

10. The compound of claim 1, or a pharmaceutically acceptable salt, stereoisomer, tautomer or prodrug thereof, wherein the compound has a structure as shown in the formula below: 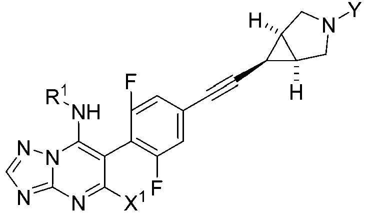 wherein
R¹ is selected from the following structures: 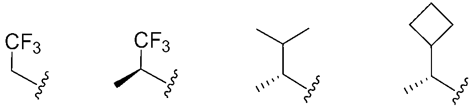
X¹ is selected from Cl, methyl, CF₃, ethyl, or cyclopropyl; Y is selected from H, C₁₋₆ alkyl, C₁₋₆ fluoroalkyl, C₃₋₆ cycloalkyl, C₃₋₆ fluorocycloalkyl,-SO₂R^{a}, -SO₂NR^{c}R^{d}, -CONR^{c}R^{d}, aryl, or heteroaryl.

11. The compound of claim 1, wherein the compound is selected from the group consistinof:
| No. | Structure | No. | |
|---|---|---|---|
| 23 | 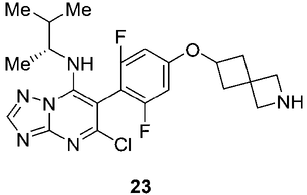 | 61A | 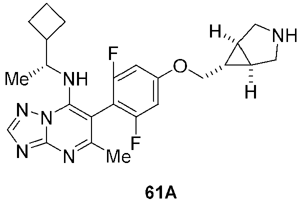 |
| 24 | 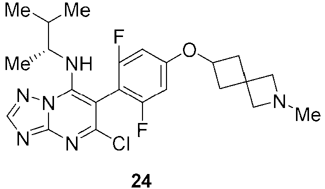 | 65 | 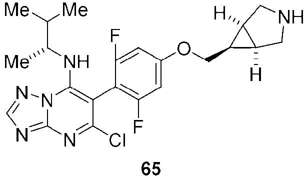 |
| 26 | 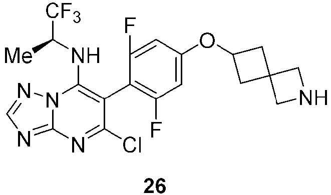 | 66 | 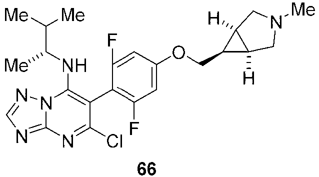 |
| 27 | 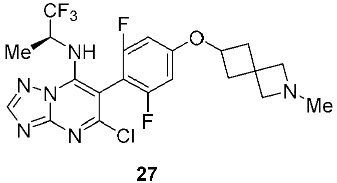 | 68 | 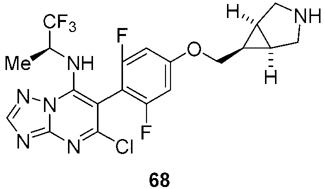 |
| 30 | 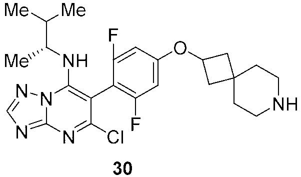 | 69 | 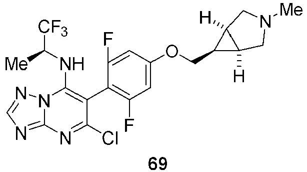 |
| 31 | 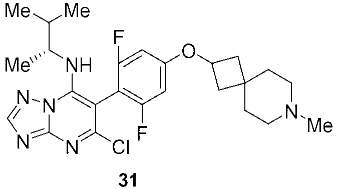 | 71 | 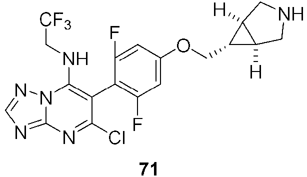 |
| 33 | 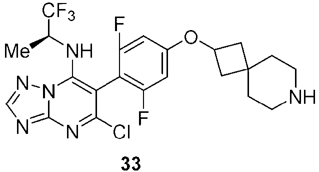 | 72 | 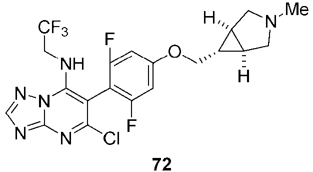 |
| 34 | 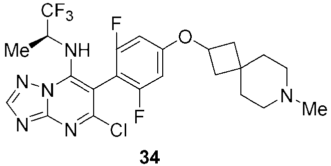 | 71A | 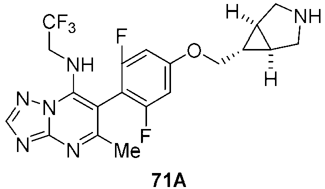 |
| 36 | 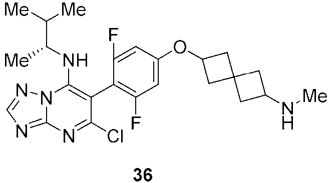 | 74 | 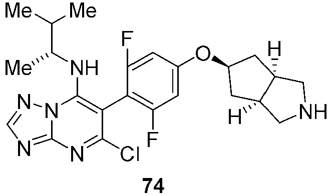 |
| 37 | 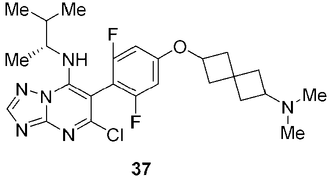 | 75 | 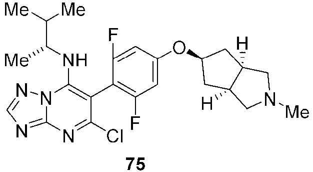 |
| 39 | 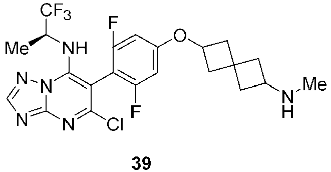 | 77 | 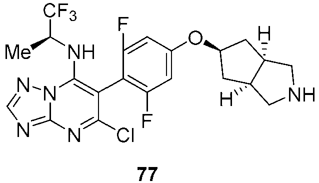 |
| 40 | 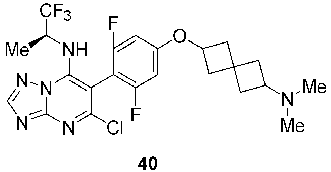 | 78 | 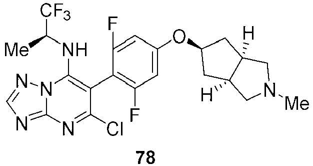 |
| 42 | 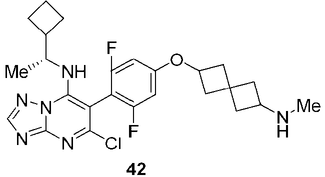 | 80 | 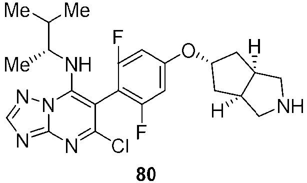 |
| 43 | 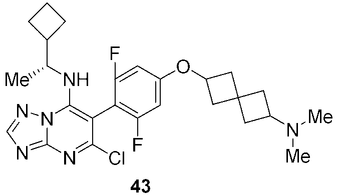 | 81 | 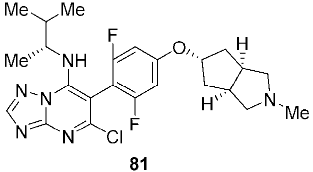 |
| 45 | 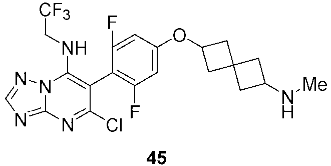 | 83 | 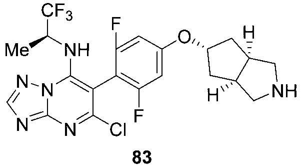 |
| 46 | 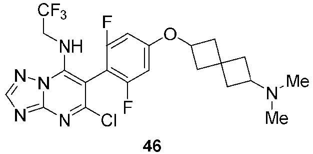 | 84 | 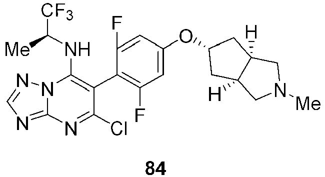 |
| 48 | 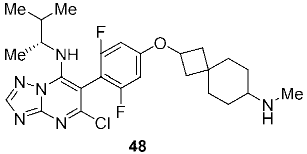 | 86 | 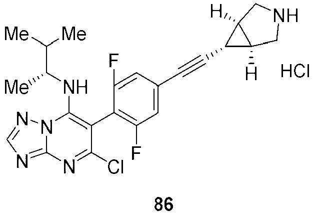 |
| 49 | 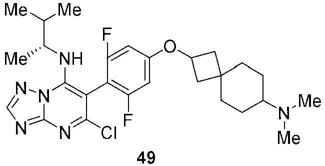 | 88 | 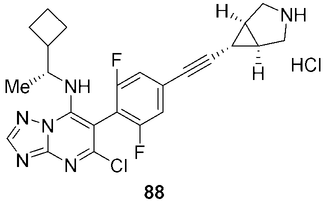 |
| 51 | 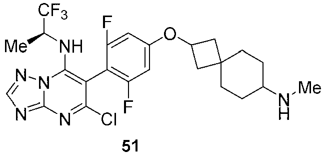 | 89 | 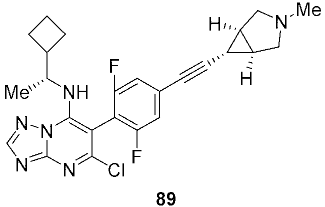 |
| 52 | 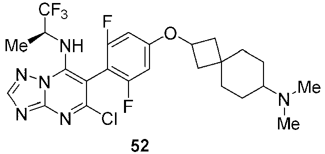 | 91 | 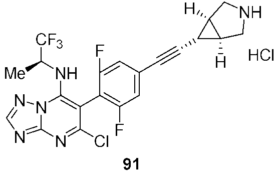 |
| 55 | 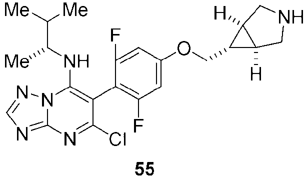 | 92 | 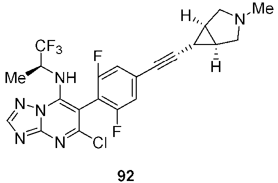 |
| 56 | 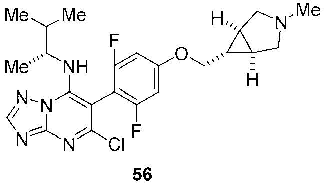 | 94 | 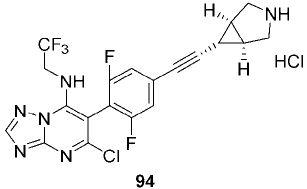 |
| 55A | 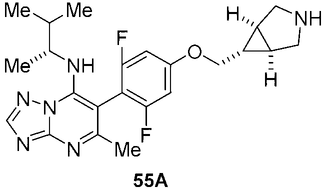 | 95 | 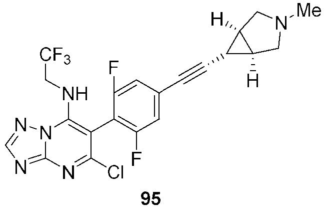 |
| 58 | 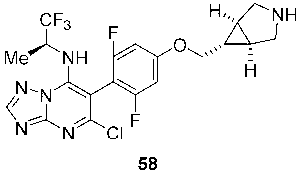 | 97 | 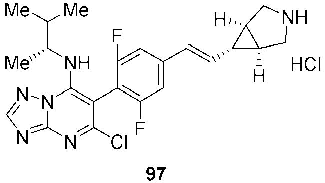 |
| 59 | 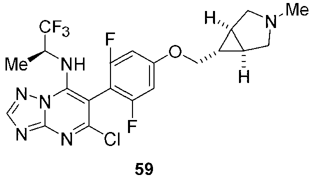 | 99 | 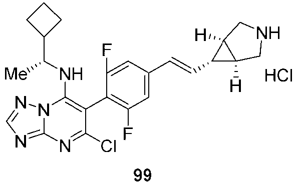 |
| 61 | 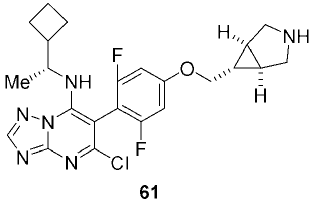 | 101 | 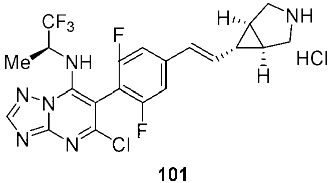 |
| 62 | 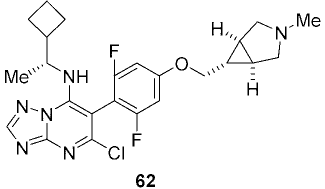 | 103 | 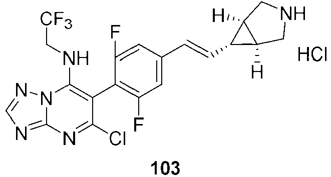 |

12. The use of the compound of any one of claims 1-11, wherein the compound is used to:
(i) prepare a microtubule stabilizer;
(ii) prepare a pharmaceutical composition for treating microtubule-mediated diseases;
(iii) prepare a medicament for preventing and/or treating cancer and a neurodegenerative disease.

13. The use of claim 12, wherein the cancer is selected from the group consisting of: glioma, colon cancer, breast cancer, gastric cancer, lung cancer, colorectal cancer, pancreatic cancer, ovarian cancer, prostate cancer, kidney cancer, liver cancer, brain cancer, melanoma, multiple myeloma, chronic myeloid leukemia, hematologic malignancies, lymphoid malignancies, or metastatic lesions in tissues or organs distant from the primary tumor site.

14. The use of claim 12, wherein the neurodegenerative disease is selected from the group consisting of: Huntington's disease, Alzheimer's disease, Parkinson's disease, multiple sclerosis, and traumatic brain injury.

15. A pharmaceutical composition, wherein the pharmaceutical composition comprises a therapeutically effective amount of the compound of any one of claims 1-6, and a pharmaceutically acceptable carrier.

16. The pharmaceutical composition of claim 15, wherein the pharmaceutical composition comprises other antitumor drugs, preferably, the pharmaceutical composition is selected from the group consisting of: PD-1 antibody, PD-L1 antibody, CTLA-4 antibody, antitumor chemotherapeutic drugs (such as temozolomide), and other targeted drugs.
